# EUROPEAN PATENT SPECIFICATION

(11) **EP 3 845 535 B1**
(45) Date of publication and mention of the grant of the patent: **26.06.2024**
(21) Application number: 19855039.4
(22) Date of filing: 29.08.2019
(51) Int. Cl.: C07D 473/18, C07D 487/14, A61K 31/522, A61P 3/00, A61P 3/06, A61P 9/00, A61P 9/12, A61P 13/12, A61P 19/06, A61P 43/00

(54) **HYDRAZINOPURINE COMPOUND AND TRIAZOLOPURINE COMPOUND FOR INHIBITING XANTHINE OXIDASE**
HYDRAZINOPURINVERBINDUNG UND TRIAZOLOPURINVERBINDUNG ZUR HEMMUNG VON XANTHINOXIDASE
COMPOSÉ HYDRAZINOPURINE ET COMPOSÉ TRIAZOLOPURINE POUR L'INHIBITION DE LA XANTHINE OXYDASE

(30) Priority: 30.08.2018 JP 2018161925
(43) Date of publication of application: 07.07.2021
(73) Proprietor: Tera Stone Co., Ltd, Yokohama-shi, Kanagawa 222-0033 (JP)
(72) Inventor: NAGAMATSU, Tomohisa, Kumamoto-shi Kumamoto 860-0811 (JP)
(74) Representative: Schulz Junghans Patentanwälte PartGmbB
(86) International application number: PCT/JP2019/033871
(87) International publication number: WO 2020/045558

(56) References cited:
- EP-A1- 0 263 071
- EP-A1- 0 811 624
- EP-A2- 0 417 790
- EP-A2- 2 049 544
- WO-A1-01/07441
- WO-A1-96/26208
- JP-A- H01 500 996
- JP-A- H03 204 879
- JP-A- H09 208 467
- JP-A- S59 232 342
- JP-A- 2009 541 356
- TORO I ET AL: "Development and validation of a capillary electrophoresis method with ultraviolet detection for the determination of the related substances in a pharmaceutical compound", JOURNAL OF CHROMATOGRAPHY A, ELSEVIER, AMSTERDAM, NL, vol. 1043, no. 2, 1 July 2004 (2004-07-01) , pages 303-315, XP008114794, ISSN: 0021-9673, DOI: 10.1016/J.CHROMA.2004.05.091
- NAGAMATSU, T. et al.: "Novel xanthine oxidase inhibitor studies. Part 2.1 Synthesis and xanthine oxidase inhibitory activities of 2-substituted 6- alkylidenehydrazino-or 6-arylmethylidenehydrazino- 7H-purines and 3-and/or 5-substituted 9H-1,2,4- triazolo[3,4-i]purines", Journal of the Chemical Society, Perkin Transactions 1: Organic and Bio- Organic Chemistry, vol. 21, 1999, pages 3117-3125, XP055695909,
- PENEZ, N. et al.: "Anti fouling Properties of Simple Indole and Purine Alkaloids from the Mediterranean Gorgonian Paramuricea clavata", Journal of Natural Products, vol. 74, no. 10, 2011, pages 2304-2308, XP055695911, ISSN: 0163-3864

## Description

### Technical Field

The present invention relates to novel triazolopurine compounds having xanthine oxidase inhibition activity, as well as to a pharmaceutical composition containing said compounds as an active ingredient.

### Background Art

Uric acid is synthesized from an oxypurine such as xanthine and hypoxanthine by xanthine oxidase (XO). Uric acid is the final oxidation product of purine metabolism in humans and many other primates and a high level of uric acid in blood causes gout attack. To lower the uric acid level, drugs have been used that suppress uric acid production: one example of such drugs is allopurinol (trade name: Zyloric). Allopurinol is metabolized by XO and converted into oxypurinol (competitive inhibition with oxypurine). Oxypurinol also acts to inhibit XO (XO enzyme inhibition). That is, this oxypurinol binds to a molybdopterin unit at the enzymatic active center of xanthine oxidase, also resulting in the inhibition of the enzyme. Specifically, due to the structural resemblance of allopurinol to xanthine, xanthine oxidase mistakenly takes up allopurinol rather than xanthine. As a result, the allopurinol is oxidized to form oxypurinol, which in turn inhibits xanthine oxidase to suppress formation of uric acid. Through such a mechanism of action, allopurinol decreases uric acid levels, thus treating hyperuricemia and gout. Since Zyloric (allopurinol) is excreted through kidney, its dosage to patients with decreased kidney function needs to be adjusted.

Since as much as three to five percents of patients administered allopurinol would suffer from side effects such as severe skin disorders including toxic epidermal necrosis, Stevens-Johnson syndrome and exfoliative dermatitis, or hypersensitivity vasculitis, and in an effort to develop new alternative drugs for patients with kidney failure, novel gout therapeutic agents with inhibitory activity against xanthine oxidoreductase (XOR) have recently been developed. Specifically, Febuxostat (trade name: Feburic), a non-purine type xanthine oxidase inhibitor developed by Teijin Pharma and approved January 2011, inhibits activity of xanthine oxidoreductase (XOR) to suppress uric acid production and decrease blood and urine concentrations of uric acid. Since Febuxostat is an inhibitor based on the structure of the active center of the enzyme protein, it is believed that unlike conventional XO inhibitor allopurinol, the manner by which it inhibits XOR does not depend on the oxidoreductive state of XOR and Febuxostat acts by mixed inhibition by strongly binding to both the oxidized and reduced forms of XOR to exhibit strong enzyme inhibitory activity. Febuxostat acts to decrease the blood and urine levels of uric acid at lower doses compared to allopurinol. Also, pharmacokinetic studies using rats revealed that about 50% of Febuxostat was excreted through non-renal excretion pathways. Febuxostat inhibits xanthine oxidase through allosteric inhibition (i.e., the drug acts on a site other than the active center of the enzyme to cause a conformational change of the enzyme, thereby indirectly decreasing the catalytic and other activities of the enzyme.), rather than through competitive inhibition. Thus, it is believed that allopurinol inhibits xanthine oxidase by directly binding to the molybdopterin unit, which is the active center of xanthine oxidase, whereas Febuxostat inhibits xanthine oxidase by binding to the proximity of the active center to interrupt the access of hypoxanthine or xanthine to the enzymatic active center. An advantageous characteristic of Febuxostat is that it is highly lipophilic and can thus be metabolized in liver, making it suitable for use in persons with kidney failure. Nonetheless, despite its stronger ability to decrease uric acid than that of allopurinol, Febuxostat may cause impaired liver function as severe side effects, such as impaired liver function associated with the elevated levels of AST (GOT) and ALT (GPT). Thus, the condition of patients must be extensively monitored during the administration period of the drug, for example, by periodically conducting tests. In addition, since xanthine oxidase is a metabolic enzyme of mercaptopurine and azathioprine, the inhibition of xanthine oxidase by Febuxostat may potentially exacerbate bone marrow suppression and other side effects. Thus, the use of Febuxostat in combination with these drugs is prohibited. Moreover, Fuji Yakuhin Co., Ltd. has developed a novel compound named topiroxostat (trade names: Topiloric, Uriadec), which has a similar structure to Febuxostat and has similar XOR inhibitory activities and side effects. The drug was approved in June 2013.

Meanwhile, as other compounds having inhibitory activities of xanthine oxidase, purine derivative compounds have been reported. None of them have yet to be approved, however (See Patent Document 1 and Non-Patent Document 1. Patent Document 1 discloses compounds that are useful in the treatment of nervous system disorders such as anxiety, convulsive conditions (i.e. epilepsy) and other disorders responsive to BR agonists or mixed agonist/antagonists. Patent Document 2 discloses purinone derivatives and their use as HM74A agonists. HM74A is a G protein-coupled receptor for nicotinic acid (niacin), which has been used clinically to treat dyslipidemia. Patent Document 3 discloses purinone derivatives for use as HM74A agonists for use in treating cardiovascular disease. Patent document 3 relates to purine derivatives with bronchodilatation, diuretic, renal protecting, and/or amnestic activity. Non Patent 1 document discloses synthesis techniques for various purine aldehyde hydrazones.

### Citation List

### Patent Documents

Patent Document 1: WO 96/26208 publication
Patent Document 2: EP 0 263 071
Patent Document 3: EP 2 049 544
Patent Document 4: EP 0 417 790Non-Patent Documents
Non-Patent Document 1: Nagamatsu et. al., J. Chem. Soc., Perkin Trans. 1, 3117 - 3125 (1999)

### Summary of the Invention

### Problem to be Solved by the Invention

Accordingly, it is a major objective of the present invention to provide novel triazolopurine compounds having xanthine oxidase inhibition activity.

### Means of Solving the Problem

In an effort to find a solution to the above-identified problem, the present inventors have attempted to synthesize and search for drugs that have less side effects, safer for use, and require less doses than the above-described conventional therapeutic agents for gout to achieve inhibition of xanthine oxidoreductase (XOR). As a result, the present inventors have found hydrazinopurine derivative compounds (I) and triazolopurine derivative compounds (II) as candidate compounds. It was found that these compounds inhibit uric acid formation through the same reaction mechanisms (competitive inhibition and enzymatic inhibition) as allopurinol and exhibit 300 to 100 times or higher strong inhibition than allopurinol in an in-vitro uric acid formation inhibition test using cow milk-derived xanthine oxidase (XO). When these compounds were further investigated for the manner of inhibition against xanthine oxidoreductase (XOR) and docking score (binding free energy, kcal/mol) using computational chemistry on a computer, a useful correlation was found from the plot of the docking score (vertical axis) and the XO inhibition (in vitro IC₅₀, horizontal axis). Also, the manner of docking to several different xanthine oxidases (XOs) were investigated for allopurinol and the novel compounds. It was also found out that the hydrazinopurine derivative compound (I) and the triazolopurine derivative compound (II) exhibit competitive inhibition similar to allopurinol and allosteric inhibition similar to Febuxostat. Accordingly, these compounds are expected to exhibit even stronger inhibitory activities than the previously developed xanthine oxidase inhibitors and are believed to be capable of being metabolized and excreted with less burden to kidney and liver. The newly developed novel compounds, which are analogue compounds of nucleic acid-related compounds such as purines and xanthines, are more lipophilic than allopurinol and can be used to synthesize lipophilic derivatives close to Febuxostat. It is considered that the novel compounds also have higher bioavailability, are more readily metabolized and excreted in urine, and are less toxic than allopurinol. In addition, the novel compounds are expected to provide effective XOR inhibition at less doses and are thus thought to cause less side effects. It is these findings and successful examples that have led to the completion of the present invention. 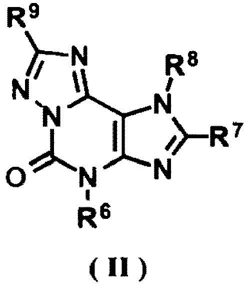 A first aspect of the invention relates to relates to a triazolopurine compound represented by the following general formula (II):
wherein R⁶ represents a hydrogen atom, and R⁸ represents a hydrogen atom or an alkyl group; R⁷ and R⁹ each independently represent hydrogen atom, alkyl group or aryl group, or a phenyl group having a substituent selected from: a halogen atom, an alkyl group, an alkoxy group, an amino group, an alkylamino group, a methylenedioxy group, a hydroxy group or nitro group.

In certain embodiments of the triazolopurine compound (II) according to claim 2 of the invention, R⁷ and R⁹ each independently are selected from hydrogen, alkyl, a methylphenyl, ethylphenyl, methoxyphenyl, ethoxyphenyl, dimethylaminophenyl, diethylaminophenyl, fluorophenyl, chlorophenyl, bromophenyl, iodophenyl, methylenedioxyphenyl; hydroxyphenyl; and nitrophenyl.

In certain embodiments according to claim 3 of the invention, R⁶, R⁷ and R⁸ are all hydrogen atoms, and R⁹ is selected from hydrogen atom, alkyl group, or a phenyl group having a substituent selected from: a halogen atom, an alkyl group, an alkoxy group, an amino group, an alkylamino group, a methylenedioxy group, a hydroxy group or nitro group.

In certain embodiments according to claim 3 of the invention, R⁶, R⁷ and R⁸ are all hydrogen atoms, and R⁹ is selected from hydrogen, alkyl, a methylphenyl, ethylphenyl, methoxyphenyl, ethoxyphenyl, dimethylaminophenyl, diethylaminophenyl, fluorophenyl, chlorophenyl, bromophenyl, iodophenyl, methylenedioxyphenyl, hydroxyphenyl, and nitrophenyl.

In certain embodiments of the triazolopurine compound (II) according to claim 4 of the invention, R⁶ and R⁸ both represent hydrogen atoms, and
- R⁷ is an alkyl group or an aryl group selected from a phenyl group, and a phenyl group having a substituent selected from: a halogen atom, an alkyl group, an alkoxy group, an amino group, an alkylamino group, a methylenedioxy group, a hydroxy group or nitro group, and
- R⁹ is hydrogen atom, an alkyl group, or a phenyl group having a substituent selected from: a halogen atom, an alkyl group, an alkoxy group, an amino group, an alkylamino group, a methylenedioxy group, a hydroxy group or nitro group.

In certain embodiments of the triazolopurine compound (II) of the invention, R⁶, R⁷, R⁸, and R⁹ each are as shown in the table provided in claim 5.

A second aspect of the invention according to claim 6 relates to a triazolopurine compound represented by the general formula (II) as provided in the first aspect of the invention, wherein R⁶ represents a hydrogen atom, and R⁸ represents a hydrogen atom or an alkyl group; R⁷ and R⁹ each independently represent a hydrogen atom, an alkyl group, phenyl group or a phenyl group having a substituent selected from: a halogen atom, an alkyl group, an alkoxy group, an amino group, an alkylamino group, a methylenedioxy group, a hydroxy group or nitro group, for use in treatment or prevention of a condition selected from hyperuricemia, gout, impaired kidney functions such as kidney failure and chronic kidney disease, hypertension, cardiovascular disease, dyslipidemia and metabolic syndrome.

In some embodiments of the second aspect of the invention corresponding to claim 7 of the invention, R⁷ and R⁹ each independently are selected from hydrogen, alkyl, phenyl, a methylphenyl, ethylphenyl, methoxyphenyl, ethoxyphenyl, dimethylaminophenyl, diethylaminophenyl, fluorophenyl, chlorophenyl, bromophenyl, iodophenyl, methylenedioxyphenyl; hydroxyphenyl; and nitrophenyl.

In alternative embodiments of the second aspect of the invention corresponding to claim 8, R⁶, R⁷ and R⁸ are all hydrogen atom, and R⁹ is selected from hydrogen atom, alkyl group, or a phenyl group having a substituent selected from: a halogen atom, an alkyl group, an alkoxy group, an amino group, an alkylamino group, a methylenedioxy group, a hydroxy group or nitro group.

In further alternative embodiments of the second aspect of the invention corresponding to claim 9, R⁶ and R⁸ are both hydrogen atom, and
- R⁷ is an alkyl group or an aryl group selected from a phenyl group, and a phenyl group having a substituent selected from: a halogen atom, an alkyl group, an alkoxy group, an amino group, an alkylamino group, a methylenedioxy group, a hydroxy group or nitro group, and
- R⁹ is hydrogen atom, an alkyl group, a phenyl group or a phenyl group having a substituent selected from: a halogen atom, an alkyl group, an alkoxy group, an amino group, an alkylamino group, a methylenedioxy group, a hydroxy group or nitro group.

In still further embodiments of the second aspect of the invention, the compound is selected from the Table provided in claim 10.

### Effect of the Invention

The triazolopurine compounds according to the present invention have stronger inhibitory activity as compared to allopurinol. In addition, they can be metabolized and excreted while posing less burden to kidney and liver and are thus expected to have higher bioavailability and weaker toxicity as it can form more stable binding with xanthine oxidase than can any of conventional xanthine oxidases such as allopurinol. Moreover, the novel compounds are expected to provide effective xanthine oxidase inhibition activity at less doses, thus causing less side effects. Since the triazolopurine compound in one aspect of the present invention have the xanthine oxidase inhibition activity, a composition containing the triazolopurine compound in one aspect of the present invention is useful as a composition for inhibiting xanthine oxidase and is thus further useful as a therapeutic agent for hyperuricemia or as a pharmaceutical composition for preventing or treating urate deposition diseases, including gout, gouty arthritis and gouty nodes caused by hyperuricemia. Also, since kidney disorders, such as kidney failure and chronic kidney disease, hypertension, cardiovascular disease, dyslipidemia and metabolic syndrome are caused by, and are closely associated with, hyperuricemia, the triazolopurine compound in one aspect of the present invention is useful as a pharmaceutical composition for preventing or treating kidney disorders, such as kidney failure and chronic kidney disease, hypertension, cardiovascular disease, dyslipidemia and metabolic syndrome.

### Description of Embodiments Description

While triazolopurine compounds are one aspect of the present invention, a production method thereof and a pharmaceutical composition containing the foregoing compounds will now be described in further details.

Unless otherwise specified, "xanthine oxidase" as used herein is a collective term for enzymes that catalyze oxidation reactions leading from hypoxanthine to xanthine and then to uric acid. While two types of xanthine oxidoreductive enzymes responsible for these reactions exist: oxidase form and dehydrogenase form, both are encompassed by "xanthine oxidase" as used herein. Unless otherwise specified, "xanthine oxidase" as in "xanthine oxidase inhibition activity" and "xanthine oxidase inhibitory composition" has the same meaning as defined above.

"Xanthine oxidase inhibition activity" refers to inhibitory activity against reactions catalyzed by the above-described xanthine oxidase. Preferably, the compound of the present invention exhibits xanthine oxidase inhibition activity at a lower dose than any of the conventional treatments having xanthine oxidase inhibition activity, such as allopurinol.

Preferably, the complex that the compound of the present invention forms with xanthine oxidase is in a more stable state in order for the compound of the present invention to exhibit higher xanthine inhibition activity or more sustained xanthine inhibition activity. Measures for evaluating the stability of the state of the complex include binding constant and binding free energy of the state of the complex formed between the compound and xanthine oxidase. When binding free energy is employed, a smaller value is preferred.

Further disclosed herein are hydrazinopurine compounds (I) having substituents shown in Table 1. Compounds (I) are not part of the invention.

In Table 1, Me represents methyl group and Ph represents phenyl group. For example, 4-MeO-C₆H₄ represents a phenyl group having a methoxy group at position 4. The same applies to the tables and to the context of the present specification hereinbelow. Compounds of Compound IDs I-1 to I-7 and I-14 to I-18 in Table 1 are described in the following articles:
(1) PCT Int. Appl. (1996) WO 96/26208, entitled "Purine Compounds and Xanthine Oxidase Inhibitors." Inventors: Tomohisa Nagamatsu, Yoko Watanabe, Kazuki Endo, and Masahiro Imaizumi.
(2) T. Nagamatsu, H. Yamasaki, T. Fujita, K. Endo, and H. Machida, J. Chem. Soc., Perkin Trans. 1, 3117 - 3125 (1999).

**[table 1]**

| | | | | | |
|---|---|---|---|---|---|
| 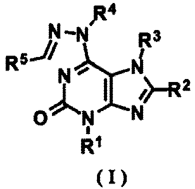 | | | | | |

| **Compound ID** | **R¹** | **R²** | **R³** | **R⁴** | **R⁵** |
|---|---|---|---|---|---|
| **I-1** | **H** | **H** | **H** | **H** | **4-F-C₆H₄** |
| **I-2** | **H** | **H** | **H** | **H** | **4-Cl-C₆H₄** |
| **I-3** | **H** | **H** | **H** | **H** | **4-Me₂N-C₆H₄** |
| **I-4** | **H** | **H** | **H** | **H** | **4-O₂N-C₆H₄** |
| **I-5** | **H** | **H** | **H** | **H** | **4-MeO-C₆H₄** |
| **I-6** | **H** | **H** | **H** | **H** | **3,4-OCH₂O-C₆H₃** |
| **I-7** | **H** | **H** | **H** | **H** | **4-HO-C₆H₄** |
| **I-8** | **H** | **H** | **H** | **H** | ***n*-C₇H₁₅** |
| **I-9** | **H** | **H** | **H** | **H** | **Ph** |
| **I-10** | **H** | **H** | **H** | **H** | **4-Me-C₆H₄** |
| **I-11** | **H** | **H** | **H** | **H** | **3,4,5-(MeO)₃-C₆H₂** |
| **I-12** | **H** | **H** | **H** | **H** | **3,4-(HO)₂-C₆H₃** |
| **I-13** | **H** | **H** | **H** | **H** | **2,3,4-(HO)₃-C_{β}H₂** |
| **I-14** | **H** | **H** | **H** | **Me** | **2-MeO-C₆H₄** |
| **I-15** | **H** | **H** | **H** | **Me** | **3-MeO-C₆H₄** |
| **I-16** | **H** | **H** | **H** | **Me** | **4-MeO-C₆H₄** |
| **I-17** | **H** | **H** | **H** | **Me** | **4-F-C₆H₄** |
| **I-18** | **H** | **H** | **H** | **Me** | **4-HO-C₆H₄** |
| **I-19** | **H** | **H** | **H** | **Me** | ***n*-C₇H₁₆** |
| **I-20** | **H** | **H** | **H** | **Me** | **3,4,5-(MeO)₃-C₆H₂** |
| **I-21** | **H** | **H** | **H** | **Me** | **3,4-(HO)₂-C₆H₃** |
| **I-22** | **H** | **H** | **H** | **Me** | **2,3,4-(HO)₃-C₆H₂** |
| **I-23** | **Me** | **H** | **Me** | **H** | **Me** |
| **I-24** | **Me** | **H** | **Me** | **H** | **Ph** |
| **I-25** | **Me** | **H** | **Me** | **H** | **4-Cl-C₆H₄** |
| **I-26** | **Me** | **H** | **Me** | **H** | **4-Me-C₆H₄** |
| **I-27** | **Me** | **H** | **Me** | **H** | **4-MeO-C₆H₄** |
| **I-28** | **Me** | **H** | **Me** | **H** | **4-O₂N-C₆H₄** |
| **I-29** | **H** | **Me** | **H** | **H** | **Ph** |
| **I-30** | **H** | **Me** | **H** | **H** | **4-Cl-C₆H₄** |
| **I-31** | **H** | **Me** | **H** | **H** | **4-Me-C₆H₄** |
| **I-32** | **H** | **Me** | **H** | **H** | **4-MeO-C₆H₄** |
| **I-33** | **H** | **Me** | **H** | **H** | **4-O₂N-C₆H₄** |
| **I-34** | **H** | **Ph** | **H** | **H** | **Ph** |
| **I-35** | **H** | **Ph** | **H** | **H** | **4-Cl-C₆H₄** |
| **I-36** | **H** | **Ph** | **H** | **H** | **4-Me-C₆H₄** |
| **I-37** | **H** | **Ph** | **H** | **H** | **4-MeO-C₆H₄** |
| **I-38** | **H** | **Ph** | **H** | **H** | **4-O₂N-C₆H₄** |
| **I-39** | **H** | **4-Cl-C₆H₄** | **H** | **H** | **Ph** |
| **I-40** | **H** | **4-Cl-C₆H₄** | **H** | **H** | **4-Cl-C₆H₄** |
| **I-41** | **H** | **4-Cl-C₆H₄** | **H** | **H** | **4-Me-C₆H₄** |
| **I-42** | **H** | **4-Cl-C₆H₄** | **H** | **H** | **4-MeO-C₆H₄** |
| **I-43** | **H** | **4-Cl-C₆H₄** | **H** | **H** | **4-O₂N-C₆H₄** |

Methods for synthesizing hydrazinopurine compounds (I) represented by the general formula (I), hydrazinopurine compounds 3a, b, wherein R¹, R² and R³ are all hydrogen atom (Table 1, Compound IDs I-1 to I-22), can be synthesized according to the following reaction scheme (Scheme 1), while the method is not limited thereto:
(In Scheme 1, R⁴ represents hydrogen atom or methyl group; and R⁵ represents alkyl group or aryl group).

In the present specification, Ac represents an acetyl group, AcOH represents acetic acid, and TFA represents trifluoroacetic acid.

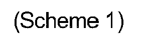

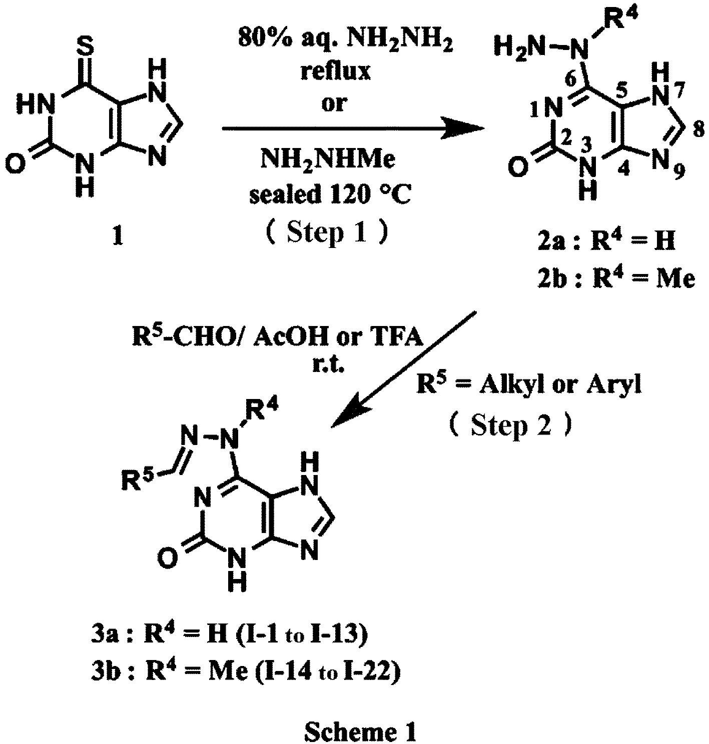

In brief, a compound represented by the formula 1 (referred to as compound 1, hereinafter) is reacted with hydrazine hydrate or methylhydrazine while heating to obtain a compound represented by the formula 2a, b (referred to as compound 2a, b, hereinafter) (Step 1). Subsequently, the compound 2a, b is reacted with various aldehydes to obtain a compound 3a, b represented by the general formula 3a, b (Step 2). Each step is now described.

### (Step 1)

This step is known; that is, the compound 2a, b can each be prepared according to the following known literatures: T. Nagamatsu, et al., J. Chem. Soc. Perkin Trans. 1, 3117 (1999), and T. Nagamatsu, et al., PCT Int. Appl. (1996), WO 9626208.

### (Step 2)

According to a known synthesis technique (T. Nagamatsu, et al., J. Chem. Soc. Perkin Trans. 1, 3117 (1999)), various purine aldehyde hydrazone compounds 3a, b can be prepared (Table 1, Compound IDs I-1 to I-22). It should be noted that compounds of Compound IDs I-1 to I-7 and I-14 to I-18 are known compounds, whereas compounds of Compound IDs I-8 to I-13 and I-19 to I-22 are novel compounds.

An aldehyde represented by R⁵-CHO (wherein R⁵ represents alkyl group or aryl group) (3.3 - 3.6 mmol) is used and the compound 2a, b (3 mmol) is reacted with the aldehyde in an organic solvent such as acetic acid and trifluoroacetic acid at 10 to 30°C for 30 min to obtain the compound 3a, b (Table 1, Compound IDs I-1 to I-22).

Of the hydrazinopurine compounds (I) represented by the general formula (I), hydrazinopurine compounds 6, wherein R¹ and R³ are both methyl group, and R² and R⁴ are both hydrogen atom, can be synthesized according to the following reaction scheme (Scheme 2), while the method is not limited thereto: 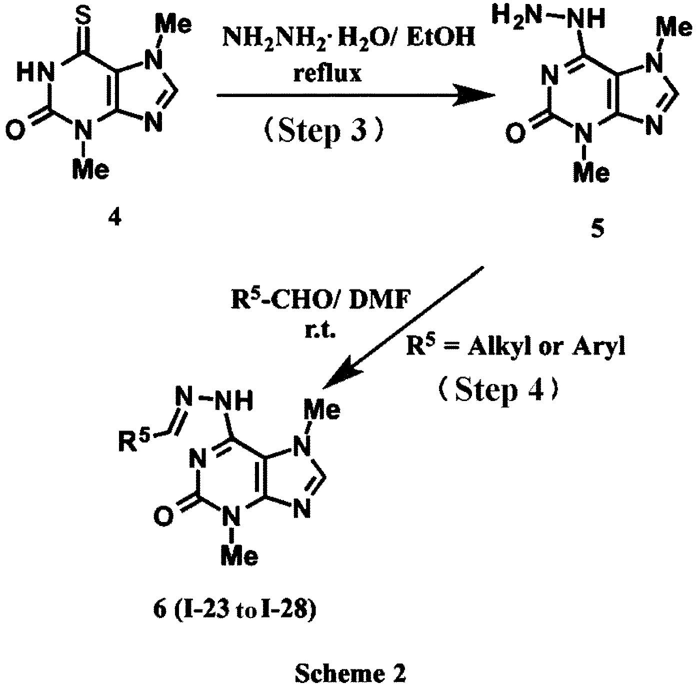 (In Scheme 2, R⁵ represents alkyl group or aryl group).

In the present specification, Et represents an ethyl group, EtOH represents ethanol, and DMF represents *N*,*N'*-dimethylformamide.

In brief, a compound 4 represented by the formula 4 can be derived from theobromine using a known synthesis technique (K. R. H. Wooldrige, et al, J. Chem. Soc., 1863 (1962)). Hydrazine hydrate (NH₂NH₂ • H₂O) can then be reacted with the compound 4 while heating to obtain a compound 5, a novel compound (Step 3). Subsequently, the compound 5 is reacted with various aldehydes to obtain a compound 6 represented by the general formula 6 (Step 4). Each step is now described.

### (Step 3)

In this step, the compound 5 can be prepared from the compound 4 according to a standard technique.

Hydrazine hydrate (4 mL) is added to the compound 4 (1 g) in ethanol and the mixture is heated to reflux for 30 min. Following the reaction, the precipitated crystals are collected by filtration and recrystallized from water to obtain a compound 5 as colorless needle-like crystals.

### (Step 4)

According to a synthesis technique similar to that described in Step 2, various novel purine aldehyde hydrazone compounds 6 can be prepared (Table 1, Compound IDs I-23 to I-28).

An aldehyde represented by R⁵-CHO (wherein R⁵ represents alkyl group or aryl group) (3.1 mmol) is used and the compound 5 (2.6 mmol) is reacted with the aldehyde in N,N-dimethylformamide (DMF) (40mL) at 10 to 30 °C for 0.5 to 2 hrs to obtain the compound 6 (Table 1, Compound IDs I-23 to I-28) (Scheme 2).

Of the hydrazinopurine compounds (I) represented by the general formula (I), hydrazinopurine compounds 10a-c, wherein R¹, R³ and R⁴ are all hydrogen atom, can be synthesized according to the following reaction scheme (Scheme 3), while the method is not limited thereto: 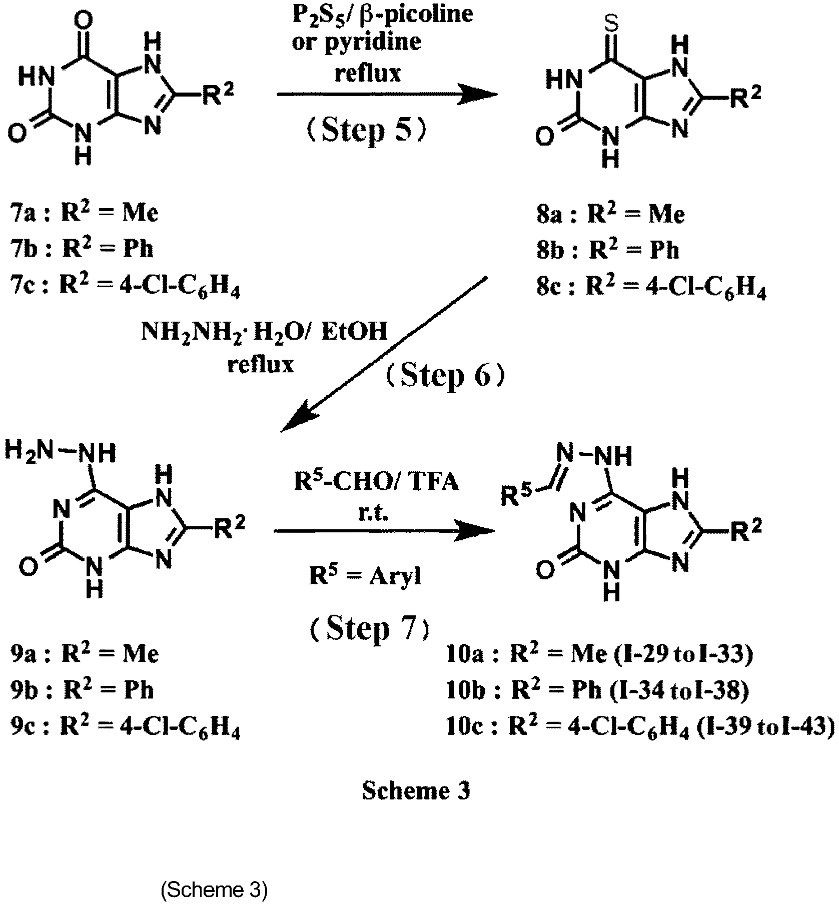 (In Scheme 3, R² represents alkyl group or aryl group, and R⁵ represents aryl group).

In brief, a compound 7a-c represented by the formula 7a-c (7a described in C. Henry, et al., J. Org. Chem., 23, 1457 (1958); 7b, c described in F. Yoneda, et al., Heterocycles. 4, 1759 (1976)) is reacted with diphosphorus pentasulfide while heating to obtain a compound 8a-c represented by the formula 8a-c (8a described in F. Bergmann, et al., J. Chem. Soc., 4468 (1961); 8b described in F. Bergmann, et al., J. Chem. Soc. (C), 1254 (1967)) (Step 5). Subsequently, the compound 8a-c is reacted with hydrazine hydrate to obtain a compound 9a-c represented by the general formula 9a-c (Step 6). Furthermore, the compound 9a-c can be reacted with various aldehydes to obtain a compound 10a-c represented by the general formula 10a-c (Step 7). Each step is now described.

### (Step 5)

In this step, the compound 8a-c can be prepared from the compound 7a-c according to a standard technique.

The compound 7a-c (15 mmol) and diphosphorus pentasulfide (45 mmol) are added to pyridine or β-picoline (200 ml) and the mixture is heated to reflux for 8 hrs. Following the reaction, the solvent is removed by evaporation under reduced pressure and the mixture is treated with hot water. The precipitated crystals are then collected by filtration. The product is treated with activated carbon in a mixed solvent of DMF and water and recrystallized from a mixed solvent of DMF and water to obtain the compounds 8a-c as yellow powdery crystals.

### (Step 6)

In this step, the compound 9a-c can be prepared from the compound 8a-c according to a standard technique.

Hydrazine hydrate (3-4 mL) is added to the compound 8a-c (1 g) in ethanol (3-4 mL) and the mixture is heated to reflux for 1 hr. Following the reaction, the precipitated crystals are collected by filtration and recrystallized from water to obtain a compound 9a-c as colorless needle-like crystals.

### (Step 7)

In this step, various purine aldehyde hydrazone compounds 10a-c can be prepared by the synthesis technique described in Step 2 (Table 1, Compound IDs I-29 to I-43).

The compound 9a-c (1.8 - 2.8 mmol) and various aldehydes (2.2 - 3.3 mmol) are added to TFA (8 mL) and each mixture is stirred at room temperature for 0.5 to 1 hr. Following the reaction, the solvent is removed by evaporation under reduced pressure and the mixture is treated with diethyl ether. The precipitated crystals are then collected by filtration. The product is washed with 1% aq. KHCOs and the resulting solid is recrystallized from a mixed solvent of DMF and water to obtain a compound 10a-c (Table 1, Compound IDs I-29 to I-43) (Scheme 3).

The triazolopurine compounds disclosed herein are represented by the general formulas (II) and (III), wherein R⁶ and R⁸ both represent hydrogen atom or alkyl group; R⁷ and R⁹ each independently represent hydrogen atom, alkyl group or aryl group; R¹⁰ and R¹² both represent alkyl group; R¹¹ represents hydrogen atom; and R¹³ represents hydrogen atom, methyl group or aryl group. Hereinafter, the hydrazinopurine compound represented by the general formula (II) and (III) defined above is denoted as triazolopurine compound (II) and (III).

Examples of the alkyl group represented by R⁶ to R⁹, R¹⁰, R¹² and R¹³ include straight-chained or branched lower alkyl group having 1 to 7 carbon atoms, such as methyl group, ethyl group, propyl group and butyl group.

Examples of the aryl group represented by R⁷, R⁹ and R¹³ include phenyl group and phenyl group having a substituent. The substituent of the phenyl group having a substituent may be halogen atom, alkyl group, alkoxy group, amino group, alkylamino group, methylenedioxy group, hydroxy group or nitro group with the number of the substituents being from 1 to 5. Specific examples of such aryl group include phenyl group, alkylphenyl group having C1 to C5 alkyl group, such as methylphenyl group and ethylphenyl group; an alkoxyphenyl group having C1 to C5 alkoxy group, such as methoxyphenyl group and ethoxyphenyl group; an alkylaminophenyl group having C1 to C5 alkylamino group, such as dimethylaminophenyl group and diethylaminophenyl group; halogenophenyl group, such as fluorophenyl group, chlorophenyl group, bromophenyl group and iodophenyl group; methylenedioxyphenyl group; hydroxyphenyl group; and nitrophenyl group.

Of the triazolopurine compounds (II) in one aspect of the present invention, preferred compounds in terms of the xanthine oxidase inhibition activity and the stability of the complex formed with xanthine oxidase are those that satisfy at least one of the following conditions:
(ii-1) R⁶, R⁷ and R⁸ are all hydrogen atom, and R⁹ is hydrogen atom, alkyl group or aryl group;
(ii-2) R⁶, R⁷ and R⁸ are all hydrogen atom, and R⁹ is C2 to C7 alkyl group or aryl group;
(ii-3) R⁶ and R⁸ are both alkyl group, R⁷ is hydrogen atom, and R⁹ is hydrogen atom, alkyl group or aryl group;
(ii-4) R⁶ and R⁸ are both alkyl group, R⁷ is hydrogen atom, and R⁹ is hydrogen atom, alkyl group, or a substituent represented by the following general formula (IV): 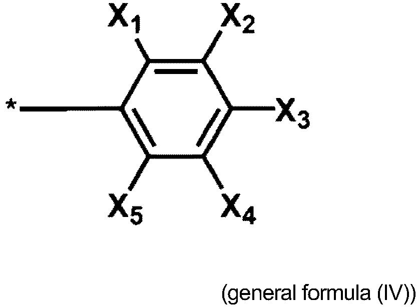 wherein X₁ to X₅ each independently represents a substituent selected from the group consisting of hydrogen atom, alkyl group, alkoxy group, amino group, alkylamino group, and hydroxy group;
(ii-5) R⁶ and R⁸ are both hydrogen atom, R⁷ is alkyl group or aryl group, and R⁹ is hydrogen atom, alkyl group or aryl group; or
(ii-6) R⁶ and R⁸ are both hydrogen atom, R⁷ is alkyl group or aryl group, and R⁹ is hydrogen atom, alkyl group, or a substituent represented by the following general formula (IV): 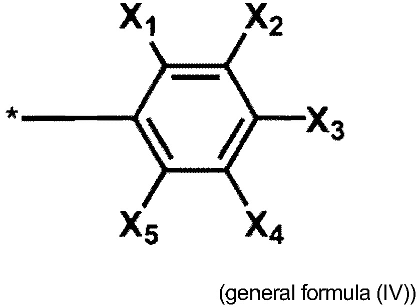 wherein X₁ to X₅ each independently represents a substituent selected from the group consisting of hydrogen atom, alkyl group, alkoxy group, amino group, alkylamino group, and hydroxy group.

In the triazolopurine compound (II) in one aspect of the present invention, R⁶ and R⁸ are preferably hydrogen atom, and R⁹ is preferably aryl group having π electrons and more preferably phenyl group or halogenphenyl group, in terms of the xanthine oxidase inhibition activity.

In the triazolopurine compound (II) in one aspect of the present invention, R⁶ and R⁸ are preferably both hydrogen atom, and R⁹ is preferably aryl group having π electrons and more preferably phenyl group, chlorophenyl group or alkoxyphenyl group, in terms of the stability of the complex formed with xanthine oxidase.

Examples of the triazolopurine compounds (II) and (III) disclosed in this application include compounds having a functional group presented in Tables 2 and 3.

**[table 2]**

| | | | | |
|---|---|---|---|---|
| 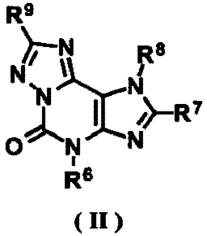 | | | | |

| **Compound ID** | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-3** | **H** | **H** | **H** | **Ph** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-C₆H₄** |
| **II-8** | **H** | **H** | **H** | **4-O₂N-C₆H₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-11** | **Me** | **H** | **Me** | **H** |
| **II-12** | **Me** | **H** | **Me** | **Me** |
| **II-13** | **Me** | **H** | **Me** | **Ph** |
| **II-14** | **Me** | **H** | **Me** | **4-Cl-C₆H₄** |
| **II-15** | **Me** | **H** | **Me** | **4-Me-C₆H₄** |
| **II-16** | **Me** | **H** | **Me** | **4-MeO-C₆H₄** |
| **II-17** | **Me** | **H** | **Me** | **4-O₂N-C₆H₄** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-C₆H₄** |
| **II-23** | **H** | **Me** | **H** | **4-MeO-C₆H₄** |
| **II-24** | **H** | **Me** | **H** | **4-O₂N-C₆H₄** |
| **II-25** | **H** | **Ph** | **H** | **H** |
| **II-26** | **H** | **Ph** | **H** | **Me** |
| **II-27** | **H** | **Ph** | **H** | **Ph** |
| **II-28** | **H** | **Ph** | **H** | **4-Cl-C₆H₄** |
| **II-29** | **H** | **Ph** | **H** | **4-Me-C₆H₄** |
| **II-30** | **H** | **Ph** | **H** | **4-MeO-C₆H₄** |
| **II-31** | **H** | **Ph** | **H** | **4-O₂N-C₆H₄** |
| **II-32** | **H** | **4-Cl-C₆H₄** | **H** | **H** |
| **II-33** | **H** | **4-Cl-C₆H₄** | **H** | **Me** |
| **II-34** | **H** | **4-Cl-C₆H₄** | **H** | **Ph** |
| **II-35** | **H** | **4-Cl-C₆H₄** | **H** | **4-Cl-C₆H₄** |
| **II-36** | **H** | **4-Cl-C₆H₄** | **H** | **4-Me-C₆H₄** |
| **II-37** | **H** | **4-Cl-C₆H₄** | **H** | **4-MeO-C₆H₄** |
| **II-38** | **H** | **4-Cl-C₆H₄** | **H** | **4-O₂N-C₆H₄** |

**[table 3]**

| | | | | |
|---|---|---|---|---|
| 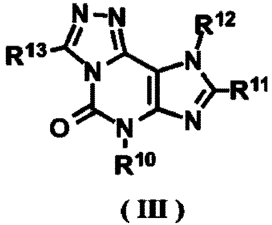 | | | | |

| **Compound ID** | **R¹⁰** | **R¹¹** | **R¹²** | **R¹³** |
|---|---|---|---|---|
| **III-1** | **Me** | **H** | **Me** | **H** |
| **III-2** | **Me** | **H** | **Me** | **Me** |
| **III-3** | **Me** | **H** | **Me** | **Ph** |
| **III-4** | **Me** | **H** | **Me** | **4-Cl-C₆H₄** |
| **III-5** | **Me** | **H** | **Me** | **4-Me-C₆H₄** |
| **III-6** | **Me** | **H** | **Me** | **4-MeO-C₆H₄** |
| **III-7** | **Me** | **H** | **Me** | **4-O₂N-C₆H₄** |

Of the triazolopurine compounds (II) represented by the general formula (II), triazolo [5,1-*i*] purine compounds 12, wherein R⁶, R⁷ and R⁸ are all hydrogen atom (Table 2, Compound IDs II-1 to II-10), can be synthesized according to the following reaction scheme (Scheme 4), while the method is not limited thereto: 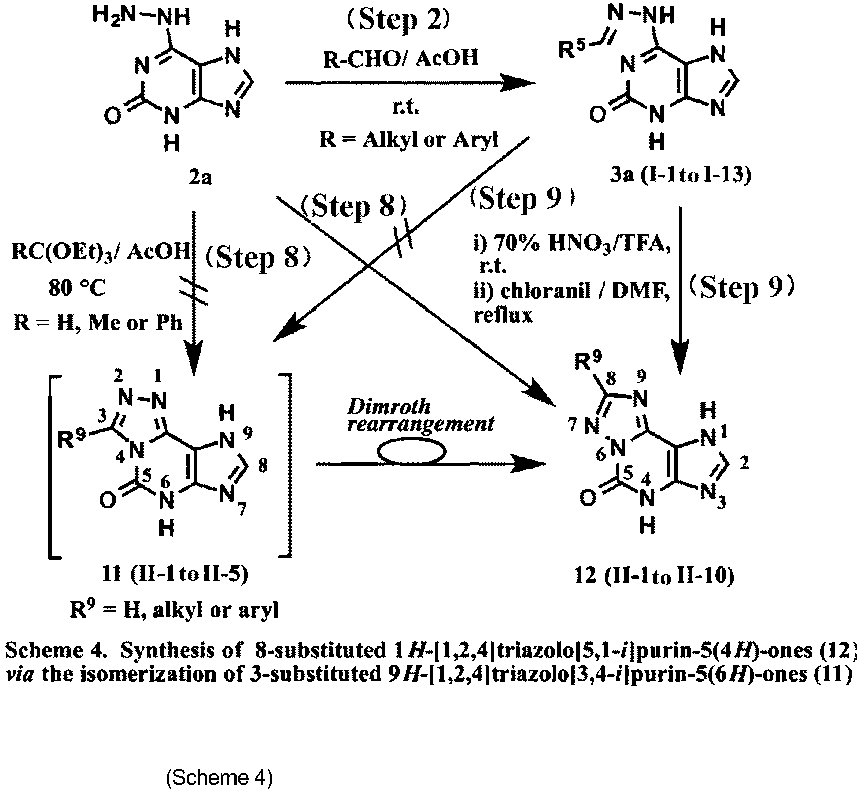 (In Scheme 4, R⁹ represents hydrogen atom, alkyl group or aryl group).

Previously, chemical structural formulas of triazolo [3,4-*i*] purine compounds 11 were reported as a condensation reaction product of compound 2a with an orthoester [RC(OEt)₃, wherein R = H, methyl group and phenyl group] and an oxidative ring-closing product of compound 3a (Table 1, Compound ID I-2, wherein R⁵ = 4-Cl-C₆H₄ and Compound ID I-5, wherein R⁵ = 4-MeO-C₆H₄) (J. Chem. Soc. Perkin Trans. 1, 3117 (1999) and PCT Int. Appl. (1996), WO9626208). However, subsequent close investigation of chemical reactions and various spectra of products, as well as alternative synthesis as described below that ensures structural reliability with high probability, had revealed that the true products were not triazolo [3,4-*i*] purine compounds 11, but were in fact triazolo [5,1-*i*] purine compounds 12 (Table 2, Compound IDs II-1 to II-5). That is, it was structurally determined that the first produced triazolo [3,4-*i*] purine compounds 11 (i.e., compounds of the general formula (II), wherein R⁶ and R⁸ are both hydrogen atom, or unsubstituted) were thermally unstable and thus undergo Dimroth rearrangement reaction to form a triazolo [5,1-*i*] purine compound 12 as a structural isomer. Specifically, compounds 12 represented by the general formula 12 can be obtained as a triazolo [5,1-*i*] purine compound 12 by adding an orthoester to a purine hydrazino derivative compound 2a, and heating the mixture to obtain a triazolo [5,1-*i*] purine compound 12 as a rearrangement product (Step 8). In order to stably form a triazolo [3,4-*i*] purine compound 11, a substituent such as methyl group can be introduced at the secondary amide at position 6 of compound 11 (R¹⁰ of the general formula (III)). In this manner, compound 11 can be formed stably without undergoing rearrangement (this will be described later). In addition, a purine aldehyde hydrazone compound 3a synthesized in Step 2 described above can be oxidized with nitric acid or chloranil to synthesize a triazolo [5,1-*i*] purine compound 12 as a rearrangement compound with various substituents introduced at R⁹ (R = alkyl group or aryl group) (Step 9). Each step is now described.

### (Step 8)

In this step, an orthoester can be added to the hydrazino compound 2a and the mixture can be heated to prepare a compound 12 (Table 2, Compound IDs II-1 to II-3).

To acetic acid (8 mL), 6-hydrazino-7*H*-purin-2(3*H*)-one compound 2a (1.2 mmol) and various orthoesters (4.8 mmol) are added and each mixture is heated for 10 to 20 min at 80 °C while being stirred. Following the reaction, the mixture is allowed to cool to room temperature and the precipitated crystals are collected by filtration and recrystallized from a mixed solvent of DMF and water to obtain a compound 12 (Table 2, Compound IDs II-1 to II-3).

### (Step 9)

In this step, a compound 3a can be oxidized with a suitable oxidizing agent to prepare compound 12 as a ring-closed product of compound 3a (Table 2, Compound IDs II-3 to II-10).
(Route i): To TFA (6 mL), 6-*n*-octylidenehydrazino-7*H*-purin-2(3*H*)-one or 6-arylmethylidenehydrazino-7*H*-purin-2(3*H*)-one compound 3a (1 mmol) and 70% nitric acid (1.5 mmol) are added and each mixture is stirred at room temperature for 10 min. Following the reaction, the solvent is removed by evaporation under reduced pressure and the mixture is treated with diethyl ether. The precipitated crystals are then collected by filtration. The product is washed with 1% aq. KHCOs and the resulting solid is recrystallized from a mixed solvent of DMF and water to obtain a compound 12 (Table 2, Compound IDs II-3 to II-10).
(Route ii): To DMF (20 mL), 6-arylmethylidenehydrazino-7*H*-purin-2(3*H*)-one compound 3a (1 mmol) and chloranil (1.5 mmol) are added and each mixture is heated to reflux for 10 min. Following the reaction, the solvent is removed by evaporation under reduced pressure and the mixture is treated with ethanol and diethyl ether. The precipitated crystals are then collected by filtration. The product is treated with activated carbon and recrystallized from a mixed solvent of DMF and water to obtain a compound 12 (Table 2, Compound IDs II-3 to II-10).

Of the triazolopurine compounds (III) represented by the general formula (III), triazolo [3,4-*i*] purine compounds 13, wherein R¹⁰ and R¹² are both methyl group (Table 3, Compound IDs III-1 to III-7), can be synthesized according to the following reaction scheme (Scheme 5), while the method is not limited thereto: 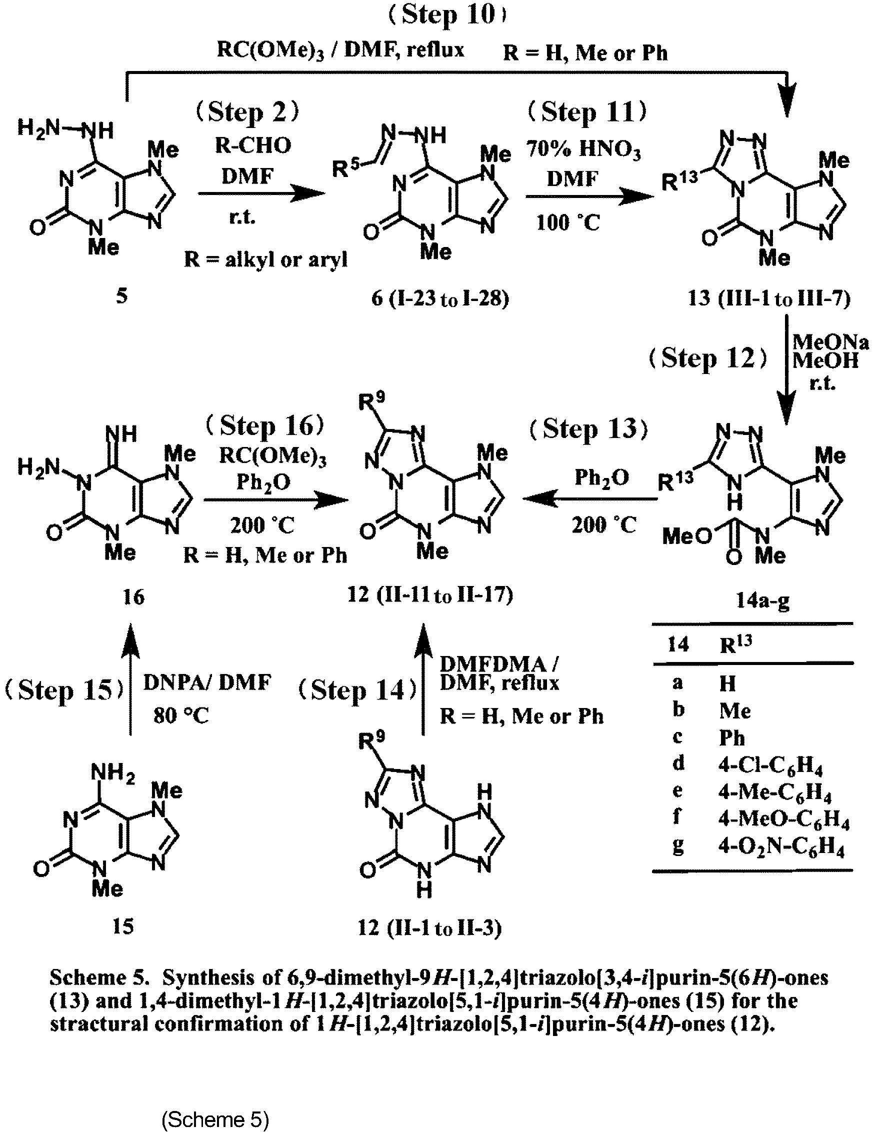 (In Scheme 5, R¹⁰ and R¹² both represent methyl group; R¹¹ represents hydrogen atom, and R¹³ represents hydrogen atom, methyl group or aryl group).

In the present specification, DMFDMA denotes *N*,*N'*-dimethylformamide dimethyl acetal, DNPA denotes *O*-(2,4-dinitrophenyl)hydroxylamine, and MeONaMeOH denotes a methanol solution of sodium methoxide.

Specifically, an orthoester is added to the 6-hydrazino-3,7-dimethyl-7*H*-purin-2(3*H*)-one compound 5 obtained in Step 3 described above and the mixture is heated. In this manner, the triazolo [3,4-*i*] purine compound 13 (Table 3, Compound IDs III-1 to III-3) can be obtained without undergoing rearrangement (Step 10). It is considered that the secondary amide position of compound 13 is *N-*methylated to a tertiary amide, which in turn stabilizes the backbone and makes the product less susceptible to rearrangement. Similarly, the triazolo [3,4-*i*] purine compound 13 (Table 3, Compound IDs III-2 to III-7) can be obtained in a stable manner at high yields by oxidizing the purine aldehyde hydrazone compound 6 obtained in Step 2 with nitric acid (Step 11). While the compound 13 is stable in a neutral solution even when heated, it is hydrolyzed at the tertiary amide moiety in a strong alkaline solution as shown in Step 12 to open the ring, forming compounds 14a-g (Step 12). Subsequently, the compound 14 can be heated in diphenyl ether to again close the ring and thus obtain 1 ,4-dimethyl compounds 12 of triazolo [5,1-*i*] purine (Table 2, Compound IDs II-11 to II-17), which are more stable rearranged isomers (Step 13). The 1,4-dimethyl compounds 12 (Table 2, Compound IDs II-11 to II-13) can be identified as compound 12 (Table 2, Compound IDs II-11 to II-13) resulting from methylation of the triazolo [5,1-*i*] purine compounds 12 obtained in Step 9 (Table 2, Compound IDs II-1 to II-3) with DMFDMA. Thus, it can be determined that the product in Step 9 is not the structure of the compound 11, but rather the compound 12, which is a rearranged isomer of the compound 11 (Table 2, Compound IDs II-1 to II-10) (Step 14). In alternative synthesis, 6-amino-3,7-dimethyl-7*H*-purin-2(3*H*)-one compound 15 can be aminated with *O*-(2,4-dinitrophenyl)hydroxylamine (DNPA) to 1-amino-6-imino-3,7-dimethyl-1,3,6,7-tetrahydro-2*H*-purin-2-one compound 16 (Step 15), which can then be reacted with orthoesters to obtain 1,4-dimethyl compounds 12 as rearranged compounds (Table 2, Compound IDs II-11 to II-13) (Step 16). Each step is now described.

### (Step 10)

In this step, an orthoester can be added to the hydrazinopurine compound 5 and the mixture can be heated to prepare a compound 13 (Table 3, Compound IDs III-1 to III-3).

To DMF (20 mL), 6-hydrazino-3,7-dimethyl-7*H*-purin-2(3*H*)-onecompound 5 (1.03 mmol) and various orthoesters (2.06 mmol) are added and each mixture is heated to reflux for 1 hr. Following the reaction, the solvent is removed by evaporation under reduced pressure and the mixture is treated with ethyl acetate. The precipitated crystals are then collected by filtration. The product is recrystallized from a mixed solvent of DMF and water to obtain a compound 13 (Table 3, Compound IDs III-1 to III-3).

### (Step 11)

In this step, the compound 6 (Table 1, Compound IDs I-23 to I-28) can be oxidized with a suitable oxidizing agent to close the ring and to thus prepare a compound 13 (Table 3, Compound IDs III-2 to III-7).

To DMF (30mL), 6-alkylidenehydrazino-3,7-dimethyl-7*H*-purin-2(3*H*)-one or 6-arylmethylidenehydrazino-3,7-dimethyl-7*H*-purin-2(3*H*)-one compound 6 (1 mmol) and 70% nitric acid (1.5 mmol) are added and each mixture is stirred at 100 °C for 0.5 to 1 hr. Following the reaction, the solvent is removed by evaporation under reduced pressure and the mixture is treated with water. The precipitated crystals are then collected by filtration. The product is recrystallized from a mixed solvent of DMF and water to obtain a compound 13 (Table 3, Compound IDs III-2 to III-7).

### (Step 12)

In this step, the compound 13 (Table 3, Compound IDs III-1 to III-7) can be hydrolyzed by an alkali to prepare a compound 14a-g as a ring-opened product of the compound 13.

To a 0.1 N methanol solution of sodium methoxide (10 to 15 mL), 6,9-dimethyl-9*H-*1,2,4-triazolo[3,4-*i*]purin-5(6*H*)-one compound 13 (Table 3, Compound IDs III-1 to III-7) (1 mmol) is added and each mixture is stirred at room temperature for 0.5 to 1 hr. Following the reaction, suspending materials are removed by filtration and the solvent is evaporated under reduced pressure. Water is then added, followed by 10% aq. HCI for neutralization to precipitate crystals. The product is collected by filtration and recrystallized from an appropriate organic solvent to obtain a compound 14a-g.

### (Step 13)

In this step, the above-described compound 14 a-g can be heated to close the ring and to thus prepare a compound 12 (Table 2, Compound IDs II-11 to !!-17).

To diphenyl ether (8 mL), the compound 14a-g (1 mmol) is added and each mixture is heated at 200 °C for 2 hrs while being stirred. Following the reaction, the mixture is allowed to cool to room temperature and the precipitated crystals are collected by filtration. The product can then be washed with diethyl ether and recrystallized from DMF to obtain a compound 12 (Table 2, Compound IDs II-11 to II-17).

### (Step 14)

This step is to confirm the structure of the compound 12 (II) obtained in the above-described Step 9. Specifically, the compound 12 (Table 2, Compound IDs II-11 to II-13) obtained by methylation of the compound 12 (Table 2, Compound IDs II-1 to II-3) with DMFDMA can be identified as the compound 12 obtained from Step 13 (Table 2, Compound IDs II-11 to II-13).

To DMF (10mL), a corresponding 1*H*-[1,2,4]triazolo[5,1-*i*]purin-5(4*H*)-one compound 12 (Table 2, Compound IDs II-1 to II-3) (1 mmol) and *N*,*N'*-dimethylformamide dimethyl acetal (DMFDMA) (10 mmol) are added and each mixture is heated to reflux for 2 to 3 hrs. Following the reaction, the solvent is removed by evaporation under reduced pressure and the mixture is treated with ethyl acetate, and the precipitated crystals are collected by filtration. The product is recrystallized from DMF to obtain a compound 12 (Table 2, Compound IDs II-11 to II-13).

### (Step 15)

This step is to confirm the structure of the compound 12 and to synthesize a compound 16 as a starting material for an alternative synthesis method.

To DMF (100 mL), a known compound 6-amino-2,3-dihydro-3,7-dimethyl-2-oxo-7*H-*purine compound 15 (1.12 mmol) and *O*-(2,4-dinitrophenyl)hydroxylamine (DNPA) (1.68 mmol) are added and the mixture is heated at 80°C for 1 hr while being stirred. Following the reaction, the solvent is evaporated under reduced pressure and the residue is separated and purified through silica gel column chromatography (Kieselgel 70-230 mesh) (ethyl acetate: ethanol = 4:1). The resulting solid is recrystallized from ethanol to obtain 1-amino-1,2,3,6-tetrahydro-6-imino-3,7-dimethyl-2-oxo-7*H-*purine compound 16.

### (Step 16)

In this step, orthoformate can be added to the compound 16 obtained in Step 15 and the mixture can be heated to prepare a compound 12 (Table 2, Compound IDs II-11 to II-13).

To diphenyl ether (8 mL), 1-amino-1,2,3,6-tetrahydro-6-imino-3,7-dimethyl-2-oxo-7*H-*purine compound 16 (1.03 mmol) and various orthoesters (3.09 mmol) are added and each mixture is heated at 200°C for 20 min while being stirred. Following the reaction, the mixture is allowed to cool to room temperature and the precipitated crystals are collected by filtration. The product can then be washed with diethyl ether and recrystallized from DMF to obtain a compound 12 (Table 2, Compound IDs II-11 to II-13).

Of the triazolopurine compounds (II) represented by the general formula (II), triazolo [5,1-*i*] purine compounds 17a-c, wherein R⁶ and R⁸ are both hydrogen atom, and R⁷ is methyl, phenyl or 4-Cl-C₆H₄ (Table 2, Compound IDs II-18 to II-38), can be synthesized according to the following reaction scheme (Scheme 6), while the method is not limited thereto: 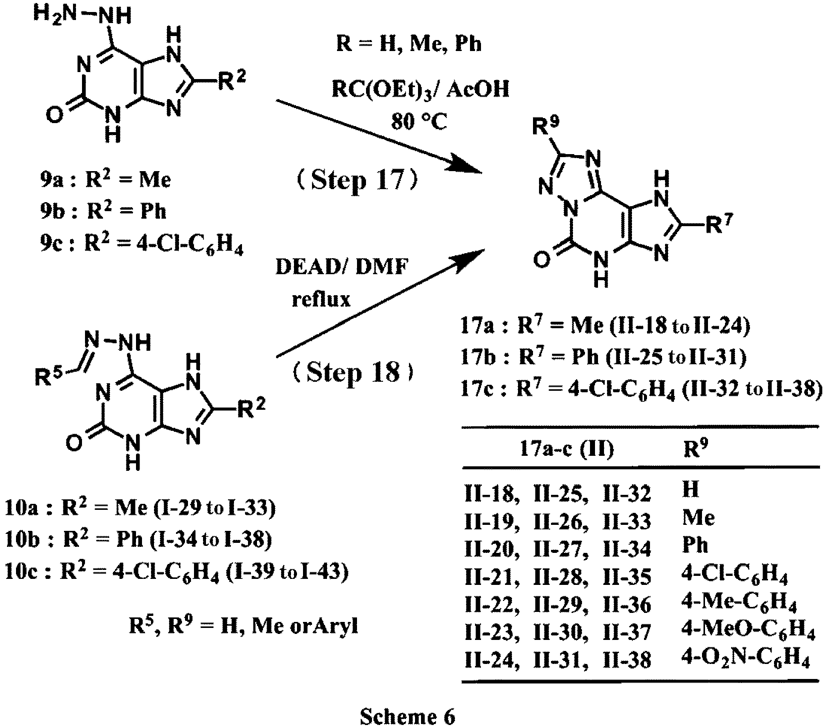 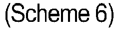 (In Scheme 6, R⁹ represents hydrogen atom, methyl group or aryl group).

In the present specification, DEAD denotes diethyl azodicarboxylate.

Specifically, an orthoester is added to the hydrazinopurine derivative compounds 9a-c obtained in Step 6 described above and each mixture is heated to obtain a triazolo [5,1-*i*] purine compound 17 (Table 2, Compound IDs II-18 to II-20, Compound IDs II-25 to II-27, and Compound IDs II-32 to II-34) as a rearranged compound (Step 17).

Further, diethylazodicarboxylate as an oxidizing agent can be added to the purine aldehyde hydrazone derivative compounds 10a-c obtained in Step 7 and the mixture can be heated to bring about ring-closed rearrangement to form compounds 17a-c (Table 2, Compound IDs II-20 to II-24, Compound IDs II-27 to II-31, and Compound IDs II-34 to II-38) (Step 18). Each step is now described.

### (Step 17)

In this step, orthoesters can be added to the hydrazinopurine compounds 9a-c and the mixtures can be heated to prepare compounds 17a-c (Table 2, Compound IDs II-18 to II-20, II-25 to II-27, and II-32 to II-34).

To acetic acid (5 to 8 mL), the hydrazinopurine compounds 9a-c (0.7 to 1.1 mmol) and various orthoesters (2.2 to 3.3 mmol) are added and each mixture is heated for 10 min at 80°C while being stirred. Following the reaction, the mixture is allowed to cool to room temperature and the precipitated crystals are collected by filtration. The product is recrystallized from a mixed solvent of DMF and water to obtain compounds 17a-c.

### (Step 18)

In this step, the purine aldehyde hydrazone compounds 10a-c are oxidized with an appropriate oxidizing agent to prepare their corresponding ring-closed compounds 17a-c (Table 2, Compound IDs II-20 to II-24, Compound IDs II-27 to II-31, and Compound IDs II-34 to II-38).

Specifically, to DMF (15 to 20 mL), 6-arylmethylidenehydrazino-8-methyl-7*H*-purin-2(3*H*)-one compounds 10a-c (Table 1, Compound IDs I-29 to I-43) (1 mmol) and DEAD (2 mmol) are added and each mixture is heated to reflux for 1 to 2 hrs. Following the reaction, the solvent is concentrated under reduced pressure and the mixture is chilled on ice. The precipitated crystals are then collected by filtration. The product is recrystallized from a mixed solvent of DMF and water to obtain compounds 17a-c.

Of the triazolopurine compounds (II) represented by the general formula (II), triazolo [5,1-*i*] purine compounds 17b, c, wherein R⁶ and R⁸ are both hydrogen atom, and R⁷ is phenyl or 4-CI-C₆H₄ (Table 2, Compound IDs II-25 to II-27 and Compound IDs II-32 to II-34), can be synthesized in an alternative structurally reliable production method according to the following reaction scheme (Scheme 7), while the method is not limited thereto: 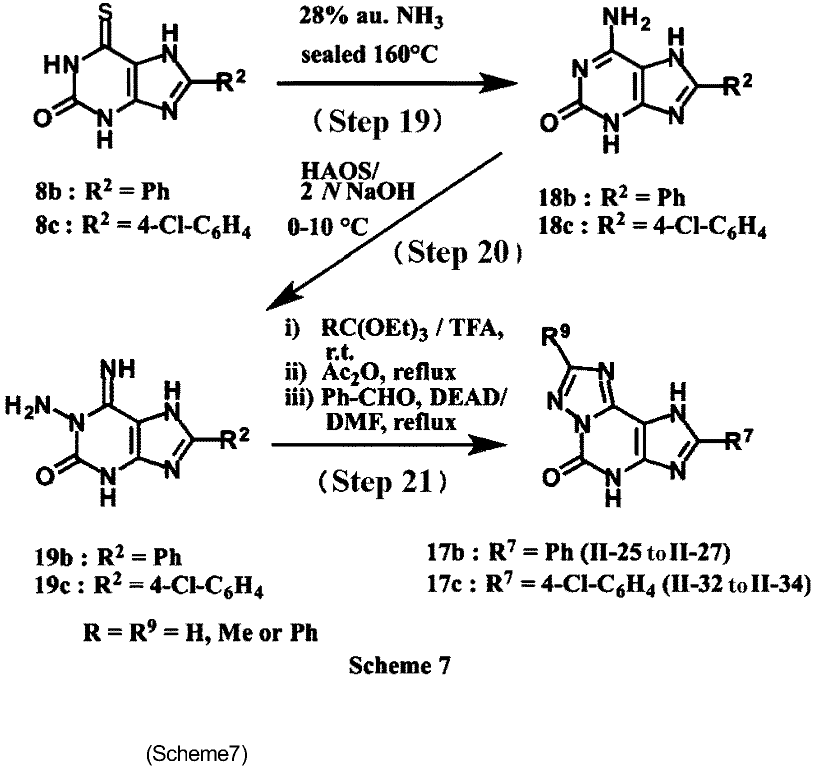 (In Scheme 7, R⁹ represents hydrogen atom, methyl group or phenyl).

In the present specification, HAOS denotes hydroxylamine-O-sulfonic acid.

Specifically, an aqueous ammonium solution can be added to thioxopurine compounds 8b, c and the mixture can be heated in a sealed tube to obtain their corresponding aminated compounds 18b, c (Step 19). Subsequently, HAOS can be added to the compounds 18b, c in an aqueous alkaline solution to aminate the compounds 18b, c to obtain 1-amino-6-iminopurine compounds 19b, c (Step 20). Furthermore, the compounds 19b, c can be reacted with various orthoesters to obtain compounds 17b, c (Step 21). In addition to this, certain derivatives may be similarly heated in anhydrous acetic acid or benzaldehyde may be added and oxidized with DEAD while heating to obtain similar products. Each step is now described.

### (Step 19)

In this step, thioxopurine compounds 8b, c (8 mmol) are added to 28% aqueous ammonia (50 mL) and the mixture is heated in a sealed tube at 160 °C for 48 hrs. Following the reaction, the mixture is chilled on ice and the precipitated crystals are collected by filtration. The product is washed with water and ethanol to obtain compounds 18b, c as colorless powdery crystals.

### (Step 20)

In this step, amino compounds 18b, c (2 mmol) are dissolved in 2N NaOH (15 mmol) and Hydroxylamine-*O*-sulfonic acid (HAOS) (7.6 to 8.6 mmol) dissolved in 3 mL water is then added at 0 to 10°C. After the reaction, the precipitated crystals are collected by filtration, dissolved in water, and neutralized with 10% aq. HCI to precipitate crystals. The products are then recrystallized from ethanol to obtain 1-amino-6-imino compounds 19b, c as colorless powdery crystals.

### (Step 21)

In this step, triazolo [5,1-*i*] purine compounds 17b, c (Table 2, Compound IDs II-25 to II-27 and Compound IDs II-32 to II-34) can be synthesized via the following three routes as alternative structurally reliable synthesis methods of triazolo [5,1-*i*] purines.
(Route i): To TFA (4 mL), 8-substituted 1-amino-1,2,3,6-tetrahydro-6-imino-2-oxo-7*H-*purine compounds 19b, c (0.8 mmol) and an orthoester (4 mmol) are added and each mixture is stirred at room temperature for 3 hrs. Following the reaction, the solvent is removed by evaporation under reduced pressure and the mixture is treated with diethyl ether. The precipitated crystals are then collected by filtration. The product is washed with 0.5% aq. KHCOs and the resulting solid is recrystallized from a mixed solvent of DMF and ethanol to obtain compounds 17b, c as colorless powdery crystals (Table 2, Compound IDs II-25 to II-32).
(Route ii): To anhydrous acetic acid (4 mL), 8-substituted 1-amino-1,2,3,6-tetrahydro-6-imino-2-oxo-7*H*-purine compounds 19b, c (0.8 mmol) are added and each mixture is heated to reflux for 3 hrs. Following the reaction, the mixture is allowed to cool to room temperature and the precipitated crystals are collected by filtration. The products can be recrystallized from a mixed solvent of DMF and ethanol to obtain compounds 17b, c as colorless powdery crystals (Table 2, Compound IDs II-26 to II-33).
(Route iii): To DMF (15 mL), 8-substituted 1-amino-1,2,3,6-tetrahydro-6-imino-2-oxo-7H purine compounds 19b, c (0.8 mmol) and benzaldehyde (1.2 mmol) are added and each mixture is heated to reflux for 30 min. Subsequently, DEAD (1.25 mmol) is added and the mixture is heated to reflux for 1 hr. Following the reaction, the solvent is concentrated under reduced pressure and the mixture is treated with water. The precipitated crystals are then collected by filtration. The products are recrystallized from a mixed solvent of DMF and ethanol to obtain compounds 17b, c as colorless powdery crystals (Table 2, Compound IDs II-27 to II-34).

The triazolopurine compounds (II) of the present invention and a synthetic intermediate may be isolated/purified by standard isolation/purification means for nucleic acid bases: for example, recrystallization and various chromatography techniques may be used for isolation/purification.

The triazolopurine compounds (II) of the present invention may be any of a free form, a salt or a hydrate (including a hydrate salt). Examples of the salt include salts of inorganic acids, such as hydrochloride, sulfate and hydrobromide, salts of organic acids, such as oxalate, citrate and malate, or ammonium salts. In particular, pharmaceutically acceptable salts are preferred.

A xanthine oxidase inhibitory composition in one aspect of the present invention is a xanthine oxidase inhibitory composition containing as an active ingredient at least one compound selected from the group consisting of the above-described triazolopurine compounds (II) of the first aspect of the invention.

A pharmaceutical composition in one aspect of the present invention is a pharmaceutical composition containing as an active ingredient at least one compound selected from the group consisting of the above-described triazolopurine compounds (II) according to the first aspect of the invention. The pharmaceutical composition in one aspect of the present invention is a pharmaceutical composition selected from the group consisting of hyperuricemia, gout, kidney disorders such as kidney failure and chronic kidney disease, hypertension, cardiovascular disease, dyslipidemia and metabolic syndrome.

The triazolopurine compounds (II) of the present invention have xanthine oxidase inhibition activity and can thus inhibit uric acid synthesis. Thus, the compounds are suitable for use in a pharmaceutical composition for lowering uric acid levels in blood and preventing or treating hyperuricemia or in a pharmaceutical composition for preventing or treating urate deposition diseases, including gout, gouty arthritis and gouty nodes caused by hyperuricemia.

Xanthine oxidase is one of the major causes of oxidative stress produced in the body and is involved in the production of reactive oxygen species especially in tissue damaged by conditions such as ischemia or tissue invasion. The reactive oxygen species formed when excess uric acid causes excess xanthine oxidase activity is responsible for the impairment of various cellular functions.

On the other hand, hyperuricemia promotes activation of transporter molecules that act as uric acid transporters. Since uric acid transporters are expressed on various cells including cells forming blood vessels such as adipocytes and vascular smooth muscle cells, large amounts of uric acid are taken up by various cells in hyperuricemia. For example, as uric acid is taken up by adipocytes and vascular smooth muscle cells, reactive oxygen species causes inflammation as described above. Inflammation of adipocytes causes aberrant adipose tissue. Inflammation of vascular endothelium and vascular smooth muscle cells also causes renal vascular lesions and further causes systemic hypertension and glomerular hypertension, leading to the onset of renal disorders and progression of renal diseases.

Accordingly, the triazolopurine compounds (II) of the present invention are suitable for use in a pharmaceutical composition for preventing or treating kidney disorders such as kidney failure and chronic kidney disease, hypertension, cardiovascular disease, dyslipidemia and metabolic syndrome.

Administration to human may be via any route such as oral, enteral, and parenteral for preventing or treating the above-described diseases. While the dose may be suitably determined depending on the age, condition and body weight of the patients, it is typically chosen from a range from 0.01 to 100 mg/kg body weight per day and is administered in a single dose or multiple doses.

When the compound of the present invention is used to produce a pharmaceutical composition, it is suitably used in a pharmaceutical composition containing a pharmaceutically acceptable carrier, such as excipient, or other additives. Examples of carriers include solid carriers, such as lactose, kaolin, sucrose, crystalline cellulose, corn starch, talc, agar, pectin, stearic acid, magnesium stearate, lecithin, and sodium chloride; and liquid carriers, such as glycerin, peanut oil, polyvinylpyrrolidone, olive oil, ethanol, benzyl alcohol, propylene glycol and water.

The pharmaceutical composition may take any dosage form: examples include tablets, powders, granules, capsules, suppositories, troches for solid carriers, and syrups, emulsions, soft gelatin capsules, creams, gels, pastes, injections for liquid carriers.

The present invention will now be described more specifically with reference to Examples, which are not intended to limit the present invention in any way.

### [Example 1. Synthesis Examples of Hydrazinopurine Compounds]

According to the reactions described in Scheme 1 described above, hydrazinopurine compounds denoted as compounds 3a (Table 1, Compound IDs I-1 to I-13. For Compound IDs, reference is made to Tables 1 to 3 above, hereinafter) and compounds 3b (Compound IDs I-14 to I-22) were synthesized (For R⁵ in Scheme 1, refer to Table 1.).

### Synthesis Example 1: Synthesis of 6-n-octylidenehydrazino-7H-purin-2(3H)-one compound 3a (Compound ID I-8: R⁵ = n-C₇H₁₅)

To TFA (10 mL), 6-hydrazino-7*H*-purin-2(3 H)-one compound 2a (0.50 g, 3.0 mmol) and octanal (0.42 g, 3.3 mmol) were added and the mixture was stirred at room temperature for 30 min. Following the reaction, the solvent was removed by evaporation under reduced pressure and the mixture was treated with water. The precipitated crystals were then collected by filtration. The product was washed with 1 % aq. KHCOs and the resulting solid was recrystallized from ethanol to obtain a compound 3a as colorless powdery crystals (Compound ID I-8) (Tables 4 and 5). Synthesis Example 2: General synthesis of 6-arylmethylidenehydrazino-7*H*-purin-2(3*H*)-one compounds 3a (Compound IDs I-9 to I-13: R⁵ = aryl group)

To TFA (10 mL), 6-hydrazino-7*H*-purin-2(3*H*)-one compound 2a (0.50 g, 3.0 mmol) and various aldehydes (3.6 mmol) were added and each mixture was stirred at room temperature for 30 min. Following the reaction, the solvent was removed by evaporation under reduced pressure and the mixture was treated with diethyl ether. The precipitated crystals were then collected by filtration. The product was washed with 1% aq. KHCO₃ and the resulting solid was recrystallized from a mixed solvent of DMF and water to obtain a compound 3a (Compound IDs I-9 to I-13) (Tables 4 and 5).

### Synthesis Example 3: Synthesis of 6-(1-methyl-2-n-octylidenehydrazino)-7H-purin-2(3H)-one compound 3b (Compound ID I-19: R⁵ = n-C₇H₁₅)

To TFA (10 mL), 6-(1-methylhydrazino)-7*H*-purin-2(3*H*)-one compound 2b (0.50 g, 2.78 mmol) and octanal (0.39 g, 3.06 mmol) were added and the mixture was stirred at room temperature for 30 min. Following the reaction, the solvent was removed by evaporation under reduced pressure and the mixture was treated with water. The precipitated crystals were then collected by filtration. The product was washed with 1% aq. KHCOs and the resulting solid was recrystallized from ethanol to obtain a compound 3b as colorless powdery crystals (Compound ID I-19) (Tables 4 and 5).

### Synthesis Example 4: General synthesis of 6-(2-arylmethylidene-1-methylhydrazino)-7H-purin-2(3H)-one compounds 3b (Compound IDs I-20 to I-22: R⁵ = aryl group)

To TFA (10 mL), 6-(1-methylhydrazino)-7*H*-purin-2(3*H*)-one compound 2b (0.50 g, 2.78 mmol) and various aldehydes (3.34 mmol) were added and each mixture was stirred at room temperature for 30 min. Following the reaction, the solvent was removed by evaporation under reduced pressure and the mixture was treated with diethyl ether. The precipitated crystals were then collected by filtration. The product was washed with 1% aq. KHCOs and the resulting solid was recrystallized from a mixed solvent of DMF and water to obtain a compound 3b (Compound IDs I-20 to I-22) (Tables 4 and 5).

According to the reactions described in Scheme 2 described above, hydrazinopurine compounds denoted as compounds 6 (Compound IDs I-23 to I-28) were synthesized (For R⁵ in Scheme 2, refer to Table 1.).

### Synthesis Example 5: Synthesis of 6-hydrazino-3,7-dimethyl-7H-purin-2(3H)-one compound 5

To ethanol (4 mL), 1,2,3,6-tetrahydro-3,7-dimethyl-2-oxo-6-thioxo-7*H*-purine compound 4 (1 g, 5.1 mmol) and hydrazine hydrate (4 mL, 117 mmol) were added and the mixture was heated to reflux for 30 min. Following the reaction, the precipitated crystals were collected by filtration and recrystallized from water to obtain 0.73 g (74% yield) of a compound 5 as colorless needle-like crystals.
¹H-NMR [200 MHz, (CD₃)₂SO] δ: 3.23 (3H, s, 3-Me), 3.76 (3H, s, 7-Me), 6.68 (3H, br, exchangeable with D₂O, 6-N*H*N*H*₂), 7.60 (1H, s, 8-H); IR: 3260 (νₐₛ, NH₂), 3190 (νₛ, NH₂), 3110 (v, NH), 1690 (v, C=O), 1640 cm⁻¹(δ, NH₂); *Anal.* Calcd. for C₇H₁₀N₆O·1/10 H₂O: C, 42.90; H, 5.25; N, 42.88 Found: C, 42.67; H, 5.37; N, 43.08; MS (FAB, glycerol matrix): *m*/*z* = 195 (MH⁺).

### Synthesis Example 6: General synthesis of 6-alkylidenehydrazino-3,7-dimethyl-7H-purin-2(3H)-one or 6-arylmethylidenehydrazino-3,7-dimethyl-7H-purin-2(3H)-one compounds 6 (Compound IDs I-23 to I-28)

To DMF (40 mL), 6-hydrazino-3,7-dimethyl-7*H*-purin-2(3*H*)-onecompound 5 (0.50 g, 2.57 mmol) and various aldehydes (3.08 mmol) were added and each mixture was stirred at room temperature for 0.5 to 2 hrs. Following the reaction, the solvent was removed by evaporation under reduced pressure and the mixture was treated with ethyl acetate. The precipitated crystals were then collected by filtration. The product was recrystallized from ethanol or a mixed solvent of DMF and water to obtain a compound 6 (Compound IDs I-23 to I-28) (Tables 6 and 7).

According to the reactions described in Scheme 3 described above, hydrazinopurine compounds denoted as compounds 10a-c (Compound IDs I-29 to I-43) were synthesized (For R² and R⁵ in Scheme 3, refer to Table 1.).

### Synthesis Example 7: Synthesis of 8-(4-chlorophenyl)-1,2,3,6-tetrahydro-2-oxo-6-thioxo-7H-purine compound 8c (R² = 4-Cl-C₆H₄)

To pyridine (200 mL), 8-(4-chlorophenyl)-1,2,3,6-tetrahydro-2,6-dioxo-7*H*-purine compound 7c (4 g, 15.2 mmol) and phosphorus pentasulfide (10 g, 45.6 mmol) were added and the mixture was heated to reflux for 8 hrs. Following the reaction, the solvent was removed by evaporation under reduced pressure and the mixture was treated with hot water. The precipitated crystals were then collected by filtration. The product was treated with activated carbon and recrystallized from a mixed solvent of DMF and water to obtain a compound 8a as yellow powdery crystals (3.5 g, 82 %, mp > 330°C).
¹H-NMR [300 MHz, (CD₃)₂SO] δ: 7.58 (2H, d, *J*_{AB} = 8.4 Hz, Ar-*m*H), 8.20 (2H, d, *J*_{AB} = 8.4 Hz, Ar-*o*H), 12.05 (1H, s, exchangeable with D₂O, 1-NH), 12.25 (1H, s, exchangeable with D₂O, 3-NH), 13.68 (1H, s, exchangeable with D₂O, 7-NH); IR: 3180 sh, 3100 sh (v, NH), 1720 cm⁻¹(ν, C=O); *Anal.* Calcd. for C₁₁H₇ClN₄OS·1/2 H₂O: C, 45.92; H, 2.80; N, 19.47 Found: C, 45.82; H, 2.79; N, 19.72; MS (FAB, glycerol matrix): *m*/*z* = 279 (MH⁺), 281 (MH⁺+2).

### Synthesis Example 8: Synthesis of 6-hydrazino-8-methyl-7H-purin-2(3H)-one compound 9a (R² = methyl group)

To ethanol (3 mL), 1 ,2,3,6-tetrahydro-8-methyl-2-oxo-6-thioxo-7*H*-purine compound 8a (1 g, 5.49 mmol) and hydrazine hydrate (3 mL, 85.6 mmol) were added and the mixture was heated to reflux for 1 hr. Following the reaction, the precipitated crystals were collected by filtration and the product was washed with water and ethanol to obtain a compound 9a as colorless powdery crystals (0.66 g, 67 %, mp 285°C (decomposed)). Although the elemental analysis as a measure of purity could not be performed since the product was poorly soluble in a general-purpose solvent and could not be subjected to recrystallization, TLC and ¹H-NMR spectra confirmed that the product was a single compound.
¹H-NMR [300 MHz, CF₃COOD] δ: 2.91 (3H, s, 8-Me); IR: 3330 (νₐₛ, NH₂), 3180 sh (νₛ, NH₂), 3140 (v, NH), 1670 (v, C=O), 1650 cm⁻¹ (δ, NH₂); MS (FAB, glycerol matrix): *m*/*z* = 181 (MH).

### Synthesis Example 9: Synthesis of 6-hydrazino-8-phenyl-7H-purin-2(3H)-one compound 9b (R² = phenyl)

To ethanol (4 mL), 1,2,3,6-tetrahydro-2-oxo-8-phenyl-6-thioxo-7*H*-purine compound 8b (1 g, 4.09 mmol) and hydrazine hydrate (4 mL, 114 mmol) were added and the mixture was heated to reflux for 1 hr. Following the reaction, the precipitated crystals were collected by filtration and the product was washed with water and ethanol to obtain a compound 9b as colorless powdery crystals (0.65 g, 66%, mp 280°C (decomposed)). Although the elemental analysis as a measure of purity could not be performed since the product was poorly soluble in a general-purpose solvent and could not be subjected to recrystallization, TLC and ¹H-NMR spectra confirmed that the product was a single compound.
¹H-NMR [300 MHz, CF₃COOD] δ: 7.65-7.78 (2H, m, Ph-*m*H), 7.80-7.88 (1H, m, Ph-*p*H), 7.96-8.08 (2H, m, Ph-*o*H); IR: 3320 (νₐₛ, NH₂), 3180 sh (νₛ, NH₂), 3070 sh (v, NH), 1680 (v, C=O), 1650 cm⁻¹ (δ, NH₂); MS (FAB, glycerol matrix): *m*/*z* = 243 (MH⁺).

### Synthesis Example 10: Synthesis of 8-(4-chlorophenyl)-6-hydrazino-7H-purin-2(3H)-one compound 9c (R² = 4-Cl-C₆H₄)

To ethanol (4 mL), 8-(4-chlorophenyl)-1,2,3,6-tetrahydro-2-oxo-6-thioxo-7*H*-purine compound 8c (1 g, 3.59 mmol) and hydrazine hydrate (4 mL, 114 mmol) were added and the mixture was heated to reflux for 1 hr. Following the reaction, the precipitated crystals were collected by filtration and the product was washed with water and ethanol to obtain a compound 9c as colorless powdery crystals (0.60 g, 60%, mp 290°C (decomposed)). Although the elemental analysis as a measure of purity could not be performed since the product was poorly soluble in a general-purpose solvent and could not be subjected to recrystallization, TLC and ¹H-NMR spectra confirmed that the product was a single compound.
¹H-NMR [300 MHz, CF₃COOD] δ: 7.69 (2H, d, *J*_{AB} = 7.5 Hz, Ar-*m*H), 7.94 (2H, d, *J*_{AB} = 7.5 Hz, Ar-*o*H); IR: 3320 (νₐₛ, NH₂), 3180 sh (νₛ, NH₂), 3070 sh (v, NH), 1670 (v, C=O), 1630 cm⁻¹ (δ, NH₂); MS (FAB, glycerol matrix): *m*/*z* = 277 (MH⁺), 279 (MH⁺+2).

### Synthesis Example 11: General synthesis of 6-arylmethylidenehydrazino-8-methyl-7H-purin-2(3H)-one compounds 10a (R² = Me) (Compound IDs I-29 to I-33)

To TFA (8 mL), 6-hydrazino-8-methyl-7*H*-purin-2(3*H*)-one compound 9a (0.50 g, 2.77 mmol) and various aldehydes (3.32 mmol) were added and each mixture was stirred at room temperature for 30 min. Following the reaction, the solvent was removed by evaporation under reduced pressure and the mixture was treated with diethyl ether. The precipitated crystals were then collected by filtration. The product was washed with 1% aq. KHCOs and the resulting solid was recrystallized from a mixed solvent of DMF and water to obtain a compound 10a (Compound IDs I-29 to I-33) (Tables 8 and 9).

### Synthesis Example 12: General synthesis of 6-arylmethylidenehydrazino-8-phenyl-7H-purin-2(3H)-one compounds 10a (R² = phenyl) (Compound IDs I-34 to I-38)

To TFA (8 mL), 6-hydrazino-8-phenyl-7*H*-purin-2(3*H*)-one compound 10b (0.50 g, 2.06 mmol) and various aldehydes (2.47 mmol) were added and each mixture was stirred at room temperature for 0.5 to 1 hr. Following the reaction, the solvent was removed by evaporation under reduced pressure and the mixture was treated with diethyl ether. The precipitated crystals were then collected by filtration. The product was washed with 1% aq. KHCOs and the resulting solid was recrystallized from a mixed solvent of DMF and water to obtain a compound 10b (Compound IDs I-34 to I-38) (Tables 10 and 11).

### Synthesis Example 13: General synthesis of 6-arylmethylidenehydrazino-8-(4-chlorophenyl)-7H-purin-2(3H)-one compounds 10c (R² = 4-Cl-C₆H₄) (Compound IDs I-39 to I-43)

To TFA (8 mL), 8-(4-chlorophenyl)-6-hydrazino-7*H*-purin-2(3*H*)-one compound 10c (0.50 g, 1.81 mmol) and various aldehydes (2.17 mmol) were added and each mixture was stirred at room temperature for 0.5 to 1 hr. Following the reaction, the solvent was removed by evaporation under reduced pressure and the mixture was treated with diethyl ether. The precipitated crystals were then collected by filtration. The product was washed with 1 % aq. KHCOs and the resulting solid was recrystallized from a mixed solvent of DMF and ethanol to obtain a compound 10c (Compound IDs I-39 to I-43) (Tables 12 and 13).

### [Example 2. Synthesis Examples of Triazolopurine Compounds]

According to the reactions described in Scheme 4 described above, triazolo [5,1-*i*] purine compounds denoted as compounds 12 (Compound IDs II-1 to II-10) were synthesized. (For R⁹ in Scheme 4, refer to Table 2)

### Synthesis Example 14: General synthesis of 1H-[1,2,4]triazolo[5,1-i]purin-5(4H)-one compounds 12 (Compound IDs II-1 to II-3)

To acetic acid (8 mL), 6-hydrazino-7*H*-purin-2(3*H*)-one compound 2a (0.20 g, 1.2 mmol) and various orthoesters (4.8 mmol) were added and each mixture was heated for 10 to 20 min at 80°C while being stirred. Following the reaction, the mixture was allowed to cool to room temperature and the precipitated crystals were collected by filtration. The product was recrystallized from a mixed solvent of DMF and water to obtain a compound 12 (Compound IDs II-1 to II-3) (Tables 14 and 15).

### Synthesis Example 15: General synthesis of 8-substituted 1H-[1,2,4]triazolo[5,1-i]purin-5(4H)-one compounds 12 (Compound IDs II-3 to II-10)

(Route i): To TFA (6 mL), 6-*n*-octylidenehydrazino-7*H*-purin-2(3*H*)-one or 6-arylmethylidenehydrazino-7*H*-purin-2(3*H*)-one compound 3a (1 mmol) and 70% nitric acid (0.10 mL, 1.5 mmol) were added and each mixture was stirred at room temperature for 10 min. Following the reaction, the solvent was removed by evaporation under reduced pressure and the mixture was treated with diethyl ether. The precipitated crystals were then collected by filtration. The product was washed with 1% aq. KHCOs and the resulting solid was recrystallized from a mixed solvent of DMF and water to obtain a compound 12 (Compound IDs II-3 to II-10) (Tables 14 and 15).

(Route ii): To DMF (20mL), a corresponding 6-arylmethylidenehydrazino-7*H*-purin-2(3*H*)-one compound 3a (1 mmol) and chloranil (0.37 g, 1.5 mmol) were added and each mixture was heated to reflux for 10 min. Following the reaction, the solvent was removed by evaporation under reduced pressure and the mixture was treated with ethanol and diethyl ether. The precipitated crystals were then collected by filtration. The product was treated with activated carbon and recrystallized from a mixed solvent of DMF and water to obtain a compound 12 (Compound IDs II-3 to II-10) (Tables 14 and 15).

According to the reactions described in Scheme 5 described above, triazolo[3,4-*i*] purine compounds denoted as compounds 13 (Compound IDs III-1 to III-7) were synthesized. (For R¹³ in Scheme 5, refer to Table 3)

### Synthesis Example 16: General synthesis of 6,9-dimethyl-9H-1,2,4-triazolo[3,4-i]purin-5(6H)-one compounds 13 (Compound IDs III-1 to III-3)

To DMF (20 mL), 6-hydrazino-3,7-dimethyl-7*H*-purin-2(3*H*)-onecompound 5 (0.20 g, 1.03 mmol) and various orthoesters (2.06 mmol) were added and each mixture was heated to reflux for 1 hr. Following the reaction, the solvent was removed by evaporation under reduced pressure and the mixture was treated with ethyl acetate. The precipitated crystals were then collected by filtration. The product was recrystallized from a mixed solvent of DMF and water to obtain a compound 13 (Compound IDs III-1 to III-3) (Tables 16 and 17).

### Synthesis Example 17: General synthesis of 3-substituted 6,9-dimethyl-9H-1,2,4-triazolo[3,4-i]purin-5(6H)-one compounds 13 (Compound IDs III-2 to III-7)

To DMF (30mL), 6-alkylidenehydrazino-3,7-dimethyl-7*H*-purin-2(3*H*)-one or 6-arylmethylidenehydrazino-3,7-dimethyl-7*H*-purin-2(3*H*)-one compound 6 (1 mmol) and 70% nitric acid (0.10 mL, 1.5 mmol) were added and each mixture was stirred at 100 °C for 0.5 to 1 hr. Following the reaction, the solvent was removed by evaporation under reduced pressure and the mixture was treated with water. The precipitated crystals were then collected by filtration. The product was recrystallized from a mixed solvent of DMF and water to obtain a compound 13 (Compound IDs III-2 to III-7) (Tables 16 and 17).

### Synthesis Example 18: Synthesis of methyl (1-methyl-5-(4H-1,2,4-triazole-3-yl)-1H-imidazole-4-yl) methyl carbamate compound 14a

To a 0.1N methanol solution of sodium methoxide (10 mL), 6,9-dimethyl-9*H-*1,2,4-triazolo[3,4-*i*]purin-5(6*H*)-one compound 13 (Compound ID III-1) (0.20 g, 0.98 mmol) was added and the mixture was stirred at room temperature for 30 min. Following the reaction, suspending materials were removed by filtration and the solvent was evaporated under reduced pressure. This was followed by the addition of water and 10% aq. HCI for neutralization. The product was then extracted with ethyl acetate (20 mL × 4) and the organic layer was dried over Mg₂SO₄. The organic layer was evaporated under reduced pressure and the resulting crystals were recrystallized form ethyl acetate to obtain a compound 14a (Tables 18 and 19).

### Synthesis Example 19: Synthesis of methyl (1-methyl-5-(5-methyl-4H-1,2,4-triazole-3-yl)-1H-imidazole-4-yl) methyl carbamate compound 14b

To a 0.1N methanol solution of sodium methoxide (10 mL), 3,6,9-trimethyl-9*H*-1,2,4-triazolo[3,4-*i*]purin-5(6*H*)-one compound 13 (Compound ID III-2) (0.20 g, 0.92 mmol) was added and the mixture was stirred at room temperature for 30 min. Following the reaction, suspending materials were removed by filtration and the solvent was evaporated under reduced pressure. This was followed by the addition of water and 10% aq. HCI for neutralization. The product was then extracted with ethyl acetate (20 mL × 4) and the organic layer was dried over Mg₂SO₄. The organic layer was evaporated under reduced pressure and the resulting crystals were recrystallized form ethyl acetate to obtain a compound 14b (Tables 18 and 19).

### Synthesis Example 20: Synthesis of methyl (1-methyl-5-(5-aryl-4H-1,2,4-triazole-3-yl)-1H-imidazole-4-yl) methyl carbamate compounds 14c-g

To a 0.1N methanol solution of sodium methoxide (15 mL), 3-aryl-6,9-dimethyl-9*H*-1,2,4-triazolo[3,4-*i*]purin-5(6*H*)-one compound 13 (Compound IDs III-3 to III-7) (1 mmol) was added and each mixture was stirred at room temperature for 0.5 to 1 hr. Following the reaction, suspending materials were removed by filtration and the solvent was evaporated under reduced pressure. Water was then added, followed by 10% aq. HCI for neutralization to precipitate crystals. The product was collected by filtration and recrystallized from ethanol or a mixed solvent of DMF and ethanol to obtain a compound 14c-g (Tables 18 and 19).

### Synthesis Example 21: General synthesis of 1,4-dimethyl-1H-[1,2,4]triazolo[5,1-i]purin-5(4H)-one compounds 12 (Compound IDs II-11 to II-17)

To diphenyl ether (8 mL), the compound 14a-g (1 mmol) was added and each mixture was heated at 200°C for 2 hrs while being stirred. Following the reaction, the mixture was allowed to cool to room temperature and the precipitated crystals were collected by filtration. The product was washed with diethyl ether and recrystallized from DMF to obtain a compound 12 (Compound IDs II-11 to II-17) (Tables 20 and 21).

### Synthesis Example 22: General synthesis of 1,4-dimethyl-1H-[1,2,4]triazolo[5,1-i]purin-5(4H)-one compounds 12 (Compound IDs II-11 to II-13)

To DMF (10mL), a corresponding 1*H*-[1,2,4]triazolo[5,1-*i*]purin-5(4*H*)-one compound 12 (Compound IDs II-1 to II-3) (1 mmol) and *N*,*N'*-dimethylformamide dimethyl acetal (1.2 g, 10 mmol) were added and each mixture was heated to reflux for 2 to 3 hrs. Following the reaction, the solvent was removed by evaporation under reduced pressure and the mixture was treated with ethyl acetate. The precipitated crystals were then collected by filtration. The product was recrystallized from DMF to obtain a compound 12 (Compound IDs II-11 to II-13) (Tables 20 and 21). Synthesis Example 23: Synthesis of 1-amino-1,2,3,6-tetrahydro-6-imino-3,7-dimethyl-2-oxo-7*H-*purine compound 16

To DMF (100 mL), a known 6-amino-2,3-dihydro-3,7-dimethyl-2-oxo-7*H*-purine compound 15 (Z. Kazimierczuk, et al., Acta Biochem. Pol., 21, 455 (1974)) (0.20 g, 1.12 mmol) and O-(2,4-dinitrophenyl)hydroxylamine (DNPA) (0.33 g, 1.68 mmol) were added and the mixture was heated at 80°C for 1 hr while being stirred. Following the reaction, the solvent was evaporated under reduced pressure and the residue was separated and purified through silica gel chromatography (Kieselgel 70-230 mesh) (ethyl acetate: ethanol = 4:1). The resulting solid was recrystallized from ethanol to obtain a compound 16 as colorless powdery crystals (0.12 g, 55%, mp 226°C (decomposed)).
¹H-NMR [200 MHz, (CD₃)₂SO] δ: 3.35 (3H, s, 3-Me), 3.92 (3H, s, 7-Me), 4.90 (2H, s, exchangeable with D₂O, 1-NH₂), 7.49 (1H, br s, exchangeable with D₂O, 6-NH), 7.81 (1H, s, 8-H); IR: 3320 (νₐₛ, NH₂), 3250 (νₛ, NH₂), 3200 (v, NH), 1690 (v, C=O), 1630 cm⁻¹(δ, NH₂); *Anal.* Calcd. for C₇H₁₀N₆O·1/3 H₂O: C, 42.00; H, 5.37; N, 41.98 Found: C, 41.80; H, 5.11; N, 41.78; MS (FAB, glycerol matrix): *m*/*z* = 195 (MH⁺).

### Synthesis Example 24: General synthesis of 1,4-dimethyl-1H-[1,2,4]triazolo[5,1-i]purin-5(4H)-one compounds 12 (Compound IDs II-11 to II-13)

To diphenyl ether (8 mL), 1-amino-1,2,3,6-tetrahydro-6-imino-3,7-dimethyl-2-oxo-7*H-*purine compound 16 (0.20 g, 1.03 mmol) and various orthoesters (3.09 mmol) were added and each mixture was heated at 200°C for 20 min while being stirred. Following the reaction, the mixture was allowed to cool to room temperature and the precipitated crystals were collected by filtration. The product was washed with diethyl ether and recrystallized from DMF to obtain a compound 12 (Compound IDs II-11 to II-13) (Tables 20 and 21).

According to the reactions described in Scheme 6 described above, triazolo[5,1-*i*] purine compounds denoted as compounds 17a-c (Compound IDs II-18 to II-38) were synthesized (For R⁷ and R⁹ in Scheme 6, refer to Table 2.).

### Synthesis Example 25: General synthesis of 2-methyl-1H-[1,2,4]triazolo[5,1-i]purin-5(4H)-one compounds 17a (Compound IDs II-18 to II-20)

To acetic acid (5 mL), 6-hydrazino-8-methyl-7*H*-purin-2(3*H*)-one compound 9a (0.20 g, 1.11 mmol) and various orthoesters (3.33 mmol) were added and each mixture was heated for 10 min at 80°C while being stirred. Following the reaction, the mixture was allowed to cool to room temperature and the precipitated crystals were collected by filtration. The product was recrystallized from a mixed solvent of DMF and water to obtain a compound 17a (Compound IDs II-18 to II-20) (Tables 22 and 23).

### Synthesis Example 26: General synthesis of 8-aryl-2-methyl-1H-[1,2,4]triazolo[5,1-i]purin-5(4H)-one compounds 17a (Compound IDs II-20 to II-24)

A: To DMF (15 mL), 6-arylmethylidenehydrazino-8-methyl-7*H*-purin-2(3*H*)-one compounds 10a (Compound IDs I-29 to I-32) (1 mmol) and DEAD (0.26 g, 1.5 mmol) were added and each mixture was heated to reflux for 1 hr. Following the reaction, the solvent was concentrated under reduced pressure and the mixture was chilled on ice. The precipitated crystals were then collected by filtration. The product was recrystallized from a mixed solvent of DMF and water to obtain a compound 17a (Compound IDs II-20 to II-23) (Tables 22 and 23).
B: To DMF (20 mL), 8-methyl-6-(4-nitrobenzylidenehydrazino)-7*H*-purin-2(3*H*-one compound 10a (Compound ID I-33) (0.30 g, 0.96 mmol) and DEAD (0.17 g, 0.96 mmol) were added and each mixture was heated to reflux for 3 hrs. It should be noted that 0.17g DEAD was added in two portions, 1 hour apart. Following the reaction, the solvent was concentrated under reduced pressure and the mixture was chilled on ice. The precipitated crystals were then collected by filtration. The product was recrystallized from a mixed solvent of DMF and water to obtain a compound 17a as colorless powdery crystals (Compound ID II-24) (Tables 22 and 23).

### Synthesis Example 27: General synthesis of 2-phenyl-1H-[1,2,4]triazolo[5,1-i]purin-5(4H)-one compounds 17b (Compound IDs II-25 to II-27)

To acetic acid (8 mL), 6-hydrazino-8-phenyl-7*H*-purin-2(3*H*)-one compound 9b (0.20 g, 0.83 mmol) and various orthoesters (2.49 mmol) were added and each mixture was heated for 10 min at 80°C while being stirred. Following the reaction, the mixture was allowed to cool to room temperature and the precipitated crystals were collected by filtration. The product was recrystallized from a mixed solvent of DMF and ethanol to obtain a compound 17b (Compound IDs II-25 to II-27) (Tables 24 and 25).

### Synthesis Example 28: General synthesis of 8-aryl-2-phenyl-1H-[1,2,4]triazolo[5,1-i]purin-5(4H)-one compounds 17b (Compound IDs II-27 to II-31)

A: To DMF (20 mL), corresponding 6-arylmethylidenehydrazino-8-phenyl-7*H*-purin-2(3*H*)-one compounds 10b (Compound IDs I-34 to I-37) (1 mmol) and DEAD (0.26 g, 1.5 mmol) were added and each mixture was heated to reflux for 1 to 1.5 hrs. Following the reaction, the solvent was concentrated under reduced pressure and the mixture was treated with water. The precipitated crystals were then collected by filtration. The product was recrystallized from a mixed solvent of DMF and ethanol to obtain a compound 17b (Compound IDs II-27 to II-30) (Tables 24 and 25).
B: To DMF (20 mL), 6-(4-nitrobenzylidenehydrazino)-8-phenyl-7*H*-purin-2(3*H*)-one compound 10b (Compound ID I-38) (0.30 g, 0.80 mmol) and DEAD (0.14 g, 0.80 mmol) were added and the mixture was heated to reflux for 2 hrs. It should be noted that 0.14 g of DEAD was added after 1 hr. Following the reaction, the solvent was concentrated under reduced pressure and the mixture was treated with water. The precipitated crystals were then collected by filtration. The product was recrystallized from a mixed solvent of DMF and ethanol to obtain a compound 17b as colorless powdery crystals (Compound ID II-31) (Tables 24 and 25).

### Synthesis Example 29: General synthesis of 2-(chlorophenyl)-1H-[1,2,4]triazolo[5,1-i]purin-5(4H)-one compounds 17c (Compound IDs II-32 to II-34)

To acetic acid (8 mL), 8-(4-chlorophenyl)-6-hydrazino-7*H*-purin-2(3*H*)-one compound 9c (0.20 g, 0.72 mmol) and various orthoesters (2.16 mmol) were added and each mixture was heated for 10 min at 80°C while being stirred. Following the reaction, the mixture was allowed to cool to room temperature and the precipitated crystals were collected by filtration. The product was recrystallized from a mixed solvent of DMF and ethanol to obtain a compound 17c (Compound IDs II-32 to II-34) (Tables 26 and 27).

### Synthesis Example 30: General synthesis of 8-aryl-2-(chlorophenyl)-1H-[1,2,4]triazolo[5,1-i]purin-5(4H)-one compounds 17c (Compound IDs II-34 to II-38)

A: To DMF (20 mL), corresponding 6-arylmethylidenehydrazino-8-(4-chlorophenyl)-7*H*-purin-2(3*H*)-one compounds 10c (Compound IDs I-39 to I-42) (1 mmol) and DEAD (0.26 g, 1.5 mmol) were added and each mixture was heated to reflux for 1 to 1.5 hrs. Following the reaction, the solvent was concentrated under reduced pressure and the mixture was treated with water. The precipitated crystals were then collected by filtration. The product was recrystallized from a mixed solvent of DMF and ethanol to obtain a compound 17c (Compound IDs II-34 to II-37) (Tables 26 and 27).
B: To DMF (20 mL), 8-(4-chlorophenyl)-6-(4-nitrobenzylidenehydrazino)-7*H*-purin-2(3*H*)-one compound 10c (Compound ID I-43) (0.30 g, 0.73 mmol) and DEAD (0.13 g, 0.73 mmol) were added and the mixture was heated to reflux for 2 hrs. It should be noted that 0.13 g of DEAD was added after 1 hr. Following the reaction, the solvent was concentrated under reduced pressure and the mixture was treated with water. The precipitated crystals were then collected by filtration. The product was recrystallized from a mixed solvent of DMF and ethanol to obtain a compound 17c as colorless powdery crystals (Compound ID II-38) (Tables 26 and 27).

According to the reactions described in Scheme 7 described above, triazolo[5,1-*i*]purine compounds denoted as compounds 17b, c (Compound IDs II-25 to II-27 and II-32 to II-34) were separately synthesized (For R⁷ and R⁹ in Scheme 7, refer to Table 2.).

### Synthesis Example 31: Synthesis of 6-amino-2,3-dihydro-2-oxo-8-phenyl-7H-purine compound 18b

To 28% aqueous ammonium (50mL), 1,2,3,6-tetrahydro-2-oxo-8-phenyl-6-thioxo-7*H-*purine compound 8b (2 g, 8.19 mmol) was added and the mixture was heated at 160°C in a sealed tube for 48 hrs. Following the reaction, the mixture was chilled on ice and the precipitated crystals were collected by filtration. The product was washed with water and ethanol to obtain a compound 18b as colorless powdery crystals (1.4 g, 75%, mp > 330°C). Although the elemental analysis as a measure of purity could not be performed since the product was poorly soluble in a general-purpose solvent and could not be subjected to recrystallization, TLC and ¹H-NMR spectra confirmed that the product was a single compound.
¹H-NMR [300 MHz, CF₃COOD] δ: 7.65-7.77 (2H, m, Ph-*m*H), 7.77-7.86 (1H, m, Ph-*p*H), 7.95-8.05 (2H, m, Ph-*o*H); IR: 3320 (νₐₛ, NH₂), 3080 (νₛ, NH₂ and v, NH), 1680 (v, C=O), 1660 cm⁻¹ (δ, NH₂); MS (FAB, glycerol matrix): *m*/*z* = 228 (MH⁺).

### Synthesis Example 32: Synthesis of 6-amino-8-(4-chlorophenyl)-2,3-dihydro-2-oxo-7H-purine compound 18c

To 28% aqueous ammonium (50mL), 8-(4-chlorophenyl)-1,2,3,6-tetrahydro-2-oxo-6-thioxo-7*H*-purine compound 8c (2 g, 7.18 mmol) was added and the mixture was heated at 160°C in a sealed tube for 48 hrs. Following the reaction, the mixture was chilled on ice and the precipitated crystals were collected by filtration. The product was washed with water and ethanol to obtain a compound 18c as colorless powdery crystals (1.44 g, 77%, mp > 330°C). Although the elemental analysis as a measure of purity could not be performed since the product was poorly soluble in a general-purpose solvent and could not be subjected to recrystallization, TLC and ¹H-NMR spectra confirmed that the product was a single compound.
**¹H-NMR [300 MHz, CF₃COOD] δ: 7.69 (2H, *J*_{AB} = 8.6 Hz, Ar-*m*H), 7.96 (2H, *J*_{AB} = 8.6 Hz, Ar-*o*H); IR: 3320 (νₐₛ, NH₂), 3090 (νₛ, NH₂ and v, NH), 1690 (v, C=O), 1660 cm⁻¹(δ, NH₂); MS (FAB, glycerol matrix): *m*/*z* = 262 (MH⁺), 264 (MH⁺+2).**

### Synthesis Example 33: Synthesis of 1-amino-1,2,3,6-tetrahydro-6-imino-2-oxo-8-phenyl-7H-purine compound 19b

6-amino-2,3-dihydro-2-oxo-8-phenyl-7*H*-purine compound 18b (0.50 g, 2.20 mmol) was dissolved in 2N NaOH (15 mL). To this solution, hydroxylamine-*O*-sulfonic acid (1 g, 8.80 mmol) dissolved in 3mL water was added dropwise at 0 to 10°C and the mixture was stirred for 30 min. Following the reaction, the precipitated crystals were collected by filtration. The product was dissolved in water, followed by the addition of 10% aq. HCI for neutralization to precipitate crystals. The product was collected by filtration and recrystallized from ethanol to obtain a compound 19b as colorless powdery crystals (0.34 g, 64%, mp 270°C (decomposed)).
¹H-NMR [300 MHz, (CD₃)₂SO] δ: 5.34 (2H, s, exchangeable with D₂O, 1-NH₂), 7.33-7.50 (3H, m, Ph-*m*,*p*H), 7.91-8.10 (2H, m, Ph-*o*H), 8.39 (1H, br, exchangeable with D₂O, 6-NH), 12.54 (1H, br s, exchangeable with D₂O, 3-NH); IR: 3400 (νₐₛ, NH₂), 3300 (νₛ, NH₂), 3120 (v, NH), 1660 (v, C=O), 1640 cm⁻¹ (δ, NH₂); *Anal.* Calcd. for C₁₁H₁₀N₆O·1/4 H₂O: C, 53.55; H, 4.29; N, 34.06 Found: C, 53.57; H, 4.52; N, 34.31; MS (FAB, glycerol matrix): *m*/*z* = 243 (MH⁺).

### Synthesis Example 34: Synthesis of 1-amino-8-(4-chlorophenyl)-1,2,3,6-tetrahydro-6-imino-2-oxo-7H-purine compound 19c

6-amino-8-(4-chlorophenyl)-2,3-dihydro-2-oxo-7*H-*purine compound 18c (0.50 g, 1.91 mmol) was dissolved in 2N NaOH (15mL). To this solution, hydroxylamine-*O*-sulfonic acid (0.86 g, 7.64 mmol) dissolved in 3mL water was added dropwise at 0 to 10°C and the mixture was stirred for 30 min. Following the reaction, the precipitated crystals were collected by filtration. The product was dissolved in water, followed by the addition of 10% aq. HCI for neutralization to precipitate crystals. The product was collected by filtration and recrystallized from ethanol to obtain a compound 19c as colorless powdery crystals (0.33 g, 62%, mp 292°C (decomposed)).
¹H-NMR [300 MHz, (CD₃)₂SO] δ: 5.39 (2H, s, exchangeable with D₂O, 1-NH₂), 7.53 (2H, d, *J*_{AB} = 8.6 Hz, Ar-*m*H), 8.02 (2H, d, *J*_{AB} = 8.6 Hz, Ar-*o*H), 8.71 (1H, br, exchangeable with D₂O, 6-NH), 12.78 (1H, br, exchangeable with D₂O, 3-NH); IR: 3460 (νₐₛ, NH₂), 3330 (νₛ, NH₂), 3090 (v, NH), 1680 (v, C=O), 1640 cm⁻¹(δ, NH₂); *Anal.* Calcd. for C₁₁H₉ClN₆O·3/4 H₂O: C, 45.53; H, 3.65; N, 28.96 Found: C, 45.72; H, 3.44; N, 29.18; MS (FAB, glycerol matrix): *mlz* = 277 (MH⁺), 279 (MH⁺+2).

### Synthesis Example 35: General synthesis of 2-phenyl-1H-[1,2,4]triazolo[5,1-i]purin-5(4H)-one compounds 17b (Compound IDs II-25 to II-27)

(Route i): To TFA (4 mL), 1-amino-1,2,3,6-tetrahydro-6-imino-2-oxo-8-phenyl-7*H*-purine compound 19b (0.20 g, 0.83 mmol) and triethyl orthoformate (0.62 g, 4.15 mmol) were added and the mixture was stirred at room temperature for 3 hrs. Following the reaction, the solvent was removed by evaporation under reduced pressure and the mixture was treated with diethyl ether. The precipitated crystals were then collected by filtration. The product was washed with 0.5% aq. KHCOs and the resulting solid was recrystallized from a mixed solvent of DMF and ethanol to obtain a compound 17b as colorless powdery crystals (Compound ID II-25) (Tables 24 and 25).

(Route ii): To anhydrous acetic acid (4 mL), 1-amino-1,2,3,6-tetrahydro-6-imino-2-oxo-8-phenyl-7*H-*purine compound 19b (0.20 g, 0.83 mmol) was added and the mixture was heated to reflux for 3 hrs. Following the reaction, the mixture was allowed to cool to room temperature and the precipitated crystals were collected by filtration. The product was recrystallized from a mixed solvent of DMF and ethanol to obtain a compound 17b as colorless powdery crystals (Compound ID II-26) (Tables 24 and 25).

(Route iii): To DMF (15 mL), 1-amino-1,2,3,6-tetrahydro-6-imino-2-oxo-8-phenyl-7*H*-purine compounds 19b (0.20 g, 0.83 mmol) and benzaldehyde (0.13 g, 1.25 mmol) were added and the mixture was heated to reflux for 30 min. Subsequently, DEAD (0.22 g, 1.25 mmol) was added and the mixture was heated to reflux for 1 hr. Following the reaction, the solvent was concentrated under reduced pressure and the mixture was treated with water. The precipitated crystals were then collected by filtration. The product was recrystallized from a mixed solvent of DMF and ethanol to obtain a compound 17b as colorless powdery crystals (Compound ID II-27) (Tables 24 and 25).

### Synthesis Example 36: General synthesis of 2-(chlorophenyl)-1H-[1,2,4]triazolo[5,1-i]purin-5(4H)-one compounds 17c (Compound IDs II-32 to II-34)

(Route i): To TFA (4 mL), 1-amino-8-(4-chlorophenyl)-1,2,3,6-tetrahydro-6-imino-2-oxo-7*H*-purine compound 19c (0.20 g, 0.72 mmol) and triethyl orthoformate (0.53 g, 3.6 mmol) were added and the mixture was stirred at room temperature for 3 hrs. Following the reaction, the solvent was removed by evaporation under reduced pressure and the mixture was treated with diethyl ether. The precipitated crystals were then collected by filtration. The product was washed with 0.5% aq. KHCOs and the resulting solid was recrystallized from a mixed solvent of DMF and ethanol to obtain a compound 17c as colorless powdery crystals (Compound ID II-32) (Tables 26 and 27).

(Route ii): To anhydrous acetic acid (4 mL), 1-amino-8-(4-chlorophenyl)-1,2,3,6-tetrahydro-6-imino-2-oxo-7*H*-purine compound 19c (0.20 g, 0.72 mmol) was added and the mixture was heated to reflux for 3 hrs. Following the reaction, the mixture was allowed to cool to room temperature and the precipitated crystals were collected by filtration. The product was recrystallized from a mixed solvent of DMF and ethanol to obtain a compound 17c as colorless powdery crystals (Compound ID II-33) (Tables 26 and 27).

(Route iii): To DMF (15 mL), 1-amino-8-(4-chlorophenyl)-1,2,3,6-tetrahydro-6-imino-2-oxo-7*H-*purine compound 19c (0.20 g, 0.72 mmol) and benzaldehyde (0.11 g, 1.08 mmol) were added and the mixture was heated to reflux for 30 min. Subsequently, DEAD (0.19 g, 1.08 mmol) was added and the mixture was heated to reflux for 1 hr. Following the reaction, the solvent was concentrated under reduced pressure and the mixture was treated with water. The precipitated crystals were then collected by filtration. The product was recrystallized from a mixed solvent of DMF and ethanol to obtain a compound 17c as colorless powdery crystals (Compound ID II-34) (Tables 26 and 27).

### [Example 3. Physical data and NMR data of the compounds]

Physical data and ¹H-NMR data for the compounds synthesized in Examples 1 and 2 are presented in Tables 4 through 27 below.

**[table 4]**

| | Physical data for compounds **I-8** to **I-13** and **I-19** to **I-22.** | | | | | |
|---|---|---|---|---|---|---|
| Compd. ID *^{a}* (Formula) | | Yield (%) | Mp / °C | νₘₐₓ(Nujol) / cm⁻¹ | 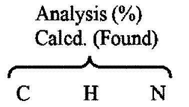 | *m*/*z*: MH^{+ *b*} |
| **I-8** | | 84 | 190 | 3380, 3180 sh, | 55.30, 7.38, 29.77 | 277 |
| C₁₃H₂₀N₆O ·1/3 H₂O | | | (decomp.) | 3080 (NH) | (55.16, 7.15, 29.54) | |
| | | | | 1700 (CO) | | |
| **I-9** | | 92 | 300 | 3380,3140, | 55.90, 4.07,32.59 | 255 |
| C₁₂H₁₀N₆O ·1/5 H₂O | | | (decomp.) | 3080 sh (NH) | (55.74, 4.34, 32.76) | |
| | | | | 1690 (CO) | | |
| **I-10** | | 92 | > 300 | 3380, 3140, | 57.43, 4.60, 30.91 | 269 |
| C₁₃H₁₂N₆O ·1/5 H₂O | | | | 3060 sh (NH) | (57.35, 4.77, 31.16) | |
| | | | | 1660 (CO) | | |
| **I-11** | | 87 | 288-290 | 3360, 3140, 3070 (NH) | 49.23, 5.07, 22.97 | 345 |
| C₁₅H₁₆N₆O₄ ·6/5 H₂O | | | (decomp.) | 1660 (CO) | (49.20, 4.93, 23.26) | |
| **I-12** | | 87 | > 300 | 3340 sh, 3150, | 47.37, 3.98, 27.62 | 287 |
| C₁₂H₁₀N₆O₃ ·H₂O | | | | 3070 (NH), 3190 (OH) | (47.48, 4.10,27.67) | |
| | | | | 1680 (CO) | | |
| **I-13** | | 85 | > 300 | 3340, 3140 sh, | 46.99, 3.45, 27.40 | 303 |
| C₁₂H₁₀N₆O₄ ·1/4 H₂O | | | | 3080 (NH) | (46.76, 3.61, 27.48) | |
| | | | | 1690 (CO) | | |
| **I-19** | | 81 | 248 | 3120, 3070 (NH) | 52.98, 7.94, 26.48 | 291 |
| C₁₄H₂₂N₆O ·3/2 H₂O | | | (decomp.) | 1650 (CO) | (53.20, 7.65, 26.62) | |
| **I-20** | | 88 | > 300 | 3280, 3120 (NH) | 52.31, 5.21, 22.88 | 359 |
| C₁₆H₁₈N₆O₄ ·1/2 H₂O | | | | 1640 (CO) | (52.42, 5.02, 23.16) | |
| **I-21** | | 79 | > 300 | 3160, 3120 (NH) | 50.98, 4.17, 27.44 | 301 |
| C₁₃H₁₂N₆O₃ ·1/3 H₂O | | | | 1630 (CO) | (50.77, 4.32, 27.28) | |
| **I-22** | | 81 | > 300 | 3180, 3120 (NH) | 46.21,4.30,24.87 | 317 |
| C₁₃H₁₂N₆O₄ ·6/5 H₂O | | | | 1630 (CO) | (46.33, 4.35,24.61) | |

| | | | | | | |
|---|---|---|---|---|---|---|
| *^{a}*All compounds were recrystallized from a mixture of DMF and H₂O except for **I-8** and **I-19** (from EtOH), and were obtained as colorless powder. *^{b}*The matrix is glycerol. | | | | | | |

**[table 5]**

| | ¹H-NMR data for compounds **I-8 to I-13** and **I-19 to I-22.** | |
|---|---|---|
| Compd. ID | | δ_{H} [200 MHz; (CD₃)₂SO; Me₄Si] |
| **I-8** | | 0.86 (3H, t, *J* = 6.8 Hz, CHCH₂CH₂[CH₂]₄*Me*), 1.28 (8H, br s, CHCH₂CH₂[C*H*₂]₄Me), 1.42-1.62 (2H, m, CHCH₂C*H*₂[CH₂]₄Me), 2.26-2.50 (2H, m, CHC*H*₂CH₂[CH₂]₄Me), 7.63 (1H, t, *J* = 5.6 Hz, C*H*CH₂CH₂[CH₂]₄Me), 7.83 (1H, s, 8-H), 11.22 (1H, br, exchangeable with D₂O, 3-NH), 12.10 (1H, br, exchangeable with D₂O, 7-NH) |
| **I-9** | | 7.30-7.55 (3H, m, Ph-*m*,*p*H), 7.84 (1H, s, 8-H), 7.88-8.10 (2H, m, Ph-oH), 8.40 (1H, s, CH-Ar ), 10.13 (1H, br s, exchangeable with D₂O, 6-NH), 11.17 (1H, br s, exchangeable with D₂O, 3-NH), 13.22 (1H, br, exchangeable with D₂O, 7-NH) |
| **I-10** | | 2.35 (3H, s, Me), 7.24 (2H, d, *J*_{AB} = 8.0 Hz, Ar-*m*H), 7.83 (1H, s, 8-H), 7.90 (2H, d, *J*_{AB} = 8.0 Hz, Ar-*o*H), 8.36 (1H, s, C*H*-Ar), 10.07 (1H, br s, exchangeable with D₂O, 6-NH), 11.18 (1H, br s, exchangeable with D₂O, 3-NH), 13.20 (1H, br, exchangeable with D₂O, 7-NH) |
| **I-11** | | 3.71 (3H, s, 4'-OMe), 3.87 (6H, s, 3'-OMe and 5'-OMe), 7.31 (2H, s, 2'-H and 6'-H), 7.83 (1H, s, 8-H), 8.32 (1H, s, CH-Ar), 10.10 (1H, s, exchangeable with D₂O, 6-NH), 11.20 (1H, br s, exchangeable with D₂O, 3-NH), 13.20 (1H, br, exchangeable with D₂O, 7-NH) |
| **I-12*^{a}*** | | 6.78 (1H, d, *J*_{5', 6'} = 8.1 Hz, 5'-H), 7.20 (1H, d, *J*_{5', 6'} = 8.1 Hz, 6'-H), 7.47 (1H, s, 2'-H), 7.90 (1H, s, 8-H), 8.24 (1H, s, C*H*-Ar), 9.02 (1H, br s, exchangeable with D₂O, 3'-OH), 9.53 (1H, br s, exchangeable with D₂O, 4'-OH), 10.32 (1H, br, exchangeable with D₂O, 6-NH), 11.30 (1H, br, exchangeable with D₂O, 3-NH), 12.93 (1H, br, exchangeable with D₂O, 7-NH) |
| **I-13*^{a}*** | | 6.38 (1H, d, *J*_{5', 6'} = 8.4 Hz, 5'-H), 7.03 (1H, d, *J*_{5', 6'} = 8.4 Hz, 6'-H), 7.83 (1H, s, 8-H), 8.44 (1H, s, C*H*-Ar), 8.70 (1H, br, exchangeable with D₂O, 3'-OH), 9.57 (1H, br, exchangeable with D₂O, 4'-OH), 9.95(1H, br, exchangeable with D₂O, 2'-OH), 10.44 (1H, br, exchangeable with D₂O, 6-NH), 11.26 (1H, br, exchangeable with D₂O, 3-NH), 13.06 (1H, br, exchangeable with D₂O, 7-NH) |
| **I-19** | | 0.87 (3H, t, *J* = 6.2 Hz, CHCH₂CH₂[CH₂]₄*Me*), 1.28 (8H, br s, CHCH₂CH₂[C*H*₂]₄Me), 1.44-1.64 (2H, m, CHCH₂C*H*₂[CH₂]₄Me), 2.50-2.65 (2H, m, CHC*H*₂CH₂[CH₂]₄Me), 3.44 (3H, s, N-Me), 7.36 (1H, t, *J* = 6.2 Hz, C*H*CH₂CH₂[CH₂]₄Me), 7.91 (1H, s, 8-H), 11.28 (1H, s, exchangeable with D₂O, 3-NH), 11.78 (1H, s, exchangeable with D₂O, 7-NH) |
| **I-20** | | 3.62 (3H, s, N-Me), 3.72 (3H, s, 4'-OMe), 3.88 (6H, s, 3'-OMe and 5'-OMe), 7.13 (2H, s, 2'-H and 6'-H), 7.95 (1H, s, 8-H), 8.17 (1H, s, CH-Ar), 11.44 (1H, br, exchangeable with D₂O, 3-NH), 11.69 (1H, brs, exchangeable with D₂O, 7-NH) |
| **I-21*^{a}*** | | 3.59 (3H, s, N-Me), 6.83 (1H, d, *J*_{5', 6'} = 8.4 Hz, 5'-H), 7.19 (1H, d, *J*_{5', 6'} = 8.4 Hz, 6'-H), 7.23 (1H, s, 2'-H), 7.94 (1H, s, 8-H), 8.02 (1H, s, C*H*-Ar), 9.10 (1H, s, exchangeable with D₂O, 3'-OH), 9.43 (1H, s, exchangeable with D₂O, 4'-OH), 11.31 (2H, s, exchangeable with D₂O, 3-NH and 7-NH) |
| **I-22** | | 3.55 (3H, s, N-Me), 6.48 (1H, d, *J*_{5', 6'} = 8.2 Hz, 5'-H), 6.89 (1H, d, *J*_{5', 6'} = 8.2 Hz, 6'-H), 7.86 (1H, s, 8-H), 7.96 (1H, s, C*H*-Ar), 9.01 (1H, brs, exchangeable with D₂O, 3'-OH), 9.94 (1H, br s, exchangeable with D₂O, 4'-OH), 10.01 (1H, brs, exchangeable with D₂O, 2'-OH), 11.22 (1H, br s, exchangeable with D₂O, 3-NH), 12.84 (1H, br s, exchangeable with D₂O, 7-NH) |

| | | |
|---|---|---|
| *^{a}*This compound was measured at 300 MHz in (CD₃)₂SO. | | |

**[table 6]**

| | Physical data for compounds **I-23** to **I-28**. | | | | | |
|---|---|---|---|---|---|---|
| Compd. ID *^{a}* (Formula) | | Yield (%) | Mp /°C | *v*ₘₐₓ(Nujol) / cm⁻¹ | 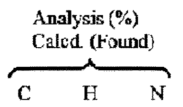 | *m*/*z*: MH^{+ *b*} |
| **I-23** | | 82 | 173 | 3190 (NH) | 48.10, 5.61, 37.40 | 221 |
| C₉H₁₂N₆O ·1/4 H₂O | | | | 1670 (CO) | (48.25, 5.45, 37.62) | |
| **I-24** | | 82 | 203 | 3110 (NH) | 59.56, 5.00, 29.77 | 283 |
| C₁₄H₁₄N₆O | | | | 1680 (CO) | (59.30, 5.16, 29.93) | |
| **I-25** | | 86 | 250 | 3120 (NH) | 53.09, 4.14, 26.53 | 317 |
| C₁₄H₁₃ClN₆O | | | | 1690 (CO) | (53.05, 4.16, 26.63) | 319 |
| **I-26** | | 90 | 237 | 3120 (NH) | 60.80, 5.44, 28.36 | 297 |
| C₁₅H₁₆N₆O | | | | 1680 (CO) | (60.86, 5.43, 28.51) | |
| **I-27** | | 85 | 193 | 3120 (NH) | 57.68, 5.16, 26.91 | 313 |
| C₁₅H₁₆N₆O₂ | | | | 1670 (CO) | (57.40, 5.14, 26.92) | |
| **I-28** | | 83 | 288 | 3140 (NH) | 50.68, 4.10, 29.55 | 328 |
| C₁₄H₁₃N₇O₃ ·1/4 H₂O | | | | 1700 (CO) | (50.47, 4.18, 29.42) | |

| | | | | | | |
|---|---|---|---|---|---|---|
| *^{a}*All compounds were recrystallized from a mixture of DMF and H₂O except for **I-23** (from EtOH), and were obtained as colorless powder except for **I-28** (orange powder). *^{b}*The matrix is glycerol | | | | | | |

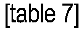

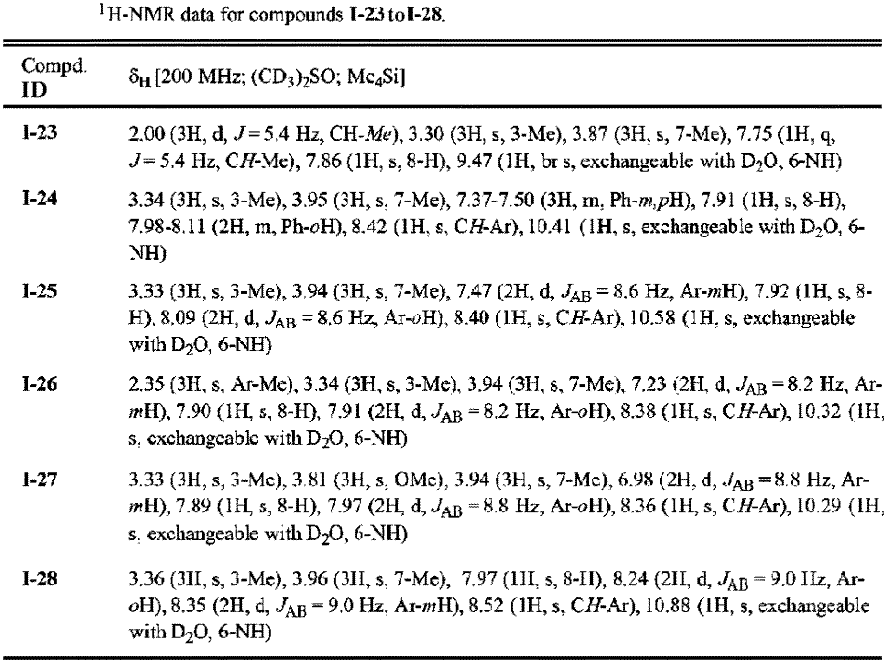

**[table 8]**

| | Physical data for compounds **I-29** to**I-33**. | | | | | |
|---|---|---|---|---|---|---|
| Compd. ID *^{a}* (Formula) | | **Yield** (%) | **Mp /°C** | νₘₐₓ(Nujol) / cm⁻¹ | 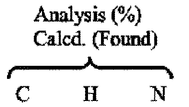 | *m*/*z*: MH^{+ *b*} |
| **I-29** | | 92 | 311 | 3380, 3180 sh, | 56.31, 4.73, 30.31 | 269 |
| C₁₃H₁₂N₆O ·1/2 H₂O | | | (decomp.) | 3110 sh (NH) | (56.02, 4.67, 30.09) | |
| | | | | 1690 (CO) | | |
| **I-30** | | 89 | > 330 | 3370, 3200, 3140 (NH) | 50.58, 3.81, 27.22 | 303 |
| C₁₃H₁₁CIN₆O ·1/3 H₂O | | | | 1680 (CO) | (50.45, 4.07, 27.47) | 305 |
| **I-31** | | 96 | > 309 | 3380, 3180, 3120 (NH) | 56.84, 5.28, 28.41 | 283 |
| C₁₄H₁₄N₆O ·3/4 H₂O | | | (decomp.) | 1690 (CO) | (56.73, 5.04, 28.35) | |
| **I-31** | | 87 | 313 | 3380, 3180, 3110 (NH) | 54.72, 4.92, 27.35 | 299 |
| C₁₄H₁₄N₆O₂ ·1/2 H₂O | | | (decomp.) | 1700 (CO) | (54.85, 5.00, 27.32) | |
| **I-33** | | 96 | > 330 | 3450, 3180 sh, 3110 sh (NH) | 47.87, 3.84, 30.06 | 314 |
| C₁₃H₁₁N₇O₃ ·5/7 H₂O | | | | | (48.14, 3.55, 29.84) | |
| | | | | 1730 (CO) | | |

| | | | | | | |
|---|---|---|---|---|---|---|
| *^{a}*All compounds were recrystallized from a mixture of DMF and H₂O, and were obtained as colorless powder except for 1-33 (yellow powder). *^{b}*The matrix is glycerol. | | | | | | |

**[table 9]**

| | ¹H-NMR data for compounds **I-29** to **I-33** |
|---|---|
| Compd. ID | δ_{H} [200 MHz; (CD₃)₂SO; Me₄Si] |
| **I-29** | 2.34 (3H, s, 8-Me), 7.35-7.53 (3H, m, Ph-*m*,*p*H), 7.86-8.09 (2H, m, Ph-*o*H), 8.37 (1H, s, C*H-*Ar), 10.10 (1H, br s, exchangeable with D₂O, 6-NH), 11.16 (1H, br s, exchangeable with D₂O, 3-NH), 12.85 (1H, br, exchangeable with D₂O, 7-NH) |
| **I-30** | 2.33 (3H, s, 8-Me), 7.47 (2H, d, *J*_{AB} = 8.6 Hz, Ar-mH), 8.07 (2H, d, *J*_{AB} = 8.6 Hz, Ar-*o*H), 8.35 (1H, s, C*H*-Ar), 10.24 (1H, s, exchangeable with D₂O, 6-NH), 11.15 (1H, s, exchangeable with D₂O, 3-NH), 12.85 (1H, br, exchangeable with D₂O, 7-NH) |
| **I-31** | 2.35 (6H, s, 8-Me and Ar-Me), 7.24 (2H, d, *J*_{AB} = 8.0 Hz, Ar-*m*H), 7.89 (2H, d, *J*_{AB} = 8.0 Hz, Ar-*o*H), 8.34 (1H, s, C*H*-Ar), 10.10 (1H, br, exchangeable with D₂O, 6-NH), 11.16 (1H, br, exchangeable with D₂O, 3-NH), 12.91 (1H, br, exchangeable with D₂O, 7-NH) |
| **I-32** | 2.33 (3H, s, 8-Me), 3.81 (3H, s, OMe), 6.97 (2H, d, *J*_{AB} = 8.6 Hz, Ar-*m*H), 7.94 (2H, d, *J*_{AB} = 8.6 Hz, Ar-*o*H), 8.31 (1H, s, C*H*-Ar), 9.93 (1H, brs, exchangeable with D₂O, 6-NH), 11.06 (1H, br s, exchangeable with D₂O, 3-NH), 12.80 (1H, br, exchangeable with D₂O, 7-NH) |
| **I-33** | 2.35 (3H, s, 8-Me), 8.24 (2H, d, *J*_{AB} = 9.6 Hz, Ar-*o*H), 8.33 (2H, d, *J*_{AB} = 9.6 Hz, Ar-*m*H), 8.45 (1H, s, C*H*-Ar), 10.54 (1H, brs, exchangeable with D₂O, 6-NH), 11.29 (1H, brs, exchangeable with D₂O, 3-NH), 12.93 (1H, br, exchangeable with D₂O, 7-NH) |

**[table 10]**

| Physical data for compounds **I-34 to I-38** | | | | | |
|---|---|---|---|---|---|
| Compd. ID *^{a}* (Formula) | Yield (%) | Mp / °C | vₘₐₓ(Nujol) / cm⁻¹ | 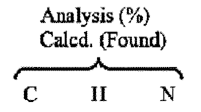 | *m*/*z*: MH^{+ *b*} |
| **I-34** | 92 | 318 | 3360 sh, 3200 sh, 3070 sh (NH) | 64.44, 4.38, 25.05 | 331 |
| C₁₈H₁₄N₆O ·2/7 H₂O | | (decomp.) | | (64.21, 4.54, 25.30) | |
| | | | 1720 (CO) | | |
| **I-35** | 90 | >330 | 3360, 3100, 3060 (NH) | 58.31, 3.72, 22.67 | 365 |
| C₁₈H₁₃ClN₆O ·1/3 H₂O | | | 1680 (CO) | (58.32, 3.96, 22.69) | 367 |
| **I-36** | 88 | 321 | 3360, 3100 sh, 3060 (NH) | 65.84, 4.73, 24.25 | 345 |
| C₁₉H₁₆N₆O ·1/8 H₂O | | (decomp.) | | (65.71, 4.98,24.26) | |
| | | | 1680 (CO) | | |
| **I-37** | 85 | 314 | 3360, 3100, 3060 (NH) | 62.54, 4.56,23.03 | 361 |
| C₁₉H₁₆N₆O₂ ·1/4 H₂O | | (decomp.) | 1670 (CO) | (62.26,4.74,23.26) | |
| **I-38** | 88 | > 330 | 3380, 3180 sh, 3100 sh (NH) | 56.69, 3.61, 25.71 | 376 |
| C₁₈H₁₃N₇O₃ ·1/3 H₂O | | | | (56.44, 3.91, 25.90) | |
| | | | 1690 (CO) | | |

| | | | | | |
|---|---|---|---|---|---|
| *^{a}*All compounds were recrystallized from a mixture of DMF and H₂O, and were obtained as colorless powder except for **I-38** (orange powder). *^{b}*The matrix is glycerol. | | | | | |

**[table 11]**

| | ¹H-NMR data for compounds **I-34** to **I-38**. |
|---|---|
| Compd. ID | δ_{H} [200 MHz; (CD₃)₂SO; Me₄Si] |
| **I-34** | 7.33-7.56 (6H, m, Ph-*m*,*p*H), 7.95-8.19 (4H, m, Ph-*o*H), 8.45 (1H, s, C*H*-Ar), 10.26 (1H, br s, exchangeable with D₂O, 6-NH), 11.36 (1H, brs, exchangeable with D₂O, 3-NH), 13.50 (1H, br, exchangeable with D₂O, 7-NH) |
| **I-35** | 7.40-7.59 (3H, m, Ph-*m*,*p*H), 7.49 (2H, d, *J*_{AB} = 8.4 Hz, Ar-*m*H), 8.03-8.21 (2H, m, Ph-oH), 8.11 (2H, d, *J*_{AB} = 8.4 Hz, Ar-oH), 8.43 (1H, s, C*H*-Ar), 10.40 (1H, s, exchangeable with D₂O, 6-NH), 11.35 (1H, s, exchangeable with D₂O, 3-NH), 13.60 (1H, br, exchangeable with D₂O, 7-NH) |
| **I-36** | 2.36 (3H, s, Me), 7.25 (2H, d, *J*_{AB} = 8.2 Hz, Ar-mH), 7.40-7.58 (3H, m, Ph-*m*,*p*H), 7.93 (2H, d, *J*_{AB} = 8.2 Hz, Ar-nH), 8.04-8.19 (2H, m, Ph-oH), 8.41 (1H, s, C*H*-Ar), 10.13 (1H, s, exchangeable with D₂O, 6-NH), 11.30 (1H, s, exchangeable with D₂O, 3-NH), 13.50 (1H, br, exchangeable with D₂O, 7-NH) |
| **I-37** | 3.82 (3H, s, OMe), 6.99 (2H, d, *J*_{AB} = 8.8 Hz, Ar-*m*H), 7.38-7.54 (3H, m, Ph-*m*,*p*H), 7.98 (2H, d, *J*_{AB} = 8.8 Hz, Ar-*o*H), 8.04-8.19 (2H, m, Ph-*o*H), 8.39 (1H, s, C*H*-Ar), 10.09 (1H, s, exchangeable with D₂O, 6-NH), 11.27 (1H, br s, exchangeable with D₂O, 3-NH), 13.30 (1H, br, exchangeable with D₂O, 7-NH) |
| **I-38** | 7.39-7.58 (3H, m, Ph-*m,p*H), 8.03-8.21 (2H, m, Ph-oH), 8.26 (2H, d, *J*_{AB} = 9.0 Hz, Ar-oH), 8.37 (2H, d, *J*_{AB} = 9.0 Hz, Ar-*m*H), 8.53 (1H, s, C*H*-Ar), 10.68 (1H, s, exchangeable with D₂O, 6-NH), 11.48 (1H, br s, exchangeable with D₂O, 3-NH), 13.70 (1H, br, exchangeable with D₂O, 7-NH) |

**[table 12]**

| Physical data for compounds **I-39 toI-43**. | | | | | |
|---|---|---|---|---|---|
| Compd. ID *^{a}* (Formula) | Yield (%) | Mp / °C | vₘₐₓ(Nujol) / cm⁻¹ | 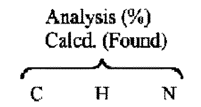 | *m*/*z*: MH^{+ *b*} |
| **I-39** | 93 | >330 | 3380, 3090 sh, 3060 (NH) | 58.78, 3.65, 22.85 | 365 |
| C₁₈H₁₃ClN₆O ·1/6H₂O | | | | (58.65,3.86, 23.05) | 367 |
| | | | 1710 (CO) | | |
| **I-40** | 87 | >330 | 3360, 3090, 3060 (NH) | 54.15, 3.03, 21.05 | 399 |
| C₁₈H₁₂Cl₂N₆O | | | 1710 (CO) | (54.29, 3.30, 20.95) | 401 |
| | | | | | 403 |
| **I-41** | 89 | >330 | 3360, 3090 sh, 3060 sh (NH) | 59.77, 4.05, 22.01 | 379 |
| C₁₉H₁₅ClN₆O ·1/6 H₂O | | | | (59.61, 4.23, 22.11) | 381 |
| | | | 1710 (CO) | | |
| **I-42** | 82 | > 330 | 3380, 3090 sh, 3070 (NH) | 57.54, 3.86, 21.19 | 395 |
| C₁₉H₁₅ClN₆O₂ ·1/10 H₂O | | | | (57.32, 4.04, 21.11) | 397 |
| | | | 1710 (CO) | | |
| **I-43** | 96 | 229 | 3280, 3180 sh, 3080 sh (NH) | 51.62, 3.13, 23.41 | 410 |
| C₁₈H₁₂ClN₇O₃ ·1/2 H₂O | | (decomp.) | | (51.37, 3.26, 23.71) | 412 |
| | | | 1690 (CO) | | |

| | | | | | |
|---|---|---|---|---|---|
| *^{a}*All compounds were recrystallized from a mixture of DMF and EtOH, and were obtained as colorless powder except for **I-39** and **I-40** (pale yellow powder), **I-43** (orange powder). *^{b}*The matrix is glycerol. | | | | | |

**[table 13]**

| | ¹H-NMR data for compounds **I-39** to **I-43**. |
|---|---|
| Compd. ID | δ_{H} [200 MHz; (CD₃)₂SO; Me₄Si] |
| **I-39** | 7.35-7.50 (3H, m, Ph-*m*,pH), 7.56 (2H, d, *J*_{AB} = 8.6 Hz, Ar-*m*H), 7.97-8.11 (2H, m, Ph-*o*H), 8.13 (2H, d, *J*_{AB} = 8.6 Hz, Ar-*o*H), 8.44 (1H, s, C*H*-Ar), 10.19 (1H, s, exchangeable with D₂O, 6-NH), 11.33 (1H, s, exchangeable with D₂O, 3-NH) |
| **I-40** | 7.49 (2H, d, *J*_{AB} = 8.0 Hz, 6-Ar-*m*H), 7.57 (2H, d, *J*_{AB} = 8.0 Hz, 8-Ar-*m*H), 8.13 (4H, d, *J*_{AB} = 8.0 Hz, Ar-*o*H), 8.42 (1H, s, C*H*-Ar), 10.43 (1H, s, exchangeable with D₂O, 6-NH), 11.37 (1H, s, exchangeable with D₂O, 3-NH) |
| **I-41** | 2.36 (3H, s, Me), 7.25 (2H, d, *J*_{AB} = 8.2 Hz, Mc-Ar-*m*H), 7.56 (2H, d, *J*_{AB} = 8.6 Hz, Cl-Ar-*m*H), 7.93 (2H, d, *J*_{AB} = 8.2 Hz, Me-Ar-*o*H), 8.12 (2H, d, *J*_{AB} = 8.6 Hz, Cl-Ar-*o*H), 8.41 (1H, s, C*H*-Ar), 10.16 (1H, s, exchangeable with D₂O, 6-NH), 11.32 (1H, s, exchangeable with D₂O, 3-NH) |
| **I-42** | 3.82 (3H, s, OMe), 6.99 (2H, d, *J*_{AB} = 8.8 Hz, MeO-Ar-*m*H), 7.55 (2H, d, *J*_{AB} = 8.6 Hz, Cl-Ar-*m*H), 7.98 (2H, d, *J*_{AB} = 8.8 Hz, MeO-Ar-*o*H), 8.12 (2H, d, *J*_{AB} =8.6 Hz, Cl-Ar-*o*H), 8.39 (1H, s, C*H*-Ar), 10.10 (1H, s, exchangeable with D₂O, 6-NH), 11.27 (1H, s, exchangeable with D₂O, 3-NH) |
| **I-43** | 7.58 (2H, d, *J*_{AB} = 8.6 Hz, Cl-Ar-*m*H), 8.14 (2H, d, *J*_{AB} = 8.6 Hz, Cl-Ar-*o*H), 8.27 (2H, d, *J*_{AB} = 8.8 Hz, O₂N-Ar-*o*H), 8.38 (2H, d, *J*_{AB} = 8.8 Hz, O₂N-Ar-*m*H), 8.53 (1H, s, C*H*-Ar), 10.78 (1H, br s, exchangeable with D₂O, 6-NH), 11.52 (1H, br s, exchangeable with D₂O, 3-NH) |

**[table 14]**

| **Physical data for compounds II-1 to II-10.** | | | | | |
|---|---|---|---|---|---|
| **Compd. ID *^{a}* (Formula)** | **Yield (%) (Route)*^{b}*** | **Mp/°C** | ***v*ₘₐₓ (Nujol) / cm⁻¹** | 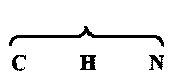 | ***m*/*z*: MH^{+ *c*}** |
| **II-1** | **85 (iii)** | **262** | **3180 sh, 3100 (NH)** | ***d*** | **177** |
| **C₆H₄N₆O** | | **(decomp.)** | **1710 (CO)** | | |
| **II-2** | **87 (iii)** | **277** | **3150, 3100 (NH)** | ***d*** | **191** |
| **C₇H₆N₆O** | | **(decomp.)** | **1720 (CO)** | | |
| **II-3** | **50 (i)** | **> 300** | **3150, 3100 sh (NH)** | ***d*** | **253** |
| **C₁₂H₈N₆O** | **75 (ii)** | | **1710 (CO)** | | |
| | **92 (iii)** | | | | |
| **II-4** | **40 (i)** | **285** | **3140, 3100 (NH)** | ***d*** | **287** |
| **C₁₂H₇ClN₆O** | **76 (ii)** | **(decomp.)** | **1705 (CO)** | | **289** |
| **II-5** | **81 (ii)** | **288** | **3140, 3100 (NH)** | ***d*** | **283** |
| **C₁₃H₁₀N₆O₂** | | **(decomp.)** | **1710 (CO)** | | |
| **II-6** | **80 (i)** | **> 270** | **3140, 3070 sh (NH)** | **53.41, 690, 28.75 (53.66, 6.73, 28.60)** | **275** |
| **C₁₃H₁₈N₆O ·H₂O** | | **(subli.)** | **1720 (CO)** | | |
| **II-7** | **40 (i)** | **> 300** | **3140, 3100 sh (NH)** | **56.72, 4.03, 30.53 (56.53, 4.11, 30.74)** | **267** |
| **C₁₃H₁₀N₆O ·1/2 H₂O** | **80 (ii)** | | **1710 (CO)** | | |
| **II-8** | **87 (ii)** | **> 300** | **3140, 3090 (NH)** | **45.72, 2.88, 31.10 (45.53, 2.91, 30.87)** | **298** |
| **C₁₂H₇N₇O₃ •H₂O** | | | **1760 (CO)** | | |
| **II-9** | **72 (ii)** | **> 283** | **3180 sh, 3110 (NH)** | **48.78, 4.64, 22.75 (48.73, 4.58, 22.59)** | **343** |
| **C₁₅H₁₄N₆O₄ ·3/2 H₂O** | | **(decomp.)** | **1720 (CO)** | | |
| **II-10** | **75 (ii)** | **> 285** | **3180 sh, 3120 sh (NH)** | | **285** |
| **C₁₂H₈N₆O₃** | | **(decomp.)** | **1720 (CO)** | | |

| | | | | | |
|---|---|---|---|---|---|
| *^{a}*All compounds were recrystallized from a mixture of DMF and H₂O except for II-6 (from EtOH), and were obtained as colorless powder. *^{b}*Route i: 70% HNO₃, TFA, r.t.; route ii: chloranil, DMF, reflux; route iii: RC(OEt)₃, AcOH, 80 °C. *^{c}*The matrix is glycerol. *^{d}*Ref. J. Chem. Soc., Perkin Trans. 1, 3117 (1999). | | | | | |

**[table 15]**

| | **¹H-NMR data for compounds II-1 to II-10** |
|---|---|
| **Compd. ID** | **δ_{H} [200 MHz; (CD₃)₂SO; Me₄Si]** |
| **II-1** | **8.12 (1H, s, 2-H), 8.38 (1H, s, 8-H), 12.89 (1H, br, exchangeable with D₂O, 4-NH), 13.77 (1H, br, exchangeable with D₂O, 1-NH)** |
| **II-2** | **2.43 (3H, s, 8-Me), 8.09 (1H, s, 2-H), 12.74 (1H, br, exchangeable with D₂O, 4-NH), 13.67 (1H, br, exchangeable with D₂O, 1-NH)** |
| **II-3** | **7.48-7.64 (3H, m, Ph-*m*,*p*H), 8.10-8.25 (2H, m, Ph-oH), 8.16 (1H, s, 2-H), 12.85 (1H, s, exchangeable with D₂O, 4-NH), 13.89 (1H, s, exchangeable with D₂O, 1-NH)** |
| **II-4** | **7.63 (2H, d, *J*_{AB} = 8.6 Hz, Ar-mH), 8.15 (1H, s, 2-H), 8.17 (2H, d, *J*_{AB} = 8.6 Hz, Ar-*o*H), 12.87 (1H, s, exchangeable with D₂O, 4-NH), 13.88 (1H, s, exchangeable with D₂O, 1-NH)** |
| **II-5*^{a}*** | **3.84 (3H, s, OMe), 7.20 (2H, d, *J*_{AB} = 8.7 Hz, Ar-mH), 8.10 (2H, d, *J*_{AB} = 8.7 Hz, Ar-*o*H), 8.11 (1H, s, 2-H), 12.77 (1H, s, exchangeable with D₂O, 4-NH), 13.82 (1H, br, exchangeable with D₂O, 1-NH)** |
| **II-6** | **0.86 (3H, t, *J* = 6.6 Hz, CH₂CH₂[CH₂]₄*Me*), 1.27 (8H, br s, CH₂CH₂[CH₂]₄Me), 1.64-1.85 (2H, m, CH₂C*H*₂[CH₂]₄Me), 2.76 (2H, t, *J* = 7.4 Hz, C*H*₂CH₂[CH₂]₄Me), 8.08 (1H, s, 2-H), 12.69 (1H, s, exchangeable with D₂O, 4-NH), 13.73 (1H, br, exchangeable with D₂O, 1-NH)** |
| **II-7** | **2.39 (3H, s, Me), 7.37 (2H, d, *J*_{AB} = 8.2 Hz, Ar-*m*H), 8.06 (2H, d, *J*_{AB} = 8.2 Hz, Ar-*o*H), 8.14 (1H, s, 2-H), 12.80 (1H, s, exchangeable with D₂O, 4-NH), 13.85 (1H, s, exchangeable with D₂O, 1-NH)** |
| **II-8*^{a}*** | **8.17 (1H, s, 2-H), 8.41 (4H, s, Ar-H), 12.93 (1H, s, exchangeable with D₂O, 4-NH), 13.90 (1H, s, exchangeable with D₂O, 1-NH)** |
| **II-9*^{a}*** | **3.74 (3H, s, 4'-OMe), 3.89 (6H, s, 3'-OMe and 5'-OMe), 7.44 (2H, s, 2'-H and 6'-H), 8.13 (1H, s, 2-H), 12.82 (1H, s, exchangeable with D₂O, 4-NH), 13.86 (1H, s, exchangeable with D₂O, 1-NH)** |
| **II-10j*^{a}*** | **6.86 (1H, d, J_{5',6'} = 8.1 z, 5'-H), 7.46 (1H, d, *J*_{5',6'} = 8.1 Hz, 6'-H), 7.57 (1H, s, 2'-H), 8.10 (1H, s, 2-H), 8.47 (1H, br, exchangeable with D₂O, 3'-OH), 9.33 (1H, br, exchangeable with D₂O, 4'-OH), 12.72 (1H, s, exchangeable with D₂O, 4-NH), 13.89 (1H, br, exchangeable with D₂O, 1-NH)** |

| | |
|---|---|
| *^{a}*This compound was measured at 300 MHz in (CD₃)₂SO. | |

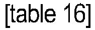

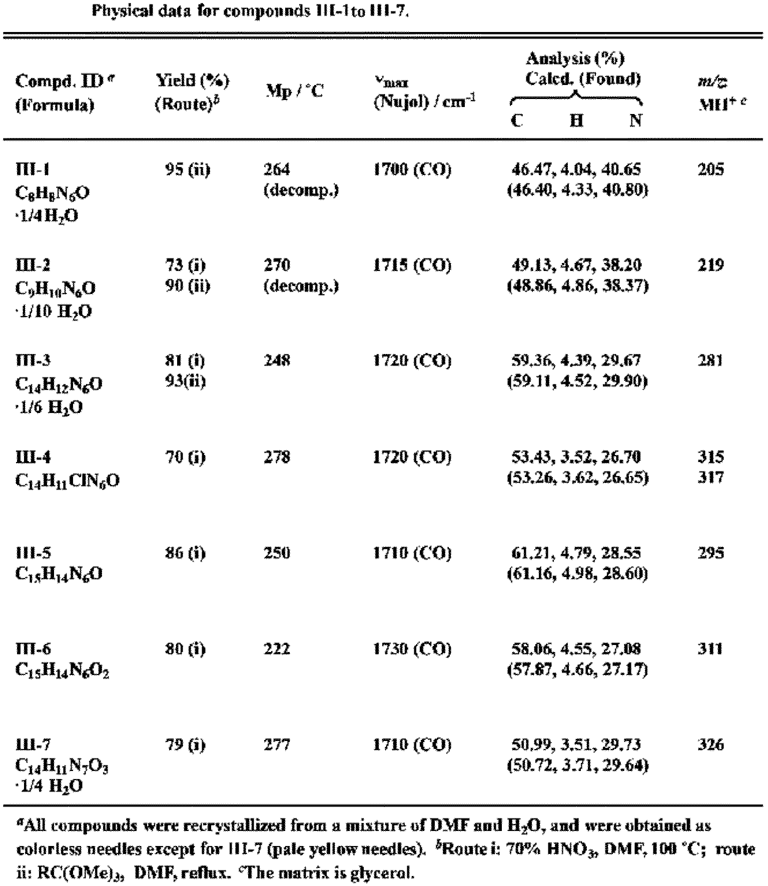

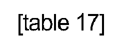

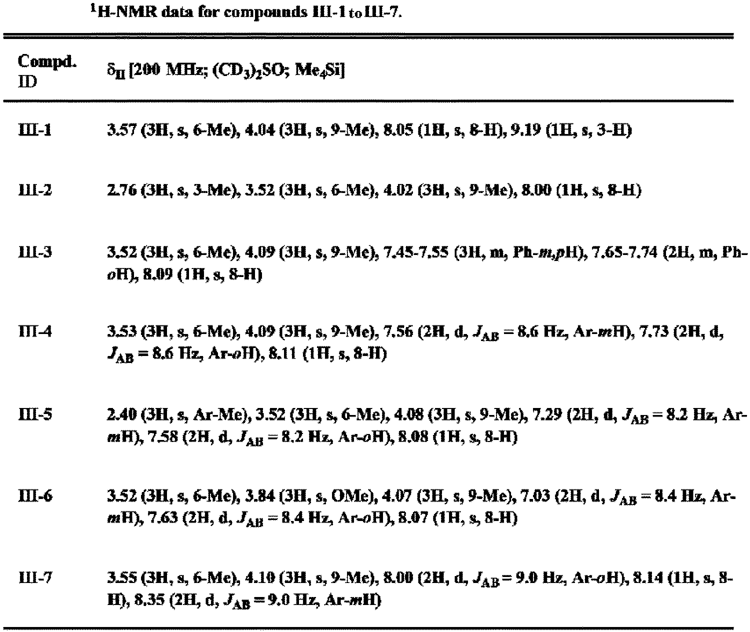

**[table 18]**

| **Physical data for compounds 14a-g.** | | | | | |
|---|---|---|---|---|---|
| **Compd. ID*^{a}* (Formula)** | **Yield (%)** | **Mp/°C** | ***ν*ₘₐₓ(Nujol) / cm⁻¹** | 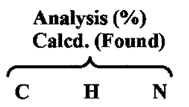 | ***m*/*z*: MH^{+ *b*}** |
| | | | | | |
| **14a** | **80** | **174** | **3120 (NH)** | **45.41, 5.17, 35.31** | **237** |
| **C₉H₁₂N₆O₂ •1/10 H₂O** | | **(convert)** | **1710 (CO)** | **(45.36, 5.16, 35.34)** | |
| **14b** | **73** | **146** | **3120 (NH)** | **47.99,5.64, 33.58** | **251** |
| **C₁₀H₁₄N₆O₂** | | **(convert)** | **1700 (CO)** | **(47.82,5.68, 33.86)** | |
| **14c** | **91** | **259** | **3120 (NH)** | **57.68, 5.16, 26.91** | **313** |
| **C₁₅H₁₆N₆O₂** | | **(convert)** | **1710 (CO)** | **(57.76, 5.35, 27.04)** | |
| **14d** | **78** | **235** | **3090 (NH)** | **51.95, 4.36, 24.24** | **347** |
| **C₁₅H₁₅ClN₆O₂** | | **(convert)** | **1700 (CO)** | **(51.69,4.26,24.47)** | **349** |
| **14e** | **94** | **217** | **3090 (NH)** | **58.88,5.56, 25.75** | **327** |
| **C₁₆H₁₈N₆O₂** | | **(convert)** | **1720 (CO)** | **(58.60, 5.63, 25.51)** | |
| **14f** | **89** | **221** | **3120 (NH)** | **56.13, 5.30, 24.55** | **343** |
| **C₁₆H₁₈N₆O₃** | | **(convert)** | **1690 (CO)** | **(56.13, 5.26, 24.84)** | |
| **14g** | **86** | **262** | **3110 (NH)** | **49.92, 4.30, 27.17** | **358** |
| **C₁₅H₁₅N₇O₄ ·1/5 H₂O** | | **(convert)** | **1710 (CO)** | **(49.82,4.26, 27.43)** | |

| | | | | | |
|---|---|---|---|---|---|
| *^{a}*All compounds were recrystallized from EtOH except for 14a and 14b (from AcOEt) and 14g (from a mixture of DMF and EtOH), and were obtained as colorless powder except for 14a (colorless needles) and 14g (yellow powder). *^{b}*The matrix is glycerol. | | | | | |

**[table 19]**

| | **¹H-NMR data for compounds 14a-g.** |
|---|---|
| **Compd. ID** | **δ_{H} [300 MHz; CDCl₃; Me₄Si]** |
| **14a** | **3.26 (3H, s, MeOOC-N*Me*), 3.81 (3H, br s, imidazole-NMe), 3.91 (3H, s, *Me*OOC-NMe), 7.47 (1H, s, imidazole-CH), 8.11 (1H, s, triazole-CH), 12.13 (1H, br, exchangeable with D₂O, NH)** |
| **14b** | **2.48 (3H, s, triazole-CMe), 3.24 (3H, s, MeOOC-N*Me*), 3.76 (3H, br s, imidazole-NMe), 3.90 (3H, s, *Me*OOC-NMe), 7.43 (1H, s, imidazole-CH), 11.72 (1H, br, exchangeable with D₂O, NH)** |
| **14c** | **3.27 (3H, s, MeOOC-N*Me*), 3.85 (3H, s, imidazole-NMe), 3.96 (3H, s, *Me*OOC-NMe), 7.40-7.54 (3H, m, Ph-*m*,*p*H), 7.48 (1H, s, imidazole-CH), 8.07-8.20 (2H, m, Ph-*o*H), 12.02 (1H, br, exchangeable with D₂O, NH)** |
| **14d** | **3.27 (3H, s, MeOOC-N*Me*), 3.86 (3H, s, imidazole-NMe), 3.95 (3H, s, *Me*OOC-NMe), 7.38 (2H, d, *J*_{AB} = 8.1 Hz, Ar-*m*H), 7.49 (1H, s, imidazole-CH), 8.07 (2H, d, *J*_{AB} = 8.1 Hz, Ar-*o*H), 12.20 (1H, br, exchangeable with D₂O, NH)** |
| **14e** | **2.41 (3H, s, Ar-Me), 3.27 (3H, s, MeOOC-N*Me*), 3.84 (3H, br s, imidazole-NMe), 3.96 (3H, s, *Me*OOC-NMe), 7.26 (2H, d, *J*_{AB} = 7.2 Hz, Ar-*m*H), 7.48 (1H, s, imidazole-CH), 8.00 (2H, d, *J*_{AB} = 7.2 Hz, Ar-*o*H), 12.10 (1H, br, exchangeable with D₂O, NH)** |
| **14f** | **3.27 (3H, s, MeOOC-N*Me*), 3.84 (3H, br s, imidazole-NMe), 3.88 (3H, s, Ar-OMe), 3.96 (3H, s, *Me*OOC-NMe), 6.98 (2H, d, *J*_{AB} = 8.7 Hz, Ar-*m*H), 7.47 (1H, s, imidazole-CH), 8.04 (2H, d, *J*_{AB} = 8.7 Hz, Ar-*o*H), 12.04 (1H, br, exchangeable with D₂O, NH)** |
| **14g** | **3.28 (3H, s, MeOOC-N*Me*), 3.89 (3H, s, imidazole-NMe), 3.98 (3H, s, *Me*OOC-NMe), 7.53 (1H, s, imidazole-CH), 8.32 (4H, s, Ar-H)** |

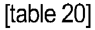

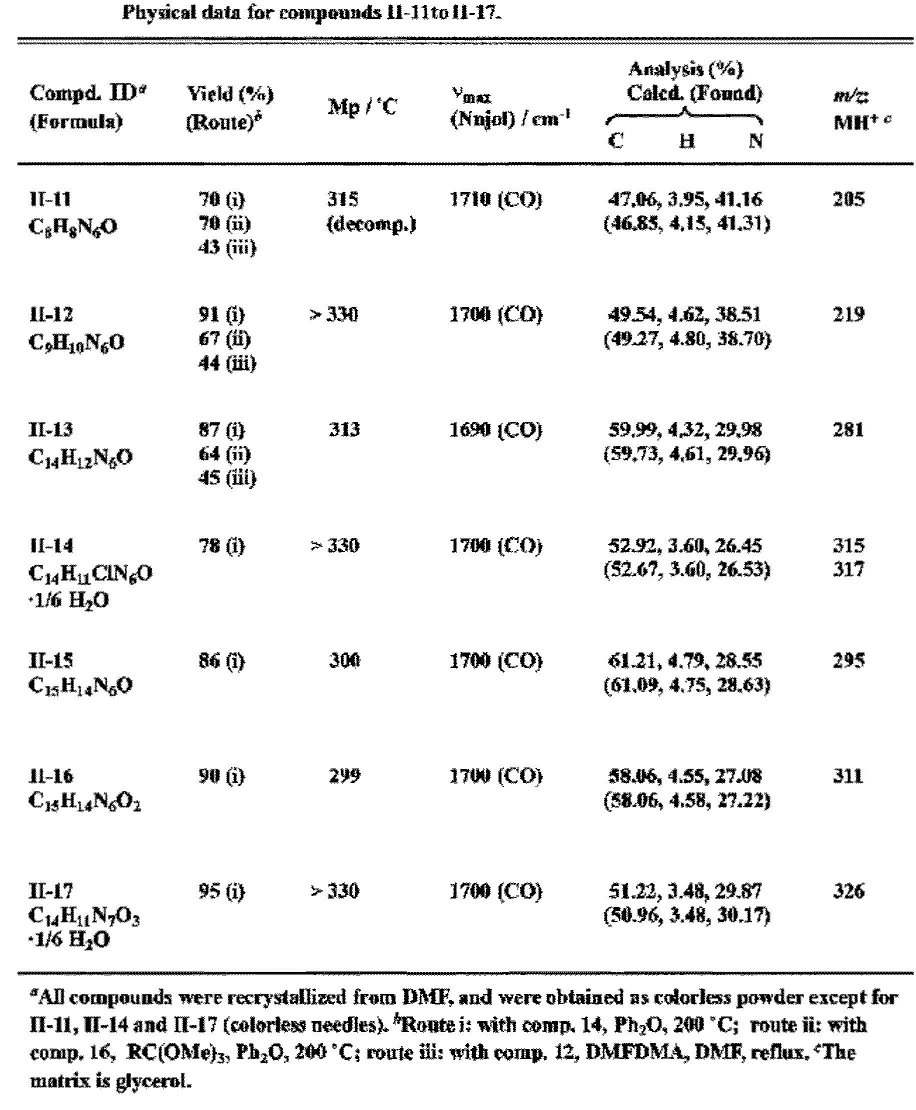

**[table 21]**

| | **¹H-NMR data for compounds II-11 to II-17.** |
|---|---|
| **Compd. ID** | **δ_{H} [200 MHz; (CD₃)₂SO; Me₄Si]** |
| **II-11** | **3.65 (3H, s, 4-Me), 4.04 (3H, s, 1-Me), 8.17 (1H, s, 2-H), 8.41 (1H, s, 8-H)** |
| **II-12** | **2.45 (3H, s, 8-Me), 3.63 (3H, s, 4-Me), 4.02 (3H, s, 1-Me), 8.15 (1H, s, 2-H)** |
| **II-13** | **3.67 (3H, s, 4-Me), 4.10 (3H, s, 1-Me), 7.50-7.65 (3H, m, Ph-*m*,*p*H), 8.13-8.28 (2H, m, Ph-*o*H), 8.18 (1H, s, 2-H)** |
| **II-14** | **3.67 (3H, s, 4-Me), 4.11 (3H, s, 1-Me), 7.63 (2H, d, *J*_{AB} = 8.4 Hz, Ar-*m*H), 8.20 (1H, s, 2, H), 8.22 (2H, d, *J*_{AB} = 8.4 Hz, Ar-*o*H)** |
| **II-15** | **2.40 (3H, s, Ar-Me), 3.66 (3H, s, 4-Me), 4.10 (3H, s, 1-Me), 7.36 (2H, d, *J*_{AB} - 8.2 Hz, Ar-*m*H), 8.09 (2H, d, *J*_{AB} = 8.2 Hz, Ar-*o*H), 8.19 (1H, s, 2-H)** |
| **II-16** | **3.66 (3H, s, 4-Me), 3.85 (3H, s, OMe), 4.10 (3H, s, 1-Me), 7.11 (2H, d, *J*_{AB} = 8.8 Hz, Ar-*m*H), 8.14 (2H, d, *J*_{AB} = 8.8 Hz, Ar-*o*H), 8.19 (1H, s, 2-H)** |
| **II-17** | **3.69 (3H, s, 4-Me), 4.13 (3H, s, 1-Me), 8.24 (1H, s, 2-H), 8.45 (4H, s, Ar-H)** |

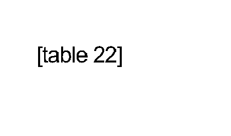

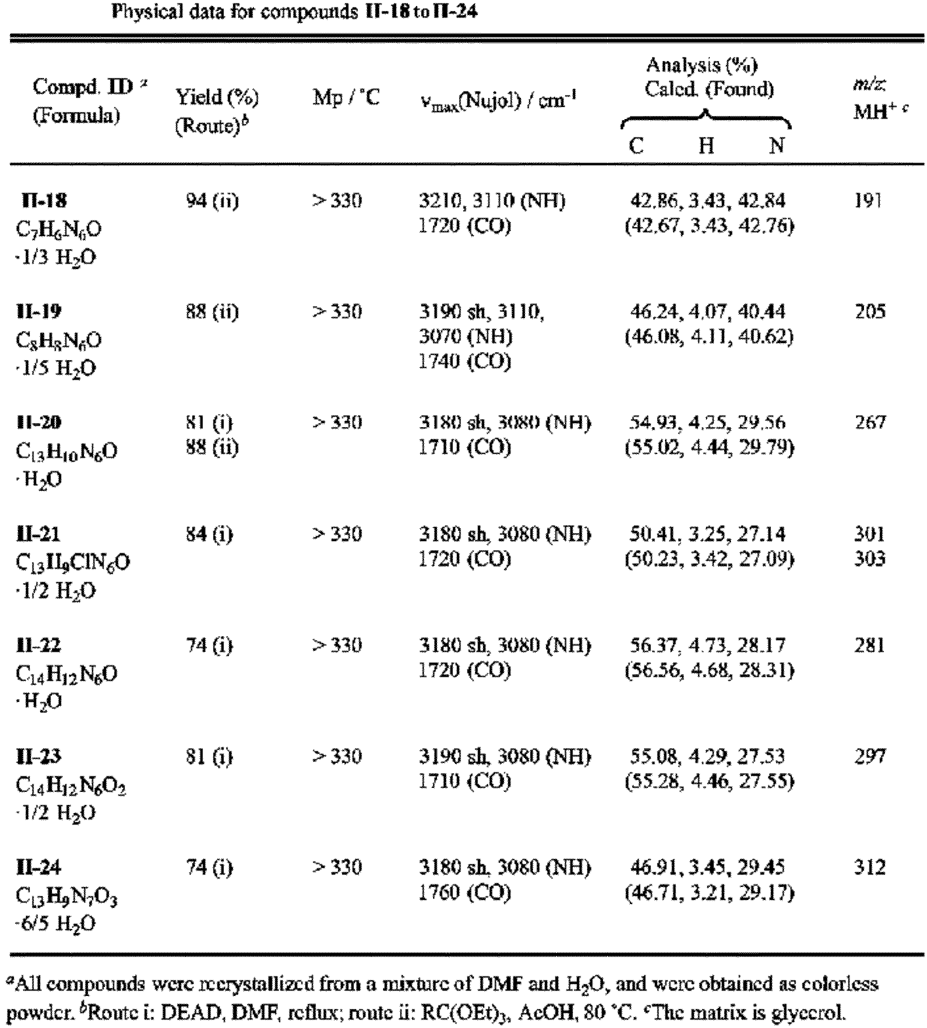

**[table 23]**

| | **¹H-NMR data of compounds II-18 to II-24** |
|---|---|
| **Compd. ID** | **δₙ [200 MHz; (CD₃)₂SO; Me₄Si]** |
| **II-18** | **2.44 (3II, s, 2-Me), 8.32 (1H, s, 8-II), 12.72 (1II, s, exchangeable with D₂O, 4-NII), 13.42 (1H, s, exchangeable with D₂O, 1-NH)** |
| **II-19** | **2.41 (3H, s, 2-Me), 2.43 (3H, s, 8-Me), 12.58 (1H, s, exchangeable with D₂O, 4-NH), 13.32 (lH, s, exchangeable with D₂O, 1-NH)** |
| **II-20** | **2.45 (3H, s, 2-Me), 7.47-7.59 (3H, m, Ph-*m*,*p*H), 8.10-8.22 (2H, m, Ph-*o*H), 12.72 (1H, s, exchangeable with D₂O, 4-NH), 13.31 (1H, s, exchangeable with D₂O, 1-NH)** |
| **II-21** | **2.46 (3H, s, 2-Me), 7.62 (2H, d, *J*_{AB} = 8.6 Hz, Ar-mH), 8.16 (2H, d, *J*_{AB} = 8.6 Hz, Ar-*o*H), 12.77 (1H, s, exchangeable with D₂O, 4-NH), 13.52 (1H, s, exchangeable with D₂O, 1-NH)** |
| **II-22** | **2.39 (3H, s, Ar-Me), 2.45 (3H, s, 2-Me), 7.36 (2H, d, *J*_{AB} = 8.2 Hz, Ar-mH), 8.05 (2H, d, *J*_{AB} = 8.2 Hz, Ar-oH), 12.70 (1H, s, exchangeable with D₂O, 4-NH), 13.49 (1H, s, exchangeable with D₂O, 1-NH)** |
| **II-23** | **2.45 (3H, s, 2-Me), 3.84 (3H, s, OMe), 7.10 (2H, d, *J*_{AB} = 8.8 Hz, Ar-mH), 8.09 (2H, d, *J*_{AB} = 8.8 Hz. Ar-oH), 12.67 (1H, s, exchangeable with D₂O, 4-NH), 13.48 (1H, s, exchangeable with D₂O, 1-NH)** |
| **II-24** | **2.46 (3H, s, 2-Me), 8.41 (4H, s, Ar-H), 12.85 (1H, br s, exchangeable with D₂O, 4-NH), 13.56 (1H, br s, exchangeable with D₂O, 1-NH)** |

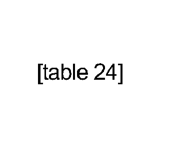

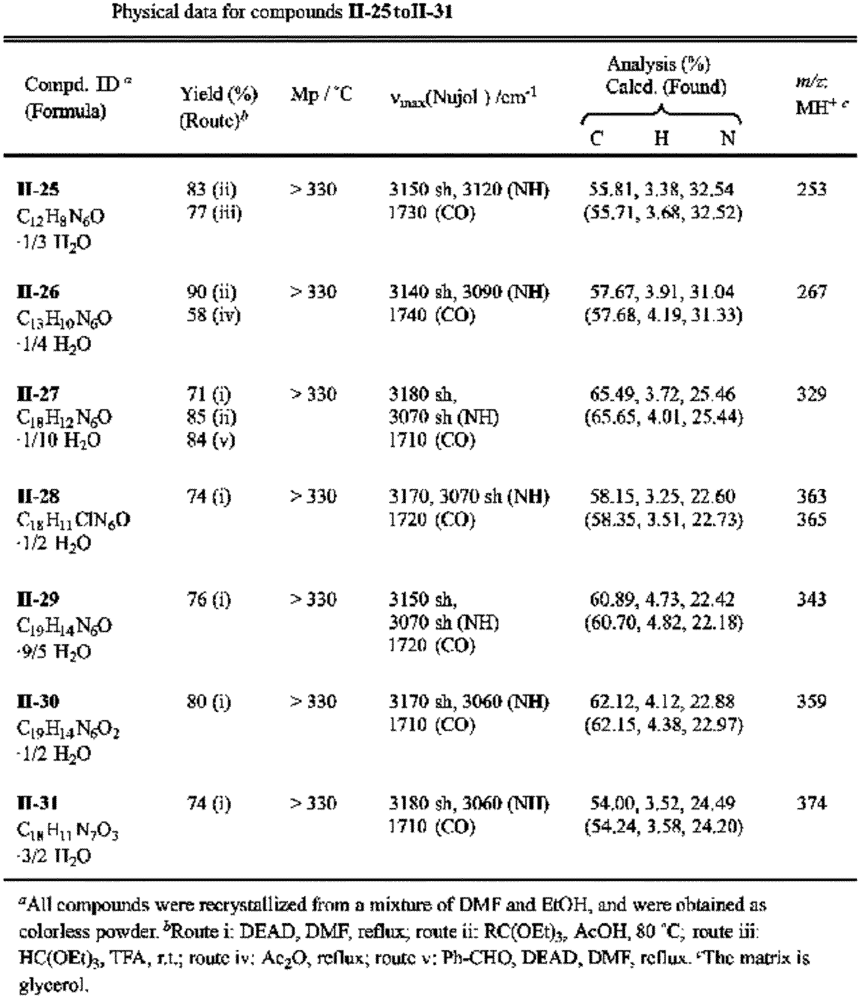

**[table 25]**

| | **¹H-NMR data for compounds II-25 to II-31** |
|---|---|
| **Compd. ID** | **δ_{H} [200 MHz; (CD₃)₂SO; Me₄Si]** |
| **II-25** | **7.42-7.60 (3H, m, Ph-*m*,*p*H), 8.05-8.21 (2H, m, Ph-*o*H), 8.41 (1H, s, 8-H), 12.92 (1H, s, exchangeable with D₂O, 4-NH), 14.14 (1H, s, exchangeable with D₂O, 1-NH)** |
| **II-26** | **2.45 (3H, s, 8-Me), 7.42-7.60 (3H, m, Ph-*m,p*H), 8.05-8.20 (2H, m, Ph-oH), 12.78 (1H, s, exchangeable with D₂O, 4-NH), 14.08 (1H, s, exchangeable with D₂O, 1-NH)** |
| **II-27** | **7.43-7.64 (6H, m, Ph-*m*,*p*H), 8.10-8.28 (4H, m, Ph-oH), 12.94 (1H, s, exchangeable with D₂O, 4-NH), 14.21 (1H, s, exchangeable with D₂O, 1-NH)** |
| **II-28** | **7.44- 7.60 (3H, m, Ph-*m*,*p*H ), 7.65 ( 2H, d, *J*_{AB} = 8.4 Hz, Ar-*m*H ), 8.06-8.21 ( 2H, m, Ph-*o*H ), 8.20 ( 2H, d, *J*_{AB} = 8.4 Hz, Ar-*o*H ), 12.97 ( 1H, s, exchangeable with D₂O, 4-NH), 14.21 (1H, s, exchangeable with D₂O, 1-NH )** |
| **II-29** | **2.40 (3H, s, Mc), 7.38 (2H, d, *J*_{AB} = 8.2 Hz, Ar-mH), 7.43-7.62 (3H, m, Ph-*m*,*p*H), 8.08 (2H, d, *J*_{AB} = 8.2 Hz, Ar-*o*H), 8.10-8.21 (2H, m, Ph-*o*H), 12.90 (1H, s, exchangeable with D₂O, 4-NH), 14.18 (1H, s, exchangeable with D₂O, 1-NH)** |
| **II-30** | **3.85 (3H, s, OMe), 7.12 (2H, d, *J*_{AB} = 9.0 Hz, Ar-*m*H), 7.42-7.64 (3H, m, Ph-*m*,*p*H), 8.13 (2H, d, *J*_{AB} - 9.0 Hz, Ar-*o*H), 8.06-8.25 (2H, m, Ph-*o*H), 12.88 (1H, s, exchangeable with D₂O, 4-NH), 14.17 (1H, s, exchangeable with D₂O, 1-NH)** |
| **II-31** | **7.40-7.64 (3H, m, Ph-*m*,*p*H), 8.06-8.22 (2H, m, Ph-*o*H), 8.43 (4H, s, Ar-H), 13.04 (1H, br s, exchangeable with D₂O, 4-NH), 14.25 (1H, br s, exchangeable with D₂O, 1-NH)** |

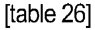

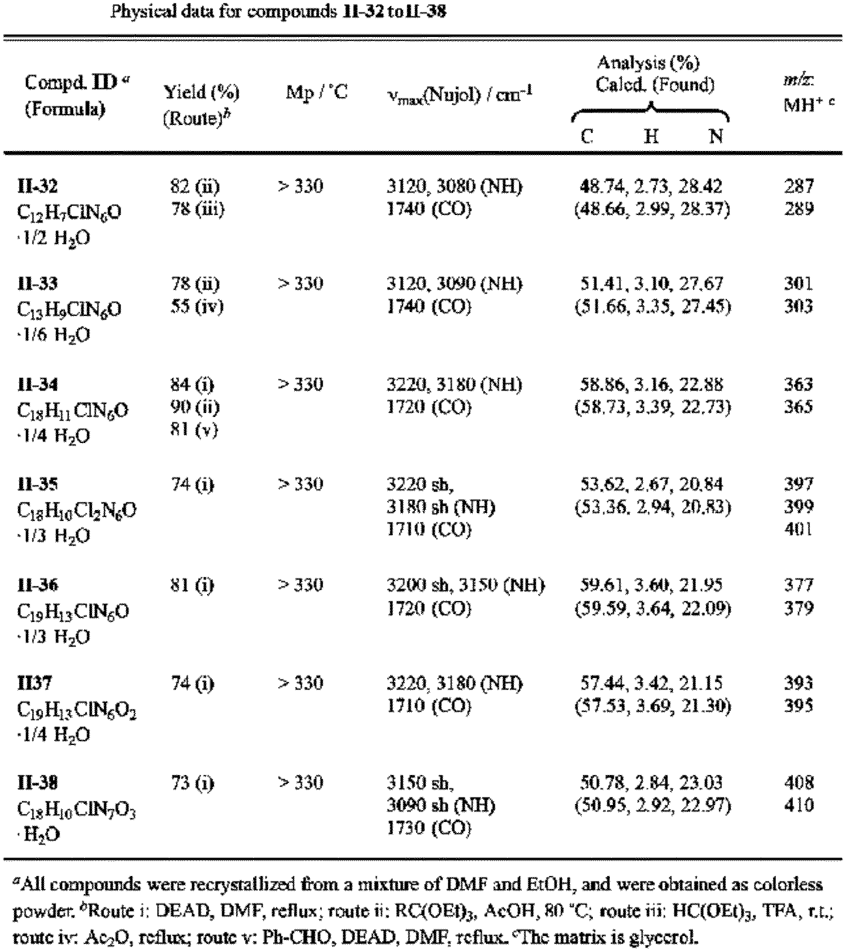

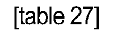

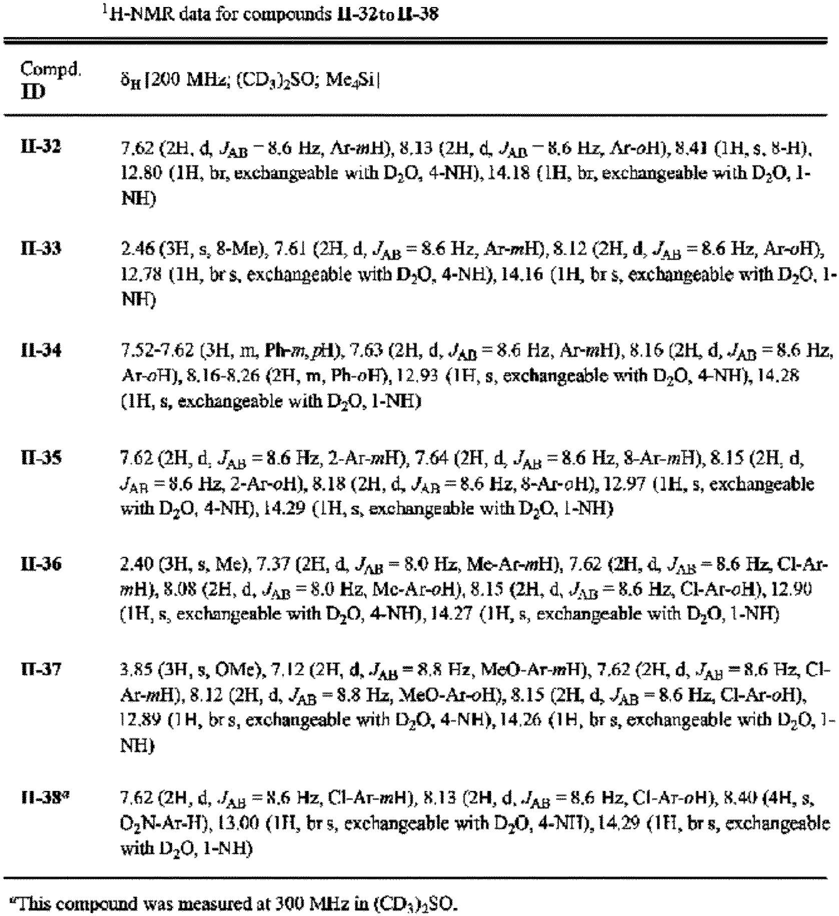

### [Example 4. Evaluation of the compounds]

### Test Example 1: Xanthine oxidase inhibition activity

Xanthine oxidase inhibition activity was measured for the compounds synthesized in Examples 1 and 2.

### 1. Measurement method

In a 50 mM phosphate buffer (pH 7.4), xanthine (100 pM), cow milk-derived xanthine oxidase (XOD) (10 mU/mL), and a test compound were mixed and the mixture was incubated at room temperature for 15 minutes. Subsequently, the conversion of xanthine to uric acid was determined as the change in optical density (O. D.) at 292 nm to determine the activity of the test compound to inhibit formation of uric acid.

Percent inhibition of uric acid formation was determined by the following equation and the dose-response curve was generated for each test compound to calculate the 50% inhibitory concentration (IC₅₀, µM). Allopurinol was used as control compound.

Percent (%) inhibition of uric acid formation = 100 - [(D - D_{B}) / T] × 100, where T is O.D. of (xanthine + XOD) solution; D is O.D. of (test compound + xanthine + XOD) solution; and D_{B} is O.D. of (test compound + XOD) solution.

### 2. Evaluation

The results of calculated IC₅₀ are given in the table below.

**[table 28]**

| **Compound ID** | | | **IC₅₀ (µM)** | **Compound ID** | | **IC₅₀ (µM)** | |
|---|---|---|---|---|---|---|---|
| | **I-1** | | **0.038** | | **I-34** | **>10** | |
| | **I-2** | | **0.025** | | **I-35** | **>10** | |
| | **I-3** | | **0.075** | | **I-36** | **>10** | |
| | **I-4** | | **0.045** | | **I-37** | | **9.210** |
| | **I-5** | | **0.057** | | **I-38** | | **4.480** |
| | **I-6** | | **0.063** | | **II-1** | **>10** | |
| | **I-7** | | **0.045** | | **II-2** | **>10** | |
| | **I-8** | | **2.020** | | **II-3** | | **2.570** |
| | **I-9** | | **0.050** | | **II-4** | | **0.066** |
| | **I-10** | | **0.020** | | **II-5** | | **0.130** |
| | **I-11** | | **0.208** | | **II-6** | | **9.200** |
| | **I-12** | | **0.349** | | **II-7** | | **2.400** |
| | **I-13** | | **1.478** | | **II-8** | | **4.270** |
| | **I-14** | | **0.079** | | **II-9** | | **2.010** |
| | **I-15** | | **0.100** | | **II-10** | | **1.480** |
| | **I-16** | | **0.076** | | **II-14** | **>10** | |
| | **I-17** | | **0.116** | | **II-17** | **>10** | |
| | **I-18** | | **0.093** | | **II-24** | **>10** | |
| | **I-19** | **>10** | | | **II-25** | | **9.910** |
| | **I-20** | **>10** | | | **II-31** | **>10** | |
| | **I-21** | **>10** | | | **II-32** | | **1.090** |
| | **I-22** | **>10** | | **allopurinol** | | **24.3** | |

As shown in Table 28, the 50% inhibitory concentration (IC₅₀) for XOD of each of the hydrazinopurine compounds of Compound IDs 1-8 to I-13, I-37, and I-38 was lower than 10 µM, which was approximately 2.6- to 1200-fold lower than the IC₅₀ of allopurinol, an existing xanthine oxidase inhibitor. In other words, the hydrazinopurine compounds of Compound IDs I-8 to 1-13, I-37, and I-38 each showed higher xanthine oxidase inhibition activity than allopurinol.

Also, the 50% inhibitory concentration (IC₅₀) for XOD of each of the triazolopurine compounds of Compound IDs II-3 to II-10, II-25, and II-32 was lower than 10 µM, which was approximately 2.5- to 370-fold lower than the IC₅₀ of allopurinol, an existing xanthine oxidase inhibitor. In other words, the triazolopurine compounds of Compound IDs II-3 to II-10, II-25, and II-32 each showed higher xanthine oxidase inhibition activity than allopurinol.

Thus, the hydrazinopurine compounds (I) and the triazolopurine compounds (II) exhibited higher xanthine oxidase inhibition activity, in particular, the hydrazinopurine compounds of Compound IDs I-8 to 1-13, 1-37, and I-38 and the triazolopurine compounds of Compound IDs II-3 to II-10, II-25, and II-32 exhibited higher xanthine oxidase inhibition activity than allopurinol. This demonstrates that a composition containing the hydrazinopurine compound (I) and the triazolopurine compound (II) is useful as a composition for inhibiting xanthine oxidase and thus, as a pharmaceutical composition for preventing or treating hyperuricemia, gout and other diseases caused by hyperuricemia.

### Test Example 2: Evaluation of docking between xanthine oxidoreductase and ligand

The docking score to xanthine oxidoreductase (binding free energy, kcal/mol) was calculated for the compounds synthesized in Examples 1 and 2.

### 1. Calculation Method

According to the calculation method described in J Comput Aided Mol Des (2010) 24: 57-75, the docking scores of the compounds synthesized in Examples 1 and 2 to xanthine oxidoreductases (XORs) (PDB codes: 1 N5X and 1V97) were calculated. As control compounds, the same calculation was performed for allopurinol, oxypurinol, uric acid and Febuxostat.

### 2. Results of Docking Score Calculation

The results of docking score to XOR of PDB code 1 N5X are presented in Table 29 and the results of docking score to XOR of PDB code 1 V97 are presented in Table 30.

**[table 29]**

| **Results of ligand docking to XOR (PDB code IN5X) using MOPAC of BioMed CACheA** | | | |
|---|---|---|---|
| Compound ID Ligand | Docking score (Kcal/moL) | Compound ID Ligand | Docking score (Kcal/moL) |
| I-1 | -106.49 | II-1 | -103.95 |
| I-2 | -104.57 | II-2 | -104.30 |
| I-3 | -87.17 | II-3 | -119.29 |
| I-4 | -104.19 | II-4 | -121.10 |
| I-5 | -100.38 | II-5 | -130.29 |
| I-6 | -108.15 | II-6 | -123.75 |
| I-7 | -97.48 | II-7 | -120.61 |
| I-8 | -74.15 | II-8 | -128.82 |
| I-10 | -88.53 | II-9 | -134.55 |
| I-11 | -101.86 | II-10 | -130.55 |
| I-12 | -110.91 | **oxypurinol** | -82.16 |
| I-13 | -107.80 | **uric acid** | -80.43 |
| **allopurinol** | -88.04 | **Febuxostat** | -109.17 |

**[table 30]**

| | Results of ligand docking to XOR (PDB code 1V97) using MOPAC of BioMed CACheA | | |
|---|---|---|---|
| Compound ID Ligand | Docking score (Kcal/moL) | Compound ID Ligand | Docking score (Kcal/moL) |
| I-1 | -106.79 | II-1 | -106.55 |
| I-2 | -106.25 | II-2 | -108.67 |
| I-3 | -86.20 | II-3 | -126.36 |
| I-4 | -95.70 | II-4 | -128.16 |
| I-5 | -105.69 | II-5 | -127.39 |
| I-6 | -102.55 | II-6 | -126.44 |
| I-7 | -108.36 | II-7 | -121.22 |
| I-8 | -98.96 | II-8 | -132.35 |
| I-10 | -93.29 | II-9 | -137.14 |
| I-11 | -85.01 | II-10 | -128.23 |
| I-12 | -105.57 | oxypurinol | -86.87 |
| I-13 | -110.36 | uric acid | -84.18 |
| allopurinol | -87.20 | Febuxostat | -86.23 |

As shown in Tables 29 and 30, the triazolopurine compounds of Compound IDs II-3 to II-10 had lower docking scores to both of XORs of PDB code 1 N5X and PDB code IV97 as compared to allopurinol, oxypurinol, uric acid and Febuxostat, suggesting that their state bound to XORs are more stable than those of allopurinol, oxypurinol, uric acid and Febuxostat. The triazolopurine compounds of Compound IDs II-3 to II-10 also had lower docking scores as compared to the hydrazinopurine compounds of Compound IDs 1-1 to I-8 and 1-10 to 1-13, suggesting that the state of each the triazolopurine compounds of Compound IDs II-3 to II-10 bound to XORs is more stable than that of each of the hydrazinopurine compounds of Compound IDs 1-1 to I-8 and 1-10 to 1-13. In the in vitro activity test in Test Example 1 described above (test for xanthine oxidase inhibition activity), the hydrazinopurine compounds of Compound IDs 1-1 to I-8 and 1-10 to 1-13 generally showed stronger xanthine oxidase inhibition activity than the triazolopurine compounds of Compound IDs II-3 to II-10. This is thought to be because in addition to inhibiting the enzyme, the hydrazinopurine compounds particularly exhibit competitive inhibition with xanthine, similar to allopurinol.

For the docking scores to XOR of PDB code 1 N5X (Table 29), the hydrazinopurine compounds of Compound IDs 1-10 to 1-13 and the triazolopurine compounds of Compound IDs II-1 and II-2 each showed a lower docking score than allopurinol, oxypurinol and uric acid, indicating that their state bound to XOR is more stable than that of allopurinol, oxypurinol and uric acid.

Also, for the docking scores to XOR of PDB code 1 V97 (Table 30), the hydrazinopurine compounds of Compound IDs I-8, 1-10, 1-12 and 1-13 and the triazolopurine compounds of Compound IDs II-1 and II-2 each showed a lower docking scores than allopurinol, oxypurinol, uric acid and Febuxostat, indicating that their state bound to XOR is more stable than that of allopurinol, oxypurinol, uric acid and Febuxostat.

Thus, the hydrazinopurine compounds (I) and the triazolopurine compounds (II) of the present disclosure are expected to remain bound to XOR in a sustained manner to ensure high bioavailability. This demonstrates that the composition containing any of the hydrazinopurine compounds (I) and the triazolopurine compounds (II) of the present disclosure is useful as a composition for inhibiting xanthine oxidase and thus, as a pharmaceutical composition for preventing or treating hyperuricemia, gout and other diseases caused by hyperuricemia.

### [Example 5. Preparation of Formulation]

Preparation examples of the composition for inhibiting xanthine oxidase using the compounds of the present invention as well as formulations for preventing or treating hyperuricemia, gout and other diseases caused by hyperuricemia will now be presented. Formulation Example 1: Tablets

| | |
|---|---|
| Compound of the invention | 30.0 mg |
| Fine powder cellulose | 25.0 mg |
| Lactose | 39.5 mg |
| Starch | 40.0 mg |
| Talc | 5.0 mg |
| Magnesium stearate | 0.5 mg |

Tablets are prepared from the above composition using a standard method.

### Formulation Example 2: Capsules

| | |
|---|---|
| Compound of the invention | 30.0 mg |
| Lactose | 40.0 mg |
| Starch | 15.0 mg |
| Talc | 5.0 mg |

Capsules are prepared from the above composition using a standard method.

### Formulation Example 3: Injections

| | |
|---|---|
| Compound of the invention | 30.0 mg |
| Glucose | 100.0 mg |

Injections are prepared by dissolving the above composition in purified water for injections.

### Industrial Applicability

The triazolopurine compounds of the present invention exhibit superior xanthine oxidase inhibitory activity to conventional allopurinol in many derivatives and are thus suitable for use in a pharmaceutical composition for preventing or treating hyperuricemia or gout attacks. The compounds of the present invention are also suitable for use in a pharmaceutical composition for preventing or treating kidney disorders such as kidney failure and chronic kidney diseases, hypertension, cardiovascular diseases, dyslipidemia and metabolic syndrome.

## Claims

1. A triazolopurine compound represented by the following general formula (II): 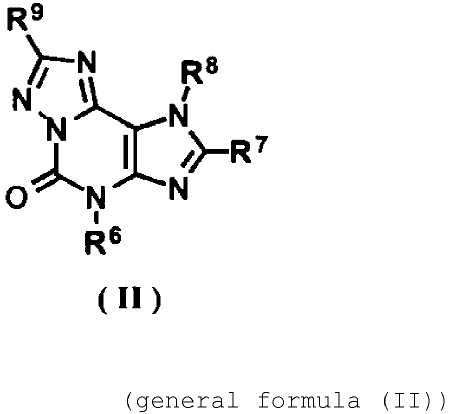 wherein R⁶ represents a hydrogen atom, and R⁸ represents a hydrogen atom or an alkyl group; R⁷ and R⁹ each independently represent a hydrogen atom, an alkyl group, or a phenyl group having a substituent selected from: a halogen atom, an alkyl group, an alkoxy group, an amino group, an alkylamino group, a methylenedioxy group, a hydroxy group or nitro group.

2. The compound according to claim 1, wherein R⁷ and R⁹ each independently are selected from hydrogen, alkyl, a methylphenyl, ethylphenyl, methoxyphenyl, ethoxyphenyl, dimethylaminophenyl, diethylaminophenyl, fluorophenyl, chlorophenyl, bromophenyl, iodophenyl, methylenedioxyphenyl; hydroxyphenyl; and nitrophenyl.

3. The compound according to claim 1 or 2, wherein R⁶, R⁷ and R⁸ are all hydrogen atom, and R⁹ is selected from hydrogen atom, alkyl group, or a phenyl group having a substituent selected from: a halogen atom, an alkyl group, an alkoxy group, an amino group, an alkylamino group, a methylenedioxy group, a hydroxy group or nitro group .

4. The compound according to claim 1, wherein R⁶ and R⁸ are both hydrogen atom, and
- R⁷ is an alkyl group or an aryl group selected from a phenyl group, and a phenyl group having a substituent selected from: a halogen atom, an alkyl group, an alkoxy group, an amino group, an alkylamino group, a methylenedioxy group, a hydroxy group or nitro group, and
- R⁹ is hydrogen atom, an alkyl group, or a phenyl group having a substituent selected from: a halogen atom, an alkyl group, an alkoxy group, an amino group, an alkylamino group, a methylenedioxy group, a hydroxy group or nitro group.

5. The compound according to claim 1 or 2, wherein R⁶, R⁷, R⁸, and R⁹ each are as shown in the table:
| | R⁶ | R⁷ | R⁸ | R⁹ |
|---|---|---|---|---|
| II-1 | H | H | H | H |
| II-2 | H | H | H | Me |
| II-4 | H | H | H | 4-Cl-C₆H₄ |
| II-5 | H | H | H | 4-MeO-C₆H₄ |
| II-6 | H | H | H | *n*-C₇H₁₅ |
| II-7 | H | H | H | 4-Me-C₆H₄ |
| II-8 | H | H | H | 4-O₂N-C₆H₄ |
| II-9 | H | H | H | 3,4,5-(MeO)₃-C₆H₂ |
| II-10 | H | H | H | 3,4-(HO)₂-C₆H₃ |
| II-18 | H | Me | H | H |
| II-19 | H | Me | H | Me |
| II-20 | H | Me | H | Ph |
| II-21 | H | Me | H | 4-Cl-C₆H₄ |
| II-22 | H | Me | H | 4-Me-C₆H₄ |
| II-23 | H | Me | H | 4-MeO-C₆H₄ |
| II-24 | H | Me | H | 4-O₂N-C₆H₄ |

6. A triazolopurine compound represented by the following general formula (II): 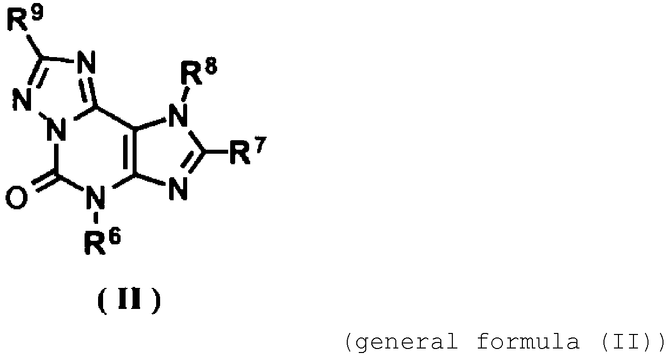
- wherein R⁶ represents a hydrogen atom, and R⁸ represents a hydrogen atom or an alkyl group; R⁷ and R⁹ each independently represent a hydrogen atom, an alkyl group, phenyl group or a phenyl group having a substituent selected from: a halogen atom, an alkyl group, an alkoxy group, an amino group, an alkylamino group, a methylenedioxy group, a hydroxy group or nitro group,
for use in treatment or prevention of a condition selected from hyperuricemia, gout, impaired kidney functions such as kidney failure and chronic kidney disease, hypertension, cardiovascular disease, dyslipidemia and metabolic syndrome.

7. The compound for use according to claim 6, wherein R⁷ and R⁹ each independently are selected from hydrogen, alkyl, phenyl, a methylphenyl, ethylphenyl, methoxyphenyl, ethoxyphenyl, dimethylaminophenyl, diethylaminophenyl, fluorophenyl, chlorophenyl, bromophenyl, iodophenyl, methylenedioxyphenyl; hydroxyphenyl; and nitrophenyl.

8. The compound for use according to claim 6, wherein R⁶, R⁷ and R⁸ are all hydrogen atom, and R⁹ is selected from hydrogen atom, alkyl group, or a phenyl group having a substituent selected from: a halogen atom, an alkyl group, an alkoxy group, an amino group, an alkylamino group, a methylenedioxy group, a hydroxy group or nitro group.

9. The compound for use according to claim 6, wherein R⁶ and R⁸ are both hydrogen atom, and wherein
- R⁷ is an alkyl group or an aryl group selected from a phenyl group, and a phenyl group having a substituent selected from: a halogen atom, an alkyl group, an alkoxy group, an amino group, an alkylamino group, a methylenedioxy group, a hydroxy group or nitro group, and
- R⁹ is hydrogen atom, an alkyl group, a phenyl group or a phenyl group having a substituent selected from: a halogen atom, an alkyl group, an alkoxy group, an amino group, an alkylamino group, a methylenedioxy group, a hydroxy group or nitro group.

10. The compound for use according to claim 6, wherein the compound is selected from the following Table:
| | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-C₆H₄** |
| **II-8** | **H** | **H** | **H** | **4-O₂N-C₆H₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-C₆H₄** |
| **II-23** | **H** | **Me** | **H** | **4-MeO-C₆H₄** |
| **II-24** | **H** | **Me** | **H** | **4-O₂N-C₆H₄** |

11. A xanthine oxidase inhibitory composition, containing as an active ingredient at least one compound selected from the group consisting of the triazolopurine compounds according to claims 1 to 5.

12. A pharmaceutical composition, containing as an active ingredient at least one compound selected from the group consisting of the triazolopurine compounds according to claims 1 to 5.

13. The pharmaceutical composition according to claim 12, wherein the pharmaceutical composition is to prevent or treat at least one disease selected from the group consisting of hyperuricemia, gout, impaired kidney functions such as kidney failure and chronic kidney disease, hypertension, cardiovascular disease, dyslipidemia and metabolic syndrome.

## Patentansprüche

1. Eine Triazolopurinverbindung, dargestellt durch die folgende allgemeine Formel (II): 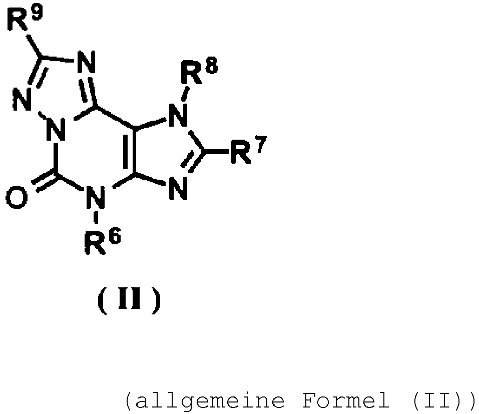 wobei R⁶ ein Wasserstoffatom darstellt und R⁸ ein Wasserstoffatom oder eine Alkylgruppe darstellt; R⁷ und R⁹ stellen jeweils unabhängig voneinander ein Wasserstoffatom, eine Alkylgruppe oder eine Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe, dar.

2. Die Verbindung gemäß Anspruch 1, wobei R⁷ und R⁹ jeweils unabhängig voneinander ausgewählt sind aus Wasserstoff, Alkyl, Methylphenyl, Ethylphenyl, Methoxyphenyl, Ethoxyphenyl, Dimethylaminophenyl, Diethylaminophenyl, Fluorphenyl, Chlorphenyl, Bromphenyl, Iodphenyl, Methylendioxyphenyl; Hydroxyphenyl; und Nitrophenyl.

3. Die Verbindung gemäß Anspruch 1 oder 2, wobei R⁶, R⁷ und R⁸ alle ein Wasserstoffatom sind und R⁹ ausgewählt ist aus einem Wasserstoffatom, einer Alkylgruppe oder einer Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe.

4. Die Verbindung gemäß Anspruch 1, wobei R⁶ und R⁸ beide ein Wasserstoffatom sind, und
- R⁷ ist eine Alkylgruppe oder eine Arylgruppe, ausgewählt aus einer Phenylgruppe und einer Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe, und
- R⁹ ist ein Wasserstoffatom, eine Alkylgruppe oder eine Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe.

5. Die Verbindung gemäß Anspruch 1 oder 2, wobei R⁶, R⁷, R⁸ und R⁹ jeweils so sind wie in der Tabelle gezeigt:
| | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-C₆H₄** |
| **II-8** | **H** | **H** | **H** | **4-O₂N-C₆H₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-C₆H₄** |
| **II-23** | **H** | **Me** | **H** | **4-MeO-C₆H₄** |
| **II-24** | **H** | **Me** | **H** | **4-O₂N-C₆H₄** |

6. Eine Triazolopurinverbindung, dargestellt durch die folgende allgemeine Formel (II): 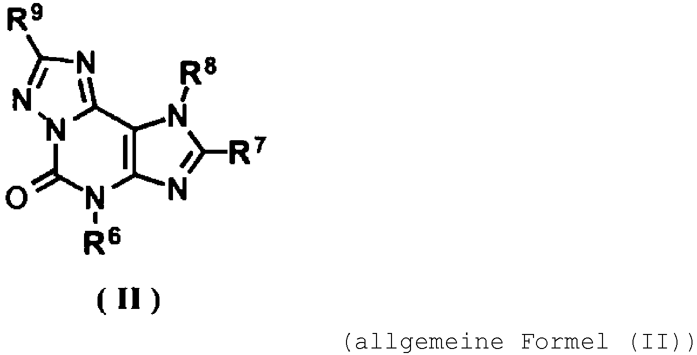
- worin R⁶ ein Wasserstoffatom darstellt und R⁸ ein Wasserstoffatom oder eine Alkylgruppe darstellt; R⁷ und R⁹ stellen jeweils unabhängig voneinander ein Wasserstoffatom, eine Alkylgruppe, eine Phenylgruppe oder eine Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe, dar,
zur Verwendung bei der Behandlung oder Vorbeugung eines Zustands, ausgewählt aus Hyperurikämie, Gicht, gestörten Nierenfunktionen wie Nierenversagen und chronischen Nierenerkrankungen, Bluthochdruck, Herz-Kreislauf-Erkrankungen, Dyslipidämie und metabolischem Syndrom.

7. Die Verbindung zur Verwendung gemäß Anspruch 6, wobei R⁷ und R⁹ jeweils unabhängig voneinander ausgewählt sind aus Wasserstoff, Alkyl, Phenyl, Methylphenyl, Ethylphenyl, Methoxyphenyl, Ethoxyphenyl, Dimethylaminophenyl, Diethylaminophenyl, Fluorphenyl, Chlorphenyl, Bromphenyl, Iodphenyl, Methylendioxyphenyl, Hydroxyphenyl und Nitrophenyl.

8. Die Verbindung zur Verwendung gemäß Anspruch 6, wobei R⁶, R⁷ und R⁸ alle ein Wasserstoffatom sind und R⁹ ausgewählt ist aus einem Wasserstoffatom, einer Alkylgruppe oder einer Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe.

9. Die Verbindung zur Verwendung gemäß Anspruch 6, wobei R⁶ und R⁸ beide ein Wasserstoffatom sind, und wobei
- R⁷ ist eine Alkylgruppe oder eine Arylgruppe, ausgewählt aus einer Phenylgruppe und einer Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe, und
- R⁹ ist ein Wasserstoffatom, eine Alkylgruppe, eine Phenylgruppe oder eine Phenylgruppe mit einem Substituenten, ausgewählt aus: einem Halogenatom, einer Alkylgruppe, einer Alkoxygruppe, einer Aminogruppe, einer Alkylaminogruppe, einer Methylendioxygruppe, einer Hydroxygruppe oder einer Nitrogruppe.

10. Die Verbindung zur Verwendung gemäß Anspruch 6, wobei die Verbindung aus der folgenden Tabelle ausgewählt ist:
| | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-C₆H₄** |
| **II-8** | **H** | **H** | **H** | **4-O₂N-C₆H₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-C₆H₄** |
| **II-23** | **H** | **Me** | **H** | **4-MeO-C₆H₄** |
| **II-24** | **H** | **Me** | **H** | **4-O₂N-C₆H₄** |

11. Eine Xanthinoxidase hemmende Zusammensetzung, die als Wirkstoff mindestens eine Verbindung enthält, die aus der Gruppe ausgewählt ist, die aus den Triazolopurinverbindungen gemäß den Ansprüchen 1 bis 5 besteht.

12. Eine pharmazeutische Zusammensetzung, die als Wirkstoff mindestens eine Verbindung enthält, die aus der Gruppe ausgewählt ist, die aus den Triazolopurinverbindungen gemäß den Ansprüchen 1 bis 5 besteht.

13. Die pharmazeutische Zusammensetzung gemäß Anspruch 12, wobei die pharmazeutische Zusammensetzung zur Vorbeugung oder Behandlung mindestens einer Krankheit dient, die aus der Gruppe ausgewählt ist, die aus Hyperurikämie, Gicht, gestörten Nierenfunktionen wie Nierenversagen und chronischer Nierenerkrankung, Bluthochdruck, Herz-Kreislauf-Erkrankungen, Dyslipidämie und metabolischem Syndrom besteht.

## Revendications

1. Composé de triazolopurine représenté par la formule générale suivante (II) : 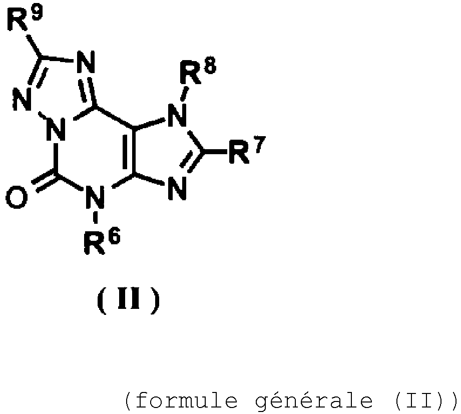 dans laquelle R⁶ représente un atome d'hydrogène, et R⁸ représente un atome d'hydrogène ou un groupe alkyle ; R⁷ et R⁹ représentent chacun indépendamment un atome d'hydrogène, un groupe alkyle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro.

2. Composé selon la revendication 1, dans lequel R⁷ et R⁹ sont sélectionnés chacun indépendamment parmi hydrogène, alkyle, un méthylphényle, éthylphényle, méthoxyphényle, éthoxyphényle, diméthylaminophényle, diéthylaminophényle, fluorophényle, chlorophényle, bromophényle, iodophényle, méthylènedioxyphényle ; hydroxyphényle ; et nitrophényle.

3. Composé selon la revendication 1 ou 2, dans lequel R⁶, R⁷ et R⁸ sont tous un atome d'hydrogène, et R⁹ est sélectionné parmi un atome d'hydrogène, un groupe alkyle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro.

4. Composé selon la revendication 1, dans lequel R⁶ et R⁸ sont tous deux un atome d'hydrogène, et
- R⁷ est un groupe alkyle ou un groupe aryle sélectionné parmi un groupe phényle, et un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro, et
- R⁹ est un atome d'hydrogène, un groupe alkyle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro.

5. Composé selon la revendication 1 ou 2, dans lequel R⁶, R⁷, R⁸ et R⁹ sont chacun tel qu'illustré dans le tableau :
| | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-CeH₄** |
| **II-8** | **H** | **H** | **H** | **4-O₂N-CₑH₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-C₆H₄** |
| **II-23** | **H** | **Me** | **H** | **4-MeO-C₆H₄** |
| **II-24** | **H** | **Me** | **H** | **4-O₂N-C₆H₄** |

6. Composé de triazolopurine représenté par la formule générale suivante (II) : 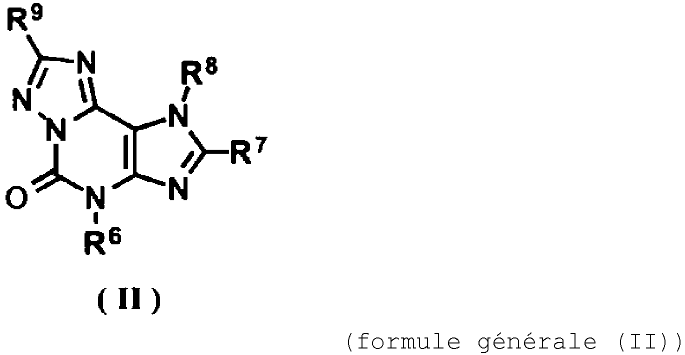
- dans laquelle R⁶ représente un atome d'hydrogène, et R⁸ représente un atome d'hydrogène ou un groupe alkyle ; R⁷ et R⁹ représentent chacun indépendamment un atome d'hydrogène, un groupe alkyle, groupe phényle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro,
destiné à être utilisé dans le traitement ou la prévention d'une affection sélectionnée parmi l'hyperuricémie, la goutte, des fonctions rénales déficientes telles que l'insuffisance rénale et la maladie rénale chronique, l'hypertension, une maladie cardiovasculaire, la dyslipidémie et le syndrome métabolique.

7. Composé destiné à être utilisé selon la revendication 6, dans lequel R⁷ et R⁹ sont sélectionnés chacun indépendamment parmi hydrogène, alkyle, phényle, un méthylphényle, éthylphényle, méthoxyphényle, éthoxyphényle, diméthylaminophényle, diéthylaminophényle, fluorophényle, chlorophényle, bromophényle, iodophényle, méthylènedioxyphényle ; hydroxyphényle ; et nitrophényle.

8. Composé destiné à être utilisé selon la revendication 6, dans lequel R⁶, R⁷ et R⁸ sont tous un atome d'hydrogène, et R⁹ est sélectionné parmi un atome d'hydrogène, un groupe alkyle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro.

9. Composé destiné à être utilisé selon la revendication 6, dans lequel R⁶ et R⁸ sont tous deux un atome d'hydrogène, et dans lequel
- R⁷ est un groupe alkyle ou un groupe aryle sélectionné parmi un groupe phényle, et un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro, et
- R⁹ est un atome d'hydrogène, un groupe alkyle, un groupe phényle ou un groupe phényle ayant un substituant sélectionné parmi : un atome d'halogène, un groupe alkyle, un groupe alcoxy, un groupe amino, un groupe alkylamino, un groupe méthylènedioxy, un groupe hydroxy ou groupe nitro.

10. Composé destiné à être utilisé selon la revendication 6, dans lequel le composé est sélectionné parmi le Tableau suivant :
| | **R⁶** | **R⁷** | **R⁸** | **R⁹** |
|---|---|---|---|---|
| **II-1** | **H** | **H** | **H** | **H** |
| **II-2** | **H** | **H** | **H** | **Me** |
| **II-4** | **H** | **H** | **H** | **4-Cl-C₆H₄** |
| **II-5** | **H** | **H** | **H** | **4-MeO-C₆H₄** |
| **II-6** | **H** | **H** | **H** | ***n*-C₇H₁₅** |
| **II-7** | **H** | **H** | **H** | **4-Me-C₆H₄** |
| **II-8** | **H** | **H** | **H** | **4-O₂N-C₆H₄** |
| **II-9** | **H** | **H** | **H** | **3,4,5-(MeO)₃-C₆H₂** |
| **II-10** | **H** | **H** | **H** | **3,4-(HO)₂-C₆H₃** |
| **II-18** | **H** | **Me** | **H** | **H** |
| **II-19** | **H** | **Me** | **H** | **Me** |
| **II-20** | **H** | **Me** | **H** | **Ph** |
| **II-21** | **H** | **Me** | **H** | **4-Cl-C₆H₄** |
| **II-22** | **H** | **Me** | **H** | **4-Me-CeH₄** |
| **II-23** | **H** | **Me** | **H** | **4-MeO-C₆H₄** |
| **II-24** | **H** | **Me** | **H** | **4-O₂N-C₆H₄** |

11. Composition inhibitrice de xanthine oxydase, contenant comme ingrédient actif au moins un composé sélectionné parmi le groupe constitué des composés de triazolopurine selon les revendications 1 à 5.

12. Composition pharmaceutique, contenant comme ingrédient actif au moins un composé sélectionné parmi le groupe constitué des composés de triazolopurine selon les revendications 1 à 5.

13. Composition pharmaceutique selon la revendication 12, dans laquelle la composition pharmaceutique est destinée à prévenir ou traiter au moins une maladie sélectionnée parmi le groupe constitué de l'hyperuricémie, la goutte, des fonctions rénales déficientes telles que l'insuffisance rénale et la maladie rénale chronique, l'hypertension, d'une maladie cardiovasculaire, la dyslipidémie et du syndrome métabolique.
